(19) Europäisches Patentamt
European Patent Office
Office européen des brevets

(11) Numéro de publication: **0 048 186**
**B1**

(12) # FASCICULE DE BREVET EUROPEEN

(45) Date de publication du fascicule du brevet: **02.05.85**

(21) Numéro de dépôt: **81401035.1**

(22) Date de dépôt: **26.06.81**

(51) Int. Cl.⁴: **C 07 D 213/64,**
C 07 D 213/68,
C 07 D 209/48,
C 07 C 69/743, C 07 C 121/75,
C 07 D 233/72,
C 07 D 307/42, A 23 K 1/16,
A 61 K 31/22, A 01 N 53/00

(54) **Nouveaux dérivés de l'acide cyclopropane carboxylique, leur préparation, leur application à la lutte contre les parasites des végétaux, des animaux et des locaux, les compositions les renfermant et les nouveaux intermédiaires obtenus.**

(30) Priorité: **02.07.80 FR 8014722**

(43) Date de publication de la demande:
**24.03.82 Bulletin 82/12**

(45) Mention de la délivrance du brevet:
**02.05.85 Bulletin 85/18**

(84) Etats contractants désignés:
**AT BE CH DE FR GB IT LI LU NL SE**

(56) Documents cités:
**EP-A-0 018 894**
**FR-A-1 439 914**
**FR-A-2 100 835**
**FR-A-2 143 820**
**FR-A-2 185 612**
**FR-A-2 418 218**

**Le dossier contient des informations techniques présentées postérieurement au dépôt de la demande et ne figurant pas dans le présent fascicule.**

(73) Titulaire: **ROUSSEL-UCLAF**
**35, boulevard des Invalides**
**F-75007 Paris (FR)**

(72) Inventeur: **Martel, Jacques**
**15, rue Douvillez**
**F-93140-Bondy (FR)**
Inventeur: **Tessier, Jean**
**30, rue Jean Moulin**
**F-94300-Vincennes (FR)**
Inventeur: **Teche, André**
**15, rue Godot de Mauroy**
**F-75009-Paris (FR)**

(74) Mandataire: **Tonnellier, Marie-José**
**ROUSSEL-UCLAF 111, route de Noisy Boîte Postale no.9**
**F-93230 Romainville (FR)**

(56) Documents cités:
**JOURNAL OF THE CHEMICAL SOCIETY, Perkin Transactions I, 1974, M. ELLIOTT et al.: "The pyrethrins and related compounds. Part XVIII. Insecticidal 2,2-dimethyl-cyclopropanecarboxylates with new unsaturated 2-substituents" pages 2470-2474**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 048 186 B1

(56) Documents cités:

**PESTICIDE SCIENCE, vol. 7, 1967, pages 499-502 Blackwell Scientific Publications M. ELLIOTT et al.: "Insecticidal activity of the pyrethrins and related compounds. X. 5-Benzyl-3-furylmethyl 2,2-dimethylcyclopropanecarboxylates with ethylenic substituents at position 3 on the cyclopropane ring"**

**J. Agric. Biol. Chem. 34 (NO. 7) p. 1119-1125 (1970)**

**Description**

La présente invention concerne de nouveaux dérivés de l'acide cyclopropane carboxylique, leur préparation, leur application à la lutte contre les parasites des végétaux, des animaux et des locaux, les compositions les renfermant et les nouveaux intermédiaires obtenus.

L'invention a plus précisément pour objet des dérivés de l'acide cyclopropane carboxylique, portant en position 3 une chaîne latérale insaturée de géométrie Z et de structure A:

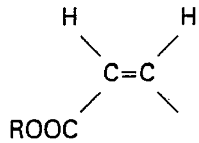

(A)

On connaissait certes des dérivés de l'acide cyclopropane carboxylique portant en position 3 un chaîne latérale de structure B:

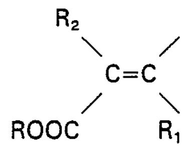

(B)

mais dont la géométrie était essentiellement E. Citons par exemple les dérivés de l'acide pyréthrique ($R=CH_3$, $R_1=H$, $R_2=CH_3$). Quelques dérivés pour lesquels $R_1=R_2=H$ ont également été décrits: Voir à ce sujet le brevet français 2.185.612 ainsi que J. Chem. Soc. Perkin I, page 2470, 1974 et Pest. Sci. *7*, page 499, 1976. Toutefois, la géométrie de la chaîne latérale de ces composés est E de façon prédominante. En effet, le procédé de synthèse utilisé pour préparer ces dérivés ne fournissait presqu'exclusivement que la géométrie E. (voir Agr. Biol. Chem. *34*, page 1119, 1970). Pour ces composés, dont la géométrie de la chaîne latérale était essentiellement E, aucune propriété intéressante n'a pu être mise en évidence.

Or, on vient de découvrir que certains produits, dont la chaîne latérale insaturée est de structure A mais dont la géométrie est Z, présentent des propriétés pesticides inattendues, notamment des propriétés insecticides exceptionelles.

L'invention a pour objet sous toutes les formes isomères possibles ou sous forme de mélanges, les composés de formule (I')

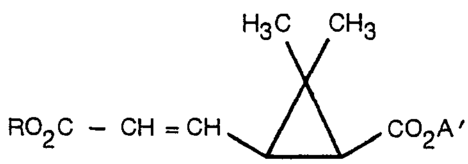

(I')

dans laquelle la copule cylcopropanique est de structure 1R cis et la double liaison de géométrie Z et dans laquelle A' représente

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

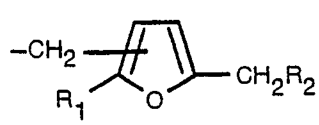

dans lequel le substituant $R_1$ représente un atome d'hydrogène ou un radical méthyle et le substituant $R_2$ un aryle monocyclique ou un groupement $-CH_2-C{\equiv}CH$,

soit un groupement

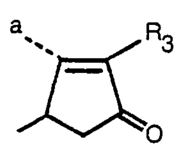

dans lequel a représente un atome d'hydrogène ou un radical méthyle et $R_3$ représente le radical $—CH_2—CH{=}CH_2$, $—CH_2—CH{=}CH—CH_3$, $—CH_2—CH{=}CH—CH{=}CH_2$, ou $—CH_2—CH{=}CH—CH_2—CH_3$,

soit un groupement

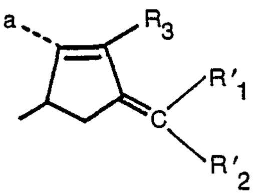

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyl comportant de 2 à 5 atomes de carbone, ou un groupement cyano.

soit un groupement

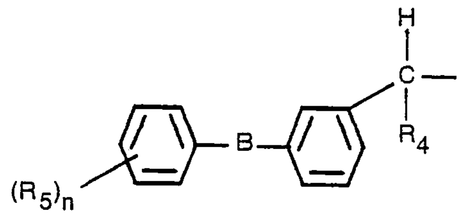

dans lequel B représente un atome d'oxygène ou de soufre ou un groupement

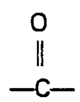

ou $—CH_2—$ et $R_4$ représente un atome d'hydrogène, un radical $—C{\equiv}N$, un radical méthyle, un radical $—CONH_2$, un radical $—CSNH_2$ ou un radical $—C{\equiv}CH$, $R_5$ représente un atome d'halogène ou un radical méthyle et n représente un nombre égal à 0, 1 ou 2,

soit un groupement

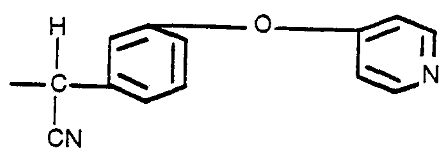

soit un groupement

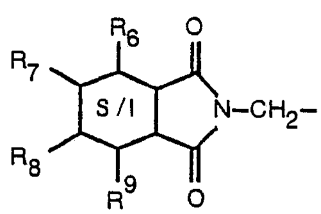

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

soit un groupement

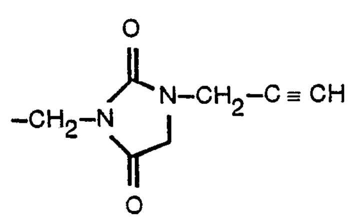

soit un groupement

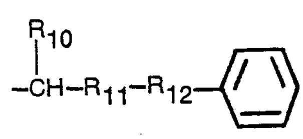

dans lequel $R_{10}$ représente un atome d'hydrogène ou un radical CN, $R_{12}$ représente un radical $—CH_2—$ ou un atome d'oxygène, $R_{11}$ représente un radical thiazolyle ou thiadiazolyle dont la liaison avec

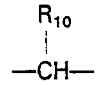

peut se trouver à l'une quelconque des positions disponibles, $R_{12}$ étant lié à $R_{11}$ par l'atome de carbone compris entre l'atome de soufre et un atome d'azote,

soit un groupement

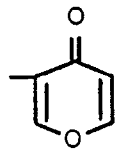

soit un groupement

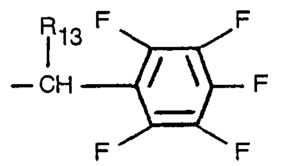

dans lequel $R_{13}$ représente un atome d'hydrogène ou un radical CN,

soit un groupement

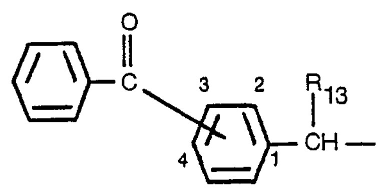

dans lequel $R_{13}$ est défini comme ci-dessus, et le radical benzoyle est en position 3 ou 4,

soit un groupement

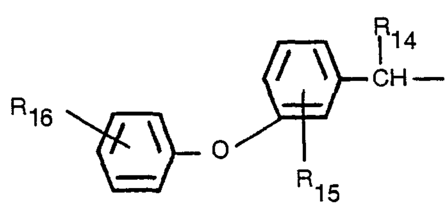

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

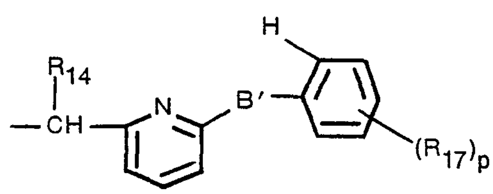

dans lequel $R_{14}$ est défini comme ci-dessus, chacun des $R_{17}$ représente indépendam-ment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre et R représente un radical alcoyle renferment de 1 à 18 atomes de carbone substitué par un ou plusieurs groupements fonctionnels identiques ou différents chosis dans le groupe constitué par les atomes d'halogène, les groupements OH our SH, les groupements OR' ou

SR' dans lesquels R' représente un radical alcoyle renfermant de 1 à 8 atomes de carbone, les groupements $NO_2$ ou

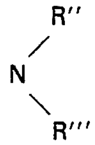

dans lesquels R'' et R''', identiques ou différents, représentent un atome d'hydrogène, ou un radical alcoyle renfermant de 1 à 8 atomes de carbone, les groupements C≡N, $SO_3H$ ou $PO_4H_2$ ou les groupements $COalc_1$, $SO_2alc_2$, ou $SO_3alc_3$ dans lesquels $alc_1$, $alc_2$ et $alc_3$ représentent des radicaux alcoyle renfermant de 1 à 18 atomes de carbone, ou R représente un radical alcoyle renferment de 1 à 18 atomes de carbone substitué par un radical aryle lui-même éventuellement substitué par un ou plusieurs groupements OH, Oalc ou alc renfermant de 1 à 8 atomes de carbone, par un ou plusieurs groupements $CF_3$, $OCF_3$, $SCF_3$, ou par un groupement (G):

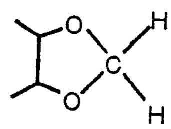

(G)

ou R représente un radical alcoyle renfermant de 1 à 18 atomes de carbone substitué sur deux carbones adjacents par un groupement ($G_1$)

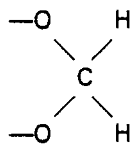

($G_1$)

ou substitué par un groupement

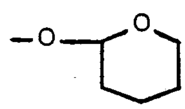

ou R représente un groupement aryle renfermant de 6 à 14 atomes de carbone éventuellement substitué par un ou plusieurs groupements OH, Oalc ou alc renfermant de 1 à 8 atomes de carbone ou par un groupement $CF_3$, $OCF_3$ ou $SCF_3$, ou R représente un radical pyridinyle, furanyle, thiophényle, oxazolyle ou thiazolyle.

Les composés de formula (I') peuvent exister sous de nombreuses formes stéréoisomères: ils possèdent, en effet, deux carbones asymétriques en 1 et en 3 du cyclopropane; ils peuvent, de plus, présenter un ou plusieurs centres d'asymétrie dans la partie A' comme dans la partie R.

Lorsque A' représente un radical alcoyle, il s'agit de préférence du radical méthyle, éthyle, n-propyle, isopropyle, n-butyle, isobutyle, ou terbutyle.

Lorsque A' représente un radical benzyle substitué par un ou plusieurs radicaux alcoyle, il s'agit de préférence des radicaux méthyle ou éthyle.

Lorsque A' représente un radical benzyle substitué par un ou plusieurs radicaux alcényle, il s'agit de préférence de radicaux vinyle, allyle, 2-méthylallyle, ou isobuténylе.

Lorsque A' représente un radical benzyle substitué par un ou plusieurs alcényloxy, il s'agit de préférence de radicaux vinyloxy, allyloxy, 2-méthylallyloxy ou isobutényloxy.

Lorsque A' repré-sente un radical benzyle substitué par un ou plusieurs atomes d'halogène, il s'agit de préférence d'atomes de chlore, de brome ou de fluor.

Lorsque R représente un radical alcoyle substitué par un ou plusieurs groupements fonctionnels mentionnés ci-dessus, on entend de préférence par alcoyle un radical renfermat de 1 à 8 atomes de carbone comme, par exemple, le radical méthyle, éthyle, propyle, isopropyle, n-butyle, isobutyle ou tert-butyle.

Lorsque R représente un radical alcoyle substitué par un radical aryle, il peut s'agir du radical benzyle ou du radical phénéthyle éventuellement substitué comme défini précé-demment.

Lorsque R représente un radical alcoyle substitué par un ou plusieurs des dits groupements fonctionnels, on peut citer comme valeurs préférées de R les radicaux:

$$—(CH_2)_n—CHal_3$$

dans lequel n est un entier de 1 à 8 et Hal un atome d'halogène, par exemple le radical

$-CH_2-CCl_3$, $-CH_2-CF_3$, $-CH_2-CH_2-CCl_3$ ou
$-CH_2-CH_2-CF_3$, $-(CH_2)_{n}1-CH\ Hal_2$

dans lequel Hal est défini comme ci-dessus et $n_1$ est un nombre de 0 à 8, par exemple le radical

$-CH_2-CHCl_2$, $-CH_2-CHF_2$ ou $-CHF_2$, $-(CH_2)_n-CH_2\ Hal$

dans lequel n et Hal sont définis comme ci-dessus, par exemple le radical

$-CH_2-CH_2Cl$ ou $-CH_2-CH_2F$, $-C-(CHal_3)_3$

dans lequel Hal est défini comme ci-dessus, par exemple le radical

$-C-(CF_3)_3$ ou 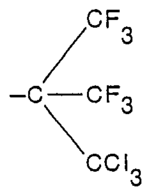 , 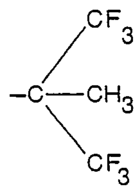 , 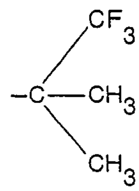 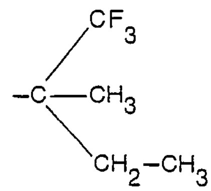

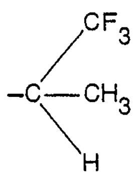 ou 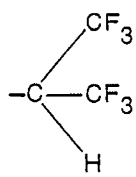

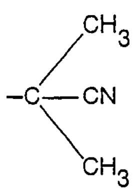 , 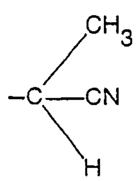 ou $-(CH_2)_n-CN$, dans lequel n est défini comme précédemment,

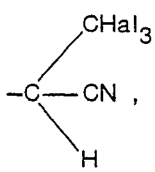 , dans lequel Hal est défini comme précédemment,

par exemple le radical 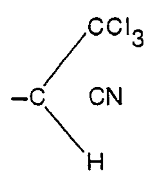 ,

$-(CH_2)_n-OR'$, dans lequel n est défini comme précédemment et R' représente un atome d'hydrogène ou un radical alcoyle linéaire ou ramifié, comportant de 1 à 8 atomes de carbone, par exemple le radical

$-CH_2-OCH_3$, $-CH_2-CH_2-O-CH_3$, $-CH_2-CH_2-O-CH_2-CH_3$ ou $-CH_2-CH_2-OH$,

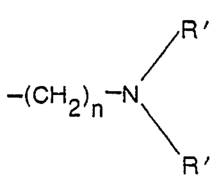 ,

dans lequel n et R′ sont définis comme précédemment et les deux radicaux R′ peuvent être différents entre eux, par exemple le radical

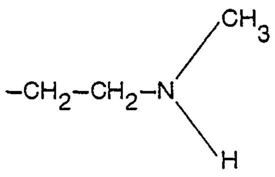 , 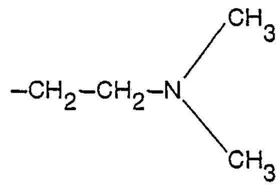 ou 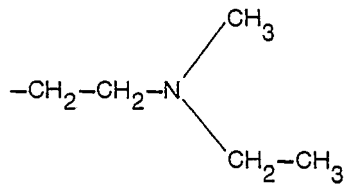 ,

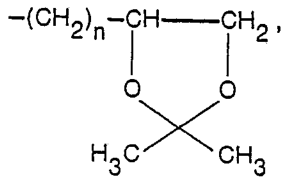

dans lequel n est défini comme précédemment, par exemple le radical

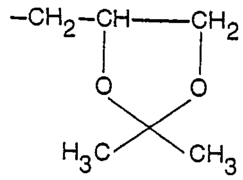 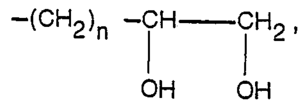

dans lequel n est défini comme précédemment, par exemple le radical

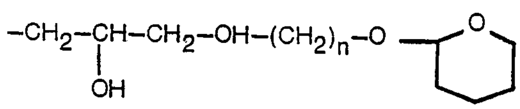 ,

dans lequel n est défini comme précédemment, par exemple le radical

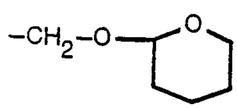 ou 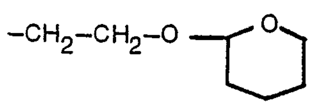 , 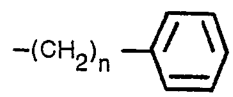 ,

dans lequel n est défini comme précédemment, par exemple le radical benzyle ou phénéthyle,

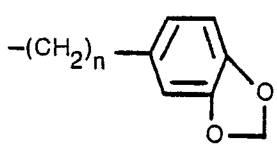 ,

dans lequel n est défini comme précédemment, par exemple le radical

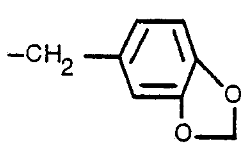

Lorsque R représente un radical aryle éventuellement substitué, il s'agit de préférence du radical phényle éventuellement substitué comme défini précédemment.

L'invention a notamment pour objet les composés de formule (I′), tell que définie précédemment, répondant à la formule (I)

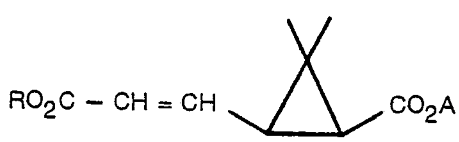 (I)

dans laquelle la structure de la copule cyclopropanique est 1R cis et la géométrie de la double liaison Z et dans laquelle A représente

soit un radical alcoyle renfermant de 1 à 18 atomes de carbone,

soit un radical benzyle éventuellement substitué comme défini précédemment, tel que défini précédemment,

soit un groupement

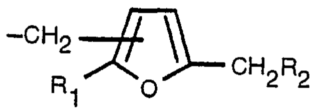

dans lequel les substituants $R_1$ et $R_2$ sont définis comme précédemment,

soit un groupement

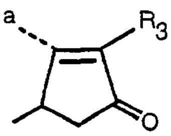

dans lequel a et $R_3$ sont définis comme précédemment,

soit un groupement

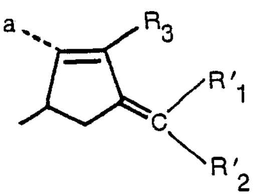

dans lequel a, $R'_1$, $R'_2$ et $R_3$ sont définis comme précédemment,

soit un groupement

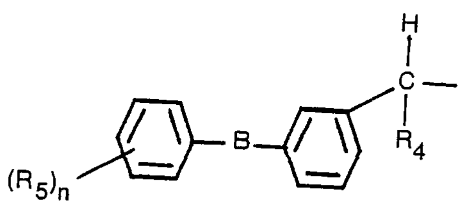

dans lequel B représente un atome d'oxygène, un groupement

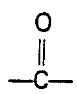

ou $—CH_2—$, $R_4$ est défini comme précédemment,

$R_5$ représente un atome de chlore ou un radical méthyle et n représente un nombre égal à 0, 1 ou 2, et notamment le groupement 3-phénoxy benzyle, α-cyano 3-phénoxy benzyle ou α-éthynyl 3-phénoxy benzyle,

soit un groupement

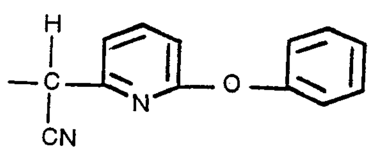

soit un groupement

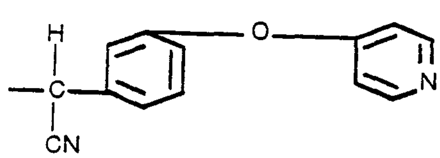

soit un groupement

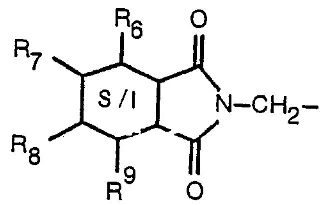

dans lequel les substituants $R_6$, $R_7$, $R_8$, $R_9$ et S/I sont définis comme précédemment,
soit un groupement

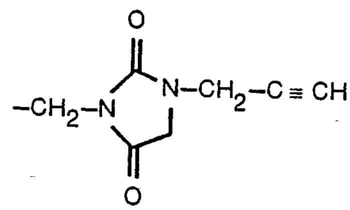

et R est défini comme précédemment.

L'invention a notamment pour object les composés de formule (I') telle que définie précédemment, dans laquelle A' représente un groupement α-cyano 3-phénoxy benzyle sous forme S, R ou RS ainsi que ceux dans laquelle A' représente un groupement 2-méthyl-4-oxo-3-(2-propényl) 2-cyclopenten-1-yle sous forme S, R ou RS.

L'invention a plus spécialement pour objet les composés de formule (I') pour lesquels R représente un radical alcoyle renfermant de 1 à 18 atomes de carbone, substitué par un ou plusieurs groupements fonctionnels mentionnés ci-dessus et tout spécialement ceux dans lesquels R représente un radical alcoyle substitué par un ou plusieurs atomes d'halogène, par exemple un ou plusieurs atomes de fluor. Comme exemple de radical alcoyle substitué par un ou plusieurs atomes d'halogène, on peut citer tout spécialement le radical $—CH_2CF_3$.

L'invention a tout particulièrement pour objet les composés dont la préparation est donnée plus loin dans la partie expérimentale et notamment, parmi ceux-ci, les composés des exemples 1, 2, 23, 26, 29, 30 et 35.

Les composés de formule (I') présentent d'intéressantes propriétés qui permettent leur utilisation dans la lutte contre les parasites, il peut s'agir par exemple de la lutte contre les parasites des végétaux, les parasites des locaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I') à la lutte contre les parasites des végétaux, les parasites des locaux et les parasites des animaux à sang chaud.

Les produits de formule (I') peuvent donc être utilsés notamment pour lutter contre les insectes dans le domaine agricole, pour lutter par exemple contre les pucerons, les larves de lépidoptères et les coléoptères. Ils sont utilisés à des doses comprises entre 10 g et 300 g de matière active à l'hectare.

Les produits de formule (I') peuvent également être utilisés pour lutter contre les insectes dans les locaux, pour lutter notamment contre les mouches, les moustiques et les blattes.

Le composé de l'exemple 1 est un produit tout à fait remarquable comme le montrent les résultats des tests ci-après. Il présente un excellent pouvoir létal et un très bon pouvoir de knock down.

Les produits de formule (I') sont de plus photostables et ne sont pas toxiques pour les mammifères.

L'ensemble de ces propriétés fait des produits de formule (I') des produits qui correspondent parfaitement aux exigences de l'industrie agrochimique moderne: ils permettent de protéger les récoltes tout en préservant l'environnement.

Les produits de formule (I') peuvent aussi être utilisés pour lutter contre les acariens et les nématodes parasites des végétaux.

Les composés de formule (I') peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits définis ci-dessus.

L'invention à notamment pour objet les compositions insecticides renfermant comme principe actif au moins l'un des produits définis ci-dessus.

Parmi les compositions notamment insecticides préférées de l'invention, on peut citer tout spécialement les compositions renfermant la (1R cis) 2,2-diméthyl 3/(Z) 3(2,2,2-trifluoroéthoxy) 3-oxo 1-propenyl/ cyclopropane carboxylate de (S) α-cyano 3-phénoxy benzyle, le (1R cis) 2,2-diméthyl 3/(Z) 3-oxo

3-(2-(1,1,1,3,3,3-hexafluoro) propoxy) 1-propényl/ cyclopropane carboxylate de (S) α-cyano 3-phénoxy benzyle,

le (1R cis) 2,2-diméthyl 3/(Z) 3-oxo 3-(2,2,2-trifluoro éthoxy) 1-propényl/ cyclopropane carboxylate de (R) α-éthynyl 3-phénoxy benzyle,

le (1R cis) 2,2-diméthyl 3/(Z) 3-oxo 3-(2,2,2-trifluoro éthoxy) 1-propényl/ cyclopropane carboxylate de (RS) cyano 6-phénoxy 2-pyridyl méthyle,

le (1R cis) 2,2-diméthyl 3/(Z) 3-oxo 3-(2-fluoro éthoxy) 1-propényl/ cyclopropane carboxylate de (S) α-cyano 3-phénoxy benzyle,

le (1R cis) 2,2-diméthyl 3/(Z) 3-oxo 3-(2,2,2-trifluoro éthoxy) 1-propényl/ cyclopropane carboxylate de (3-propargyl 2,5-dioxoimidazolidinyl) méthyle,

le (1R cis) 2,2-diméthyl 3/(Z) 3-oxo 3-(2,2,2-trifluoro éthoxy) 1-propényl/ cyclopropane carboxylate de (1S) 2-méthyl-4-oxo 3-(2-propényl) 2-cyclopenten-1-yle.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005% à 10% en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un électromosquito destroyer.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1% en poids.

Dans le cas où l'on utilise un support fibreux incombustible, la dose de matière active peut alors être, par exemple, de 0,03 à 95% en poids.

Les compositions selon l'invention pour un usage dans les locaux peuvent aussi être obtenues en préparant une huile pulvérisable à base de principe actif, cette huile imbibant la mèche d'une lampe et étant alors soumis à la combustion.

La concentration du principe actif incorporé à l'huile est, de préférence, de 0,03 à 95% en poids.

Les compositions insecticides selon l'invention, comme les compositions acaricides et nématicides peuvent être additionées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80% ou des liquides pour pulvérisation foliaire contenant de 1 à 500 g/l de principe actif. On peut également employer des poudres pour poudrage foliaires contenant de 0,05 à 3% de matière active.

Pour l'usage nématicide, on utilise de préférence des liquides pour traitement des sols contennant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo/2,2-1/5-heptène-2,3-di carboximide, ou le pipéronyl-bis-2-(2'-n-butoxy éthoxy) éthylacétal (ou tropital).

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poisons, des acides aminés de synthèse, des sels minéraux, des vitamines et des antioxydants.

L'invention a donc ainsi pour objet les compositions alimentaires définies ci-dessus.

Les composés de formule (I') présentent une excellente tolérance générale, et l'invention a donc également pour objet les produits de formule (I'), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisées en médicine humaine pour lutter contre les poux à titre préventif ou curatif et pour lutter contre la gale. Ils peuvent également être utilisés comme anthelmintiques.

Les médicaments de l'invention peuvent être administrés par voie externe, par vaporisation, par shampooing, par bain ou badigeonnage.

Les médicaments de l'invention à usage vétérinaire peuvent être également administrés par badigeonnage de l'épine dorsale selon la méthode dite méthode "pour-on". Ils peuvent être également administrés par voie digestive ou parentérale.

L'invention a donc pour object les compositions pharmaceutiques renfermant comme principe actif au moins un des médicaments définis précédemment.

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5-6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido-méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-térahalo éthyl)cyclopropane-1-carboxyliques, dans lesquels "halo" représente un atome de fluor, de chlore ou de brome, étant entendu que les composés (I') peuvent exister sous toutes leurs formes stéréoisomères possibles de même que les copules acides et alcools des esters pyréthrinoïdes ci-dessus.

Les associations selon l'invention présentent notamment l'interêt soit de permettre de combattre, par la polyvalence de leur action, une gamme de parasites, plus étendue, soit de manifester, dans certains cas, un effet de synergie.

L'invention a également pour objet un procédé de préparation des composés de formule (I'), caractérisé en ce que l'on fait réagir un acide de formule (II)

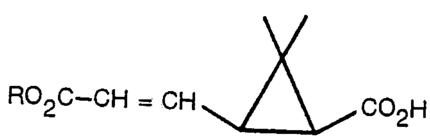

(II)

dans laquelle la copule cyclopropanique est de structure 1R cis et la double liaison de géométrie Z et dans laquelle R conserve la même signification que précédemment, ou un dérivé fonctionnel de cet acide, avec un alcool de formule (III)

A'OH (III)

dans laquelle A' conserve la même signification que précédemment, pour obtenir le composé de formule (I') correspondant.

Le dérivé fonctionnel d'acide utilisé est de préférence un chlorure d'acide.

Lorsque l'on fait réagir l'acide de formule (II) sur l'alcool de formule (III) on opère de préférence en présence de dicyclohexylcarbodiimide.

L'invention a également pour objet un procédé de préparation tel que défini précédemment, caractérisé en ce que le composé de formule (II) est préparé en soumettant au sein d'un solvant organique, un composé de formule ($B_1$)

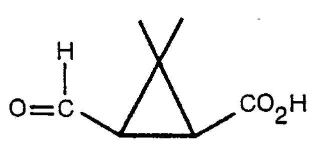

($B_1$)

sous forme de lactone cis, à l'action d'un composé de formule ($B_2$)

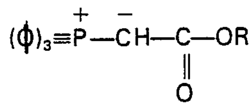

(B$_2$)

dans laquelle R conserve sa signification précédente, pour obtenir le composé de formule (II) correspondant

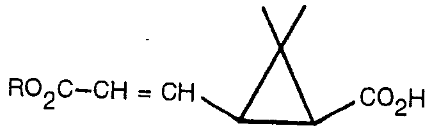

(II)

dans laquelle la copule cyclopropanique est de structure 1R cis sous forme d'un mélange d'isomères E et Z, que l'on sépare, si désiré, en chacun des isomères.

Dans un mode de réalisation préférée, le solvant utilisé est choisi dans le groupe constitué par l'éther éthylique, le diméthylsulfoxyde, le diméthylformamide, le tétrahydrofurane, le diméthoxyéthane, les alcanols, l'éther monométhylique du diéthylèneglycol et l'éther diéthylique du diéthylèneglycol.

Le composé de formule (B$_2$) est préparé par action d'un composé de formule

$$(\phi)_3\equiv\overset{\oplus}{P}-CH_2-CO_2R,\ \overset{\ominus}{Hal}$$

Hal représentant un anion halogénure, sur une base forte. Comme base forte on peut utiliser par exemple un hydrure, un amidure, ou un alcoolate alcalin, ou un alcoyllithien.

L'invention a également pour objet un procédé de préparation tel que défini précédemment, caractérisé en ce que le composé de formule (II) est préparé en faisant réagir un composé de formule (IV)

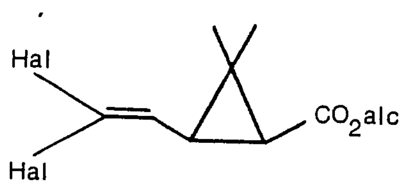

(IV)

dans laquelle la copule cyclopropanique est de structure 1R cis et dans laquelle Hal représente un atome d'halogène et alc représente un radical alcoyle renfermant de 1 à 20 atomes de carbone, dans un premier temps avec un agent alcalin capable d'arracher les atomes d'halogène puis, dans un deuxième temps,

*soit* avec un agent capable d'introduire le groupement carboxylique pour obtenir le composé de formule (V)

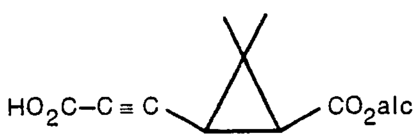

(V)

dans laquelle la copule cyclopropanique est de structure 1R cis que l'on soumet à l'action d'un agent d'estérification, pour obtenir un composé de formule (VI)

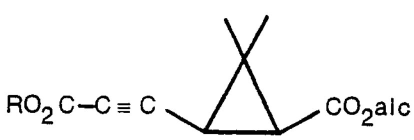

(VI)

dans laquelle la copule cyclopropanique est de structure 1R cis et dans laquelle R conserve la même signification que précédemment

*soit* avec un dérivé de formule

$$Hal-CO_2-R$$

dans laquelle Hal représente un atome d'halogène et R conserve sa signification précédente, pour obtenir directement le composé de formule (VI)

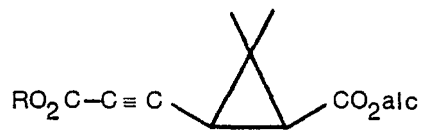

(VI)

dans laquelle la copule cyclopropanique est de structure 1R cis puis soumet le composé de formule (VI) à l'action d'un agent d'hydrogénation ménagée, pour obtenir le composé de formule (VII)

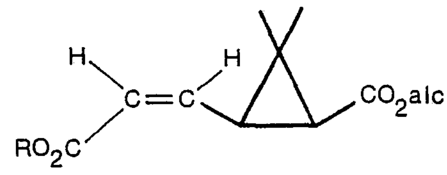

(VII)

dans laquelle la copule cyclopropanique est de structure 1R cis et la double liaison de géométrie Z, que l'on soumet à l'action d'un agent d'hydrolyse acide capable de cliver sélectivement la fonction ester sur le carbone en 1 du cyclopropane, pour obtenir le composé de formule (II) correspondant.

Dans un mode de réalisation préférée du procédé ci-dessus,

Hal représente un atome de brome ou de chlore,

alc représente un radical terbutyle

l'agent alcalin capable d'arracher les halogènes vinyliques est le butyllithium,

l'agent capable d'introduire le groupement carboxylique est le gaz carbonique,

l'agent d'hydrogénation ménagée est l'hydrogène en présence d'un catalyseur comme le palladium en présence de traces de quinoléïne,

l'agent d'hydrolyse acide capable de cliver sélectivement la fonction ester $CO_2$alc est l'acide paratoluène sulfonique.

Le procédé comporte également une variante évidente pour le chimiste dans laquelle le composé de formule (V) est soumis d'abord à l'action d'un agent d'hydrogénation menagée puis dans un deuxième temps à l'action d'un agent de réduction.

L'invention a donc également pour objet une variante du procédé tel que défini précédemment, caractérisée en ce que l'on soumet d'abord le composé de formule (V) à l'action d'un agent d'hydrogénation ménagée pour obtenir le composé de formule (VIII)

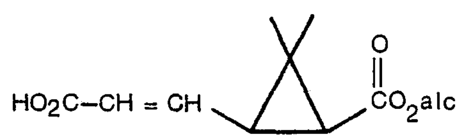

(VIII)

dans laquelle la copule cyclopropanique est de structure 1R cis et la double liaison de géométrie Z que l'on soumet à l'action d'un agent d'estérification, pour obtenir le composé de formule (VII) correspondant

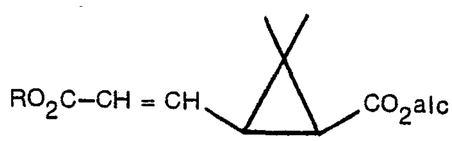

(VII)

dans laquelle la copule cyclopropanique est de structure 1R cis et la double liaison de géométrie Z et dans laquelle R conserve sa signification précédente, puis poursuit la synthèse comme décrit précédemment.

Le procédé ci-dessus comporte une seconde variante évidente, dans laquelle l'ordre de certains stades est modfié.

L'invention a donc également pour objet un procédé tel que défini précédemment, caractérisé en ce que l'on soumet un composé de formule (VI)

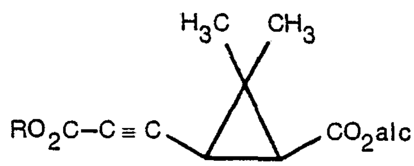

(VI)

dans laquelle la copule cyclopropanique est de structure 1R cis et dans laquelle R et alc, sont définis comme précédemment, à l'action d'un agent d'hydrolyse acide capable de cliver sélectivement la fonction ester en 1 du cyclopropane, pour obtenir le composé de formule (IX)

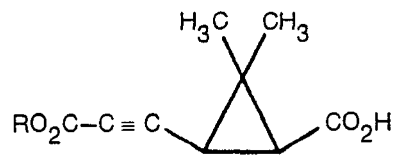

(IX)

dans laquelle la copule cyclopropanique est de structure 1R cis et dans laquelle R est défini comme précédemment, que

*soit l'on soumet*, le case échéant sous forme d'un dérivé fonctionnel, à l'action d'un alcool de formule (III)

A'—OH (III)

dans laquelle A' conserve la même signification que précédemment, pour obtenir le composé de formule (X)

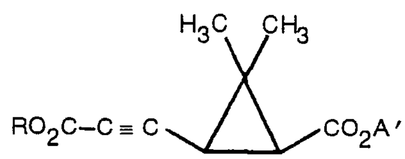

(X)

dans laquelle la copule cyclopropanique est de structure 1R cis et dans laquelle R et A' conservent la même signification que précédemment, que l'on soumet à l'action d'un agent d'hydrogénation ménagée, pour obtenir le composé de formule (I'),

*soit l'on soumet* d'abord à l'action d'un agent d'hydrogénation ménagée, pour obtenir le composé de formule (II)

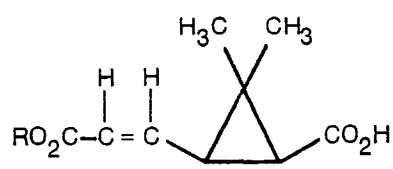

(II)

dans laquelle la copule cyclopropanique est de structure 1R cis et la double liaison de géométrie Z, et dans laquelle R est défini comme précédemment puis, le cas échéant sous forme d'un dérivé fonctionnel, à l'action d'un alcool (III), pour obtenir le composé de formule (I').

Les conditions préférentielles d'exécution du procédé ci-dessus dont identiques à celles qui ont été définies précédemment pour les opérations analogues.

L'invention a également pour objet un procédé de préparation des composés de formule (I'), caractérisé en ce que l'on soumet un composé de formule (XI)

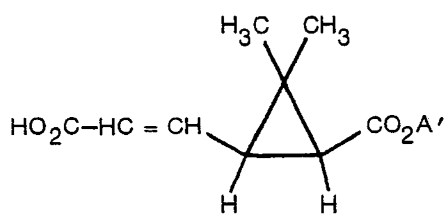

(XI)

dans laquelle la copule cyclopropanique est de structure 1R cis et la double liaison de géométrie Z, à l'action d'un agent d'estérification, pour obtenir le composé de formule (I') correspondant.

Dans un mode de réalisation préféré du procédé ci-dessus, l'estérification est effectuée avec un dérivé fonctionnel d'alcool, à savoir un dérivé de la N,N' diisopropylurée de formule

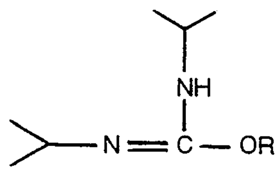

L'invention a également pour objet un procédé tel que défini précédemment, caractérisé en ce que le produit de formule (XI) est préparé en soumettant un acide de formule (V)

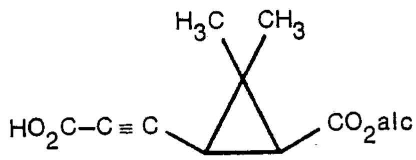

(V)

dans laquelle la copule cyclopropanique est de structure 1R cis et dans laquelle alc représente un radical alcoyle renfermant de 1 à 8 atomes de carbone, à l'action du 2,2,2-trichloroéthanol pour obtenir le composé de formule (XII)

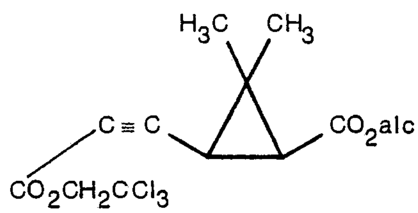

(XII)

dans laquelle la copule cyclopropanique est de structure 1R cis, que l'on soumet à l'action d'un agent d'hydrolyse acide pour obtenir le composé de formule (XIII)

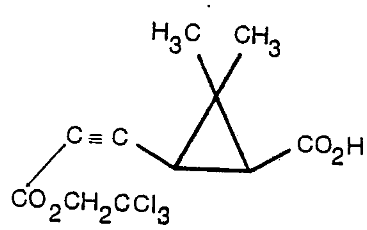

(XIII)

dans laquelle la copule cyclopropanique est de structure 1R cis que l'on soumet à l'action d'un alcool de formule (III)

$$A'\text{—}OH \qquad (III)$$

dans laquelle A' conserve la même signification que précédemment, pour obtenir le composé de formule (XIV)

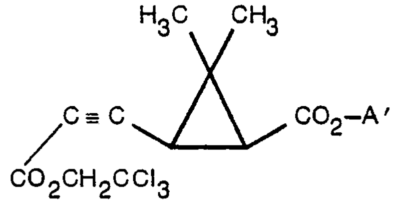

(XIV)

dans laquelle la copule cyclopropanique est de structure 1R cis que l'on soumet à l'action d'un agent de clivage de la fonction ester porté par le carbone acétylénique, pour obtenir le composé de formule (XV)

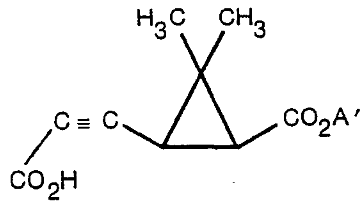

(XV)

dans laquelle la copule cyclopropanique est de structure 1R cis que l'on soumet à l'action d'un agent d'hydrogénation ménagée pour obtenir le composé de formule (XI).

Dans un mode de réalisation préféré du procédé de l'invention ci-dessus,

alc dans la formule (V) représente un radical terbutyle,

l'agent d'hydrolyse acide est l'acide paratoluène sulfonique;

l'estérification du composé (XIII) se fait en faisant réagir le composé (XIII) avec l'alcool (III), en présence de dicyclohexyl carbodiimide ou de diisopropyl carbodiimide;

le clivage de l'ester (XIV) se fait en utilisant une poudre métallique, par exemple la poudre de zinc, en milieu acide;

l'agent d'hydrogénation ménagée est l'hydrogène en présence d'un catalyseur comme le palladium, en présence de traces de quinoléïne.

Le procédé ci-dessus comporte une variante évidente selon laquelle les stades d'hydrogénation et d'estérification sont inversés.

L'invention a ainsi également pour objet un procédé tel que défini précédemment, caractérisé en ce que l'on soumet un composé de formule (XV)

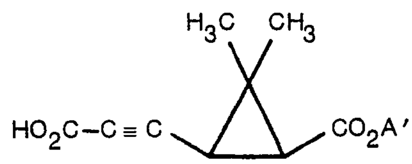

(XV)

dans laquelle la copule cyclopropanique est de structure 1R cis et dans laquelle A' est défini comme précédemment, à l'action d'un agent d'estérification, pour obtenir le composé de formule (X)

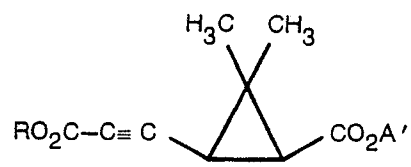

(X)

dans laquelle la copule cyclopropanique est de structure 1R cis et dans laquelle R et A' sont définis comme précédemment, que l'on soumet à l'action d'un agent d'hydrogénation ménagée, pour obtenir le composé de formule (I').

Les conditions préférentielles d'exécution du procédé ci-dessus sont identiques à celles qui ont été définies précédemment pour les opérations analogues.

Pour le cas où l'on désire préparer un composé de formule (I') dans laquelle R représente un radical alcoyle substitué par un ou plusieurs radicaux hydroxy, on prépare d'abord, selon l'un quelconque des procédés ci-dessus, un composé de formule (I') dans laquelle R représente un radical alcoyle substitué par un ou plusieurs radicaux hydroxy protégés, par exemple par un groupement dioxolanyl ou tétrahydropyranyl, puis on hydrolyse ledit composé à l'aide d'un agent d'hydrolyse acide.

L'agent d'hydrolyse acide utilisé dans le procédé ci-dessus peut être, par exemple, l'acide chlorhydrique ou l'acide paratoluène sulfonique.

La plupart des procédés qui viennent d'être décrits ci-dessus, conduisent à des composés dans lesquels la double liaison a la géométrie Z. Ce sont naturellement ces procédés qui sont les plus adaptés pour la préparation des composés de formule (I') dans lesquels la double liaison a la géométrie Z.

D'excellents rendements sont obtenus comme le montre clairement la partie expérimentale exposée ci-après.

Les composés (II), (VI) et (VII), ainsi que les composés (IX) et (X), obtenus lors de la mise en oeuvre du procédé de l'invention sont des produits chimiques nouveaux. L'invention a donc également pour objet ces produits, à titre de produits chimiques nouveaux, et plus particulièrement, à titre de produits intermédiaires nécessaires à la préparation des composés de formule (I') tels que définis précédemment.

Exemple 1:

(1R cis) 2,2-diméthyl 3/(Z) 3-(2,2,2-trifluoroéthoxy) 3-oxo 1-propényl/ cyclopropane carboxylate de (S) α-cyano 3-phénoxy benzyle.

On mélange sous agitation 1,3 g d'acide (1R cis) 2,2-diméthyl 3/(Z) 3-oxo 3-(2,2,2-trifluoroéthoxy) 1-propenyl/ cyclopropane carboxylique, 0,1 cm3 de pyridine et 15 cm3 de chlorure de méthylène puis l'on ajoute 1,05 g de dicyclohexylcarbodiimide. On ajoute ensuite 1,35 g de (S) α-hydroxy 3-phénoxy benzène acétonitrile en solution dans 5 cm3 de chlorure de méthylène. On agite 5 heures à la température ambiante, filtre l'insoluble et rince au chlorure de méthylène. On additionne le filtrat d'acide chlorhydrique 2N. On décante, lave à l'eau, sèche et amène à sec. On purifie l'huile obtenue par chromatographie sur silice (éluant: cyclohexane-acétate d'éthyle 95—5). On obtient ainsi 1,33 g du produit recherché.

$\alpha_D = + 42° \mp 2°$ (c = 0,7 benzène)

RMN $CDCl_3$: ppm

1,26 et 1,28 H des méthyles en 2,
1,97—2,11 H du carbone en 1 du cyclopropane,
3,1 à 3,4 H du carbone en 3 du cyclopropane,
6,5 à 6,9 H du carbone en 1 du radical propenyl,
5,9—5,93 H du carbone en 2 du radical propenyl,
6,3 H du carbone portant le groupement CN,
4,3 à 4,7 H du radical trifluoroéthoxy.

Dans l'exemple 1 l'acide utilisé est préparé comme suit:

Préparation I:

Acide (1R cis) 2,2-diméthyl 3/(Z) 3-(2,2,2-trifluoroéthoxy) 3-oxo 1-propenyl/ cyclopropane carboxylique.

*Stade A: (1R cis) 2,2-diméthyl-3-(3-hydroxy 3-oxo 1-propynyl) cyclopropane carboxylate du 1,1-diméthyléthyle.*

On introduit 26 g de (1R cis) 2,2-diméthyl 3-(2,2-dibromovinyl)cyclopropane carboxylate de 1,1-diméthyléthyl dans 175 cm3 de tétrahydrofurane anhydre. On ajoute ensuite à −65°C 60 cm3 d'une solution de butyl lithium à 20% dans le cyclohexane. On agite 1 heure à − 60°C puis fait barboter un courant de gaz carbonique pendant une heure et demie, verse le mélange réactionnel dans l'eau glacée additionnée de soude N. On lave à l'éther. La phase aqueuse alcaline est acidifiée à pH 4 et extraite à l'éther. On sèche les phases organiques, amène à sec sous pression réduite. On obtient ainsi un produit que l'on recristallise dans l'éther de pétrole (Eb 60—80°C). On obtient alors 8,3 g du produit recherché fondant à 144°C.

RMN $CDCl_3$: ppm

1,22 et 1,37: protons des méthyles en 2 du cyclopropane,
1,78: proton en 1 et 3 du cyclopropane,
1,47: protons du terbutyle,
8,25: proton du groupement

—C—OH
‖
O

*Stade B: (1R cis) 2,2-diméthyl-3-/3-oxo-3-(2,2,2-trifluoroéthoxy) 1-propynyl/ cyclopropane carboxylate de 1,1-diméthyl éthyle.*

On introduit 4 g du produit préparé au stade A, 3,5 g de dicyclohexylcarbodiimide, dans une solution renfermant 20 cm3 de chlorure de méthylène et 1 cm3 de pyridine. On maintient sous agitation le mélange réactionnel pendant une heure. On ajoute ensuite 2,15 g de trifluoroéthanol et 5 cm3 de chlorure de méthylène. On maintient sous agitation à 20°C pendant 16 heures. On filtre, et rince au chlorure de méthylène. On amène le filtrat à sec. On reprend à l'éther sulfurique, lave à l'acide chlorhydrique N puis à l'eau et sèche. On concentre et isole 5 g d'un produit que l'on chromatographie sur silice (éluant-benzène-acétate d'éthyle (95—5). On obtient ainsi 3,5 g de produit recherché.

RMN $CDCl_3$: ppm

1,2 et 1,37 H des méthyles en 2,
1,77 H des carbones en 1 et 3 du cyclopropane,
1,43 H des méthyles du radical 1,1-diméthyléthyle,
4,3 à 4,7 H du radical trifluoroéthoxy.

*Stade C: Acide (1R cis) 2,2-diméthyl 3/3-oxo-3-(2,2,2-trifluoroéthoxy-1-propynyl/ cyclopropane carboxylique.*

On porte au reflux un mélange renfermant 3,3 g du produit préparé au stade précédent, 30 cm3 de toluène et 100 mg d'acide paratoluène sulfonique. On maintient au reflux jusqu'à la fin du dégagement gazeux. On refroidit, lave à l'eau, sèche et amène à sec. On obtient ainsi 2,6 g du produit recherché que l'on utilise tel quel dans le stade suivant.

*Stade D: Acide (1R cis) 2,2-diméthyl 3/(Z)-3-oxo-3-(2,2,2-trifluoroéthoxy) 1-propényl/ cyclopropane carboxylique.*

On place dans un ballon relié à un appareil à hydrogène 500 mg d'hydroxyde de palladium à 10% sur sulfate de baryum, et 5 cm3 d'acétate d'éthyle. On ajoute 2 g du produit préparé au stade précédent, 45 cm3 d'acétate d'éthyle et 0,5 cm3 de quinoléïne. On hydrogène jusqu'à la fin d'absorbtion. On filtre le produit obtenu. On lave le filtrat à l'acide chlorhydrique N puis à l'eau, et amène à sec. On obtient 2 g d'un produit que l'on chromatographie sur silice (éluant cyclohexane, acétate d'éthyle, acide acétique (70—30—1). On obtient ainsi 1,3 g du produit recherché.

Spectre RMN $CDCl_3$ ppm

1,3 et 1,32 H des méthyles en 2,
1,92—2,06 H du carbone en 1 du cyclopropane,
3,07 à 3,38 H du carbone en 3 du cyclopropane,
6,6 à 6,9 H du carbone en 1 du radical propenyle,
5,9—6,0 H du carbone en 2 du radical propenyle,
4,3 à 4,7 H du radical trifluoroéthoxy.

Exemple 2:

(1R cis) 2,2-diméthyl-3-/(Z)-3-oxo-3-(2,2,2-trifluoroéthoxy) 1-propényl/ cyclopropane carboxylate de (1S) 2-diméthyl-4-oxo-3-(2-propényl) 2-cyclopenten-1-yle.

On mélange 1,9 g d'acide (1R cis) 2,2-diméthyl 3/(Z)-3-oxo-3-(2,2,2-trifluoroéthoxy) 1-propényl/ cyclopropane carboxylique, 12 cm3 de chlorure de méthylène et 100 mg de diméthylamino pyridine. On introduit ensuite 1,4 g de dicyclohexylcarbodiimide, puis 1,1 g de (S) 3-(2-propényl) 1-hydroxy 2-méthyl 4-oxo cyclopent-2-ène et 5 cm3 de chlorure de méthylène. On maintient sous agitation à la température ambiante pendant 2 heures. On élimine par filtration l'insoluble formé. On lave le filtrat à l'acide chlorhydrique 0,5 N, puis à l'eau, le sèche, et l'amène à sec. On obtient ainsi 3 g d'un produit que l'on chromatographie sur silice (éluant benzène-acétate d'éthyle (95—5)).

On obtient ainsi 2,2 g du produit recherché.

$\alpha_D = +38° \mp 2,5°$ (c = 0,5% benzène)

Spectre RMN $CDCl_3$ ppm

1,29 et 1,32 H des méthyles en 2 du cyclopropane,
1,97—2,11 H en 1 du cyclopropane,
3,05 à 3,37 H en 3 du cyclopropane,
6,7 à 7 H du carbone en 1 du radical propényle,
5,9 } 6,1 } H du carbone en 2 du radical propényle,
4,3 à 4,75 H du radical trifluoroéthoxy,
5,7 H du cyclopentène en α du $CO_2$,
2 H du méthyle porté par le cyclopentène,
4,8 à 5,25 H en 3 du propényle porté par le cyclopentène.

Exemple 3:

(1R cis) 2,2-diméthyl 3/(Z)-3-oxo 3-(phénylméthoxy) 1-propényl/ cyclopropane carboxylate de (1S) α cyano-3-phénoxy benzyle.

*Stade A: Chlorure de l'acide (1R cis) 2,2-diméthyl 3-/(Z) 3-oxo-3-(phénylméthoxy) 1-propényl/ cyclopropane carboxylique.*

On agite 5 heures sous courant d'azote, un mélange renfermant 1,6 g d'acide (1R cis) 2,2-diméthyl 3/(Z) 3-oxo 3-(phénylméthoxy) 1-propényl/ cyclopropane carboxylique, 10 cm3 d'isoprène et 1 cm3 de chlorure de thionyle, concentre et obtient ainsi 2 g d'un produit que l'on utilise tel quel dans le stade suivant.

*Stade B: (1R cis) 2,2-diméthyl-3-/(Z)-3-oxo 3-(phénylméthoxy) 1-propényl/ cyclopropane carboxylate de (1S) α cyano-3-phénoxy benzyle.*

On introduit 1 g du produit préparé au stade A dans une solution renfermant 700 mg de (S) α-hydroxy 3-phénoxy benzène acétonitrile, 20 cm3 de benzène et 0,6 cm3 de pyridine. On maintient le mélange réactionnel sous agitation pendant 16 heures, à la température ambiante, verse sur un mélange d'eau glacée et d'acide chlorhydrique N. On agite la suspension obtenue et extrait au benzène. On lave les extraits benzéniques, à l'eau, séche, filtre et amène à sec. On obtient 1,5 g d'un produit que l'on chromatographie sur silice (éluant cyclohexane-acétate d'éthyle (8—2)).

On obtient ainsi 861 mg du produit recherché fondant à 83°C.

$\alpha_D = +69° \pm 5°$ (c = 0,2% benzène)

Spectre RMN $CDCl_3$ ppm

1,25 H des méthyles en 2 du cyclopropane,
6,33 H du carbone portant le groupement CN.

Préparation II

Acide (1R cis) 2,2-diméthyl-3-/(Z)-3-oxo-3-(phényl méthoxy)-1-propényl/ cyclopropane carboxylique.

*Stade A: (1R cis) 2,2-diméthyl 3/(Z) 2-carboxy éthényl/ cyclopropane carboxylate de 1,1-diméthyléthyl.*

On hydrogène 2 g de (1R cis) 2,2-diméthyl 3/2-carboxy éthynyl/ cyclopropane carboxylate de 1,1-diméthyléthyl dans 40 cm3 d'acétate d'éthyle en présence de 0,38 g d'hydroxyde de palladium à 10% sur sulfate de baryum et 0,4 cm3 de quinoléïne. On filtre, lave le filtrat à l'acide chlorhydrique 0,5 N, puis à l'eau jusqu'à neutralité, sèche, concentre à sec sous pression réduite et obtient 2 g du produit recherché fondant à 94°C.

*Stade B: (1R cis) 2,2-diméthyl-3-/(Z)-3-oxo-3-(phényl méthoxy)-1-propényl/ cyclopropane carboxylate de 1,1-diméthyléthyle.*

On introduit 2,4 g du produit préparé au stade A dans 20 cm3 d'acétate d'éthyle. On ajoute ensuite 2,34 g d'O-benzyl N,N-diisopropyl isourée (décrit par ESCHIMDT et col Liebig Ann. Chem. 1965 *685* 161.). On agite 16 heures à la température ambiante, filtre et concentre le filtrat sous pression réduite. On obtient 4,3 g d'une huile jaune que l'on chromatographie sur silice (éluant benzène-cyclohexane (7—3)). On obtient ainsi 2 g du produit recherché.

Spectre RMN $CDCl_3$ ppm
1,22 et 1,28 H des méthyles en 2 du cyclopropane,
1,77—1,91 H du carbone en 1 du cyclopropane,
2,98 à 3,3 H du carbone en 3 du cyclopropane,
6,5 à 6,8 H du carbone en 1 du radical propényle,
5,8—6 H du carbone en 2 du radical propényle,
1,43 H des méthyles du radical diméthyléthyle,
5,1 H du méthoxy du radical phénylméthoxy.

*Stade C: Acide (1R cis) 2,2-diméthyl-3-/(Z)-3-oxo-3-(phénylméthoxy) 1-propényl/ cyclopropane carboxylique.*

On porte à 90°C un mélange renfermant 2 g du produit préparé au stade précédent, 30 cm3 de toluène, 100 mg d'acide paratoluène sulfonique. On maintient sous agitation pendant 2 heures environ. On amène à sec et obtient 2 g d'un produit que l'on chromatographie sur silice (éluant: cyclohexaneacétate d'éthyle-acide acétique 60/40/1).

On obtient ainsi 1,4 g du produit recherché.

Spectre RMN $CDCl_3$ ppm
1,25 et 1,3 H des méthyles en 2 du cyclopropane,
1,84—1,98 H du carbone en 1 du cyclopropane,
3,14 à 3,43 H du carbone en 3 du cyclopropane,
6,4 à 6,77 H du carbone en 1 du radical propényle,
5,98 H du carbone en 2 du radical propényle.

Exemple 4:

(1R cis) 2,2-diméthyl 3-/(Z) 3-oxo-3-(phénylméthoxy)-1-propényl/ cyclopropane carboxylate de (1S)-2-méthyl-4-oxo-3(2-propényl) 2-cyclopenten-1-yle.

On introduit 1 g de chlorure d'acide (1R cis) 2,2-diméthyl 3/(Z) 3-oxo-3(phénylméthoxy) 1-propényl/ cyclopropane carboxylique dans un mélange de 450 mg de (S) 3-(2-propényl) 1-hydroxy 2-méthyl 4-oxo cyclopent-2-en-1-yle, 20 cm3 de benzène et 0,6 cm3 de pyridine. On maintient sous agitation pendant 16 heures et verse le mélange réactionnel sur un mélange d'eau glacée et d'acide chlorhydrique N. On extrait au benzène, réunit les phases benzéniques, les lave à l'eau, sèche et amène à sec. On obtient 1,5 g d'un produit que l'on chromatographie sur silice (éluant: cyclohexane-acétate d'éthyle (8—2)).

On obtient ainsi 500 mg du produit recherché.

$\alpha_D = + 37° \pm 2{,}5$ (c = 0,5% benzène)

Spectre RMN $CDCl_3$ ppm
1,27 et 1,31 H des méthyles en 2 du cyclopropane,
1,87—2 H du carbone en 1 du cyclopropane,
3,12 à 3,45 H du carbone en 3 du cyclopropane,
5,8 à 6,8 H en 1 et 2 du radical propényl,
5,2 H du méthoxy du radical phényl méthoxy,
5,6 à 5,7 H du cyclopentène en α du $CO_2$,
2 H du méthyle porté par le cyclopentène,
4,8 à 5,2 H du propényle porté par le cyclopentène.

Exemple 5:

(1R cis) 2,2-diméthyl-3/(Z)-3-oxo-3-phenoxy-1-propényl/ cyclopropane carboxylate de (S) α cyano 3-phénoxy benzyle.

En opérant comme à l'exemple 1, à partir de 1,5 g d'acide (1R cis) 2,2-diméthyl-3/(Z) 3-oxo-3-phénoxy-

1-propényl/ cyclopropane carboxylique et 1,45 g de (S) α-hydroxy 3-phénoxy benzène acétonitrile, on obtient 1,8 g du produit recherché.

$\alpha_D = + 54° \pm 2,5°$ (c = 0,5% dans le benzène)

Spectre RMN $CDCl_3$ ppm

1,25 H en 2 du cyclopropane,
1,97—2,12 H en 1 du cyclopropane,
3,25 à 3,6 H en 3 du cyclopropane,
6,6 à 7 H en 1 du radical propényle,
6,1—6,3 H en 2 du radical propényle,
6,9 à 7,7 H aromatiques du radical 3-phénoxy phényle.

Préparation III:

acide (1R cis) 2,2-diméthyl-3/(Z) 3-oxo-3-phénoxy-1-propényl/ cyclopropane carboxylique.

*Stade A: (1R cis) 2,2-diméthyl-3-/3-oxo-3-phénoxy-1-propynyl/ cyclopropane carboxylate de 1,1-diméthyléthyle.*

On dissout 25 g de (1R cis) 2,2-diméthyl 3-(2',2'-dibromovinyl) cyclopropane carboxylate de 1,1-diméthyl éthyle dans 250 cc de tétrahydrofuran. On introduit ensuite sous agitation à −65°C, 48 cm3 d'une solution à 20% de butyllithium dans le cyclohexane. On maintient l'agitation pendant une heure à −65°C et introduit 9,6 cm3 de chloroformiate de phényle. On maintient à nouveau sous agitation à −65°C, pendant une heure, et laisse revenir à la température ambiante tout en maintenant l'agitation. On verse sur une solution aqueuse saturée de phosphate monosodique, extrait à l'éther, lave à l'eau, sèche et obtient 24,6 g d'une huile que l'on purifie par chromatographie sur silice (éluant: cyclohexane-acétate d'éthyle (9—1)). On isole ainsi 14,4 g du produit recherché.

Spectre RMN $CDCl_3$ ppm

1,23 et 1,42 H des méthyles en 2 du cyclopropane,
1,82 H en 1 et 3 du cyclopropane,
1,5 H du radical diméthyl éthyle,
7 à 7,6 H aromatiques.

*Stade B: (1R cis) 2,2-diméthyl-3-/(Z) 3-oxo-3-phénoxy-1-propényl/ cyclopropane carboxylate de 1,1-diméthyl éthyle.*

En présence de 800 mg d'hydroxyde de palladium sur sulfate de baryum, 0,8 cm3 de quinoléïne et 20 cm3 d'acétate d'éthyle, on hydrogène 4 g du produit préparé au stade A en solution dans 60 cm3 d'acétate d'éthyle, on filtre et ajoute 200 cm3 d'une solution d'acide chlorhydrique 2N. On décante, lave à l'eau et sèche. On obtient 4,1 g d'une huile que l'on purifie sur silice (éluant: cyclohexane-acétate d'éthyle (95—5)). On obtient 3,35 g du produit recherché.

Spectre RMN $CDCl_3$ ppm

1,23 et 1,3 H en 2 du cyclopropane,
1,83—1,97 H en 1 du cyclopropane,
3 à 3,33 H en 3 du cyclopropane,
1,44 H du radical méthyléthyle,
6,7 à 7 H en 1 du radical propényle,
6,03—6,21 H en 2 du radical propényle,
7 à 7,5 H aromatiques.

*Stade C: acide (1R cis) 2,2-diméthyl-3-/(Z) 3-oxo 3-phénoxy 1-propényl/ cyclopropane carboxylique.*

On porte un reflux un mélange de 3,3 g du produit préparé au stade précédent, 35 cm3 de toluène et 100 mg d'acide paratoluène sulfonique monohydraté. On arrête le reflux dès la fin du dégagement gazeux. On amène à sec sous pression réduite et obtient 3,4 g d'un produit que l'on chromatographie sur silice (éluant: cyclohexane-acétate d'éthyle-acide acétique (70—30—1). On obtient 2,4 g du produit recherché fondant à 57°C.

Spectre RMN $CDCl_3$ ppm

1,25 à 1,33 H des méthyles en 2 du cyclopropane,
1,9—2,04 H en 1 du cyclopropane,
3,2 à 3,5 H en 3 du cyclopropane,
6,6 à 6,9 H en 1 du propényle,
6.0 — 6,2 H en 2 du propényle.

Exemple 6:

(1R cis) 2,2-diméthyl-3/(Z)-3-oxo-3-phénoxy-1-propényl/ cyclopropane carboxylate de (1S) 2-méthyl 4-oxo-3-(2-propényl) 2-cyclopenten-1-yle.

En opérant comme à l'exemple 2, à partir de 1,5 g d'acide (1R cis) 2,2-diméthyl-3/(Z)-3-oxo 3-phénoxy-

1-propényl/ cyclopropane carboxylique et 1 g de (S) 3-(2-propényl) 1-hydroxy 2-méthyl 4-oxo cyclopent-2-en-1-yle, on obtient 1,6 g du produit recherché.

$\alpha_D = + 66° \pm 2,5$ (c = 0,5% benzène)

Spectre RMN $CDCl_3$ ppm

1,26 et 1,33 H en 2 du cyclopropane,
1,95—2,09 H en 1 du cyclopropane,
5,7 H du cyclopentène en α du $CO_2$,
4,8 à 5,2 H en 3 du propényle porté par le cyclopentène,
2 H du méthyle porté par le cyclopentène,
6,1 à 6,7 H du propényle porté par le cyclopropane,
7 à 7,7 H aromatiques.

Exemple 7:

(1R cis) 2,2-diméthyl-3/(Z)-3-oxo-3-méthoxy méthoxy 1-propényl/ cyclopropane carboxylate de (RS) α-cyano 3-phénoxy benzyle.

*Stade A: (1R cis) 2,2-diméthyl-3-(3-oxo-3-méthoxy méthoxy 1-propynyl) cyclopropane carboxylate de (RS) α-cyano 3-phénoxy benzyle.*

On refroidit à +10°C une solution de 3 g de (1R cis) 2,2-diméthyl-3-(3-hydroxy 3-oxo 1-propynyl) cyclopropane carboxylate de (RS) α-cyano 3-phénoxy benzyle dans 30 cm3 de diméthylformamide anhydre, ajoute par fraction, 300 mg d'hydrure de sodium à 61% dans l'huile puis en 15 minutes, 2,5 cm3 de solution d'éther chlorométhylique préparée comme ci-dessous. On agite pendant 2 heures, verse sur une solution aqueuse de phosphate monosodique et extrait à l'acétate d'éthyle, lave à l'eau sèche et concentre à sec. On chromatographie le résidu sur silice, élue avec un mélange: cyclohexane-acétate d'éthyle (75/25) et recueille 2 g de produit attendu.

RMN $CDCl_3$ ppm

1,23—1,27 et 1,35—1,45 protons des méthyles en 2 du cyclopropane,
1,95 protons en 1 et 3 du cyclopropane,
5,28 proton du méthylène du méthoxy méthoxyle,
3,5 proton du méthyle du méthoxy méthoxyle,
6,42 et 6,47 proton porté par le même carbone que CN,
6,92 à 7,58 protons aromatiques.

Préparation de la solution d'éther chlorométhylique.

On mélange 4,5 cm3 de méthylal et 0,52 cm3 de méthanol puis ajoute lentement 3,53 cm3 de chlorure d'acétyle. On agite pendant 36 heures à température ambiante pour obtenir la solution attendue.

*Stade B: (1R cis) 2,2-diméthyl-3/(Z)-3-oxo-3-méthoxy méthoxy 1-propényl/ cyclopropane carboxylate de (RS) α-cyano 3-phénoxy benzyle.*

On hydrogène 2,2 g de produit obtenu comme ci-dessus dans 50 cm3 d'acétate d'éthyle en présence de 450 mg d'hydroxyde de palladium à 10% sur sulfate de baryum dans 30 cm3 d'acétate d'éthyle et 0,5 cm3 de quinoléïne. On filtre, lave le filtrat à l'acide chlorhydrique N, à l'eau, sèche et amène à sec. On chromatographie le résidu sur silice, élue par un mélange: cyclohexane-acétate d'éthyle (8—2) et recueille 1,2 g de produit attendu.

$\alpha_D = + 41° \pm 3$ (c = 0,3% $CHCl_3$)

RMN $CDCl_3$ ppm

1,27—1,28 et 1,33—1,35 protons des méthyles en 2 du cyclopropane,
1,93—2,1 proton en 1 du cyclopropane,
3,17 à 3,5 proton en 3 du cyclopropane,
6,47 à 6,82 proton éthylénique en 1,
5,85—6,0 et 5,88—6,1 proton éthylénique en 2,
5,27 et 5,3 proton du $CH_2$ du méthoxy,
3,47 et 3,5 protons du méthyle du méthoxy,
6,4 porté par le même carbone que CN,
6,92 à 7,67 protons aromatiques.

Le (1R cis) 2,2-diméthyl 3-/-3-hydroxy 3-oxo 1-propynyl/ cyclopropane carboxylate de (RS) α-cyano 3-phénoxy benzyle utilisé au début de l'éxemple peut être préparé d'une manière analogue à celle de l'ester (S) décrite plus loin, en utilisant l'alcool (RS) correspondant.

Exemple 8:

(1R cis) 2,2-diméthyl-3-/(Z)-3-oxo 3-cyano méthoxy 1-propényl/ cyclopropane carboxylate de (S) α-cyano 3-phénoxy benzyle.

*Stade A: (1R cis) 2,2-diméthyl-3-/3-oxo 3-cyano méthoxy 1-propynyl/ cyclopropane carboxylate de (RS) α-cyano 3-phénoxy benzyle.*

On opère comme à l'exemple 7 en utilisant 2 cm3 de chloro-acétonitrile; après extraction à l'éther et élution avec un mélange: cyclohéxane-acétate d'éthyle (9—1), on obtient 2,69 g de produit attendu.

*Stade B: (1R cis) 2,2-diméthyl-3-/(Z)-3-oxo 3-cyano méthoxy 1-propényl/ cyclopropane carboxylate de (RS) α-cyano 3-phénoxy benzyle.*

En opérant comme à l'exemple 7 au départ de 2,69 g du produit obtenu précédemment, on obtient 2,02 g de produit attendu après élution avec un mélange: cyclohexane-acétate d'éthyle (9—1).

*Stade C: (1R cis) 2,2-diméthyl-3-/(Z)-3-oxo 3-cyano méthoxy 1-propényl/ cyclopropane carboxylate de (S) α-cyano 3-phénoxy benzyle.*

On chromatographie sur silice 1,4 g du produit ci-dessus, élue au chlorure de méthylène et obtient 0,41 g de produit attendu.

$\alpha_D = + 55° \pm 1,5$ (c = 1% $CHCl_3$)

Exemple 9:

(1R cis) 2,2-diméthyl-3-/(Z)-3-oxo 3-éthoxyéthoxy-1-propényl/ cyclopropane carboxylate de (S) α-cyano 3-phénoxy benzyle.

*Stade A: (1R cis) 2,2-diméthyl-3-/3-oxo-3-éthoxyéthoxy 1-propynyl/ cyclopropane carboxylate de (S) α-cyano 3-phénoxy benzyle.*

On refroidit à 0 — + 5°C, 2 g de (1R cis) 2,2-diméthyl 3-/3-hydroxy 3-oxo 1-propynyl) cyclopropane carboxylate de (S) α-cyano 3-phénoxy benzyle, 20 cm3 de chlorure de méthylène et 0,7 cm3 d'éthoxy éthanol. On ajoute 1,1 g de dicyclohexylcarbodiimide, 5 cm3 de chlorure de méthylène et 15 mg de diméthyl aminopyridine. On agite 1 heure à +5°C et 2 heures à température ambiante. On filtre, concentre à sec le filtrat et chromatographie le résidu sur silice, élue par un mélange: cyclohexane-acétate d'éthyle (75—25) et obtient 1,3 g de produit attendu.

RMN $CDCl_3$ ppm

1,22—1,32 protons des méthyles en 2 du cyclopropane,
1,93 protons en 1 et 3 du cyclopropane,
4,17 à 4,38 protons en 1 du COO—$C\underline{H_2}$—$CH_2$—O
3,55 à 3,73 protons en 2 du COO—$CH_2$—$C\underline{H_2}$—O
6,57 proton porté par le même carbone que CN
7 à 7,67 protons aromatiques
1,08—1,2—1,3 et 1,52 (q) protons de l'éthyle

*Stade B: (1R cis) 2,2-diméthyl 3/(Z)-3-oxo 3-(éthoxy éthoxy) 1-propényl/ cyclopropane carboxylate de (S) α-cyano-3-phénoxy benzyle.*

En opérant comme à l'exemple 7, stade B, à partir de 1,3 g du produit obtenu ci-dessus on obtient 1,0 g de produit attendu.

$\alpha_D = + 37°,5 \pm 2,5$ (c = 0,5% $CHCl_3$)

Exemple 10:

(1R cis) 2,2-diméthyl-3-/(Z)-3-oxo 3(RS) (1-1-1-trifluorométhyl éthoxy) 1-propényl cyclopropane carboxylate de (S) α-cyano 3-phénoxy benzyle.

On opère comme au stade B de l'exemple 7 en utilisant 2,6 g de (1R cis) 2,2-diméthyl 3-/3-oxo 3-((RS) 1,1,1-trifluorométhyl éthoxy) propynyl/ cyclopropane carboxylate de (S) α-cyano 3-phénoxy benzyle. Après élution par un mélange cyclohexane-acétate d'éthyle (9—1) on obtient 2,1 g de produit attendu.

$\alpha_D = + 44° \pm 2$ (c = 0,4% benzène)

Préparation du (1R cis) 2,2-diméthyl 3/3-oxo 3-((RS) 1,1,1-trifluorométhyléthoxy) propynyl/ cyclopropane carboxylate de (S) α-cyano 3-phénoxy benzyle.

On opère comme au stade A de l'exemple 9 en utilisant 4,6 g de 1,1,1-trifluorométhyl éthanol et 3,8 g de (1R cis) 2,2-diméthyl 3-/3-oxo 3-hydroxy propynyl/ cyclopropane carboxylate de (S) α-cyano 3-phénoxy benzyle pour obtenir, après élution par un mélange cyclohexane-acétate d'éthyle (8—2) 2,6 g de produit attendu.

Exemple 11:

(1R cis) 2,2-diméthyl-3-/(Z)-3-oxo 3-(2,2-difluoroéthoxy) propényl/ cyclopropane carboxylate de (S) α-cyano 3-phénoxy benzyle.

*Stade A: (1R cis) 2,2-diméthyl-3-/3-oxo 3-(2,2-difluoroéthoxy) propynyl/ cyclopropane carboxylate de terbutyle.*

En opérant comme au stade A de l'exemple 9 à partir de 5 g de (1R cis) 2,2-diméthyl-3-/3-hydroxy 3-oxo-1-propynyl/ cyclopropane carboxylate de terbutyle et après élution par un mélange n-hexane-éther isopropylique (7—3) on obtient 5,25 g de produit attendu.

IR $CHCl_3$

—C≡C— conj 2232 $cm^{-1}$
C=O ester 1725 $cm^{-1}$
asymétrique 1710 $cm^{-1}$
gem diméthyl $\begin{cases} 1393\ cm^{-1} \\ 1380\ cm^{-1} \end{cases}$
terbutyle 1372 $cm^{-1}$

*Stade B: Acide (1R cis) 2,2-diméthyl-3-/3-oxo 3-(2,2-difluoroéthoxy) 1-propynyl/ cyclopropane carboxylique.*

On chauffe au reflux 5,2 g du produit obtenu ci-dessus, 500 mg d'acide paratoluène sulfonique dans 40 cm3 de toluène pendant 25 minutes. Après refroidissement on ajoute 400 cm3 d'éther, lave à l'eau, sèche la phase organique et concentre à sec pour obtenir, 4,1 g de produit attendu.

*Stade C: (1R cis) 2,2-diméthyl-3-/3-oxo 3-(2,2-difluoroéthoxy) 1-propynyl/ cyclopropane carboxylate de (S) α-cyano 3-phénoxy benzyle.*

En opérant comme au stade A de l'exemple 9 à partir de 4,1 g de l'acide obtenu ci-dessus et 4,5 g d'alcool (S) α-cyano 3-phénoxy benzylique on obtient, après élution par un mélange éther de pétrole (eb 40—70°C) — éther isopropylique (6—4) 4,7 g de produit attendu.

IR $CHCl_3$

OH 3580 $cm^{-1}$

gem di Me { 1392 $cm^{-1}$ ; 1380 $cm^{-1}$ }

—C≡C— conj 2235 $cm^{-1}$

C=O ester 1755 $cm^{-1}$

ester conj 1725 $cm^{-1}$

aromatiques { 1588 $cm^{-1}$ ; 1488 $cm^{-1}$ }

*Stade D: (1R cis), 2,2-diméthyl-3-/(Z)-3-oxo 3-(2,2-difluoroéthoxy) propényl/ cyclopropane carboxylate de (S)* α-cyano 3-phénoxy benzyle.

On hydrogène 4,7 g de produit obtenu ci-dessus de la même manière qu'au stade B de l'exemple 7. Après élution par un mélange n-Hexane — éther isopropylique (7—3) on obtient 3,2 g de produit attendu.

$\alpha_D = + 44° \pm 2,5$ (c = 0,5% $CHCl_3$)

Exemple 12:

(1R cis) 2,2-diméthyl-3-/(Z)-3-oxo-3-(2,2-dichloroéthoxy) 1-propényl/ cyclopropane carboxylate de (S) α-cyano 3-phénoxy benzyle.

*Stade A: (1R cis) 2,2-diméthyl-3-/(Z)-3-oxo-3-(2,2-dichloroéthoxy) 1-propényl/ cyclopropane carboxylate de terbutyle.*

On opère comme à l'exemple 9 stade A à partir de 4,8 g de (1R cis) 2,2-diméthyl-3-/(Z) 3-hydroxy 3-oxo 1-propényl/ cyclopropane carboxylate de terbutyle et 2 cm3 de 2,2-dichloroéthanol. Après élution par un mélange cyclohexane-acétate d'éthyle (9—1) on obtient 5,6 g de produit attendu.

*Stade B: Acide (1R cis) 2,2-diméthyl-3-/(Z)-3-oxo-3-(2,2-dichloroéthoxy) 1-propényl/ cyclopropane carboxylique.*

On opère comme au stade B de l'exemple 11 en partant de 5,6 g du produit ci-dessus et obtient 4,5 g de produit attendu.

*Stade C: (1R cis) 2,2-diméthyl-3-/(Z)-3-oxo-3-(2,2-dichloroéthoxy) 1-propényl/ cyclopropane carboxylate de (S) α-cyano 3-phénoxy benzyle.*

On opère comme au stade A de l'exemple 9 à partir de 3 g du produit obtenu en B et 2,25 g d'alcool de (S) α-cyano 3-phénoxy benzylique. Après élution par les mélanges cyclohexane-acétate d'éthyle (8—3) puis (9—1) on obtient 1,6 g de produit attendu.

$\alpha_D = + 54° \pm 2°$ (c = 1% dans benzène)

Les exemples suivants sont préparés de manière analoque à celle décrite au stade A de l'exemple 9 à partir:

1°) de l'acide (1R cis) 2,2-diméthyl-3-/(Z)-3-oxo 3-(2,2-difluoroéthoxy) 1-propényl/ cyclopropane carboxylique et de l'alcool correspondant.

Exemple 13:

(1R cis) 2,2-diméthyl-3-/(Z)-3-oxo 3-(2,2-difluoroéthoxy) 1-propényl/ cyclopropane carboxylate de (RS) α-cyano 6-phénoxy 2-pyridyl méthyle.

$\alpha_D = + 50,5° \pm 2°$ (c = 0,8% $CHCl_3$)

Exemple 14:

(1R cis) 2,2-diméthyl-3-/(Z)-3-oxo 3-(2,2-difluoroéthoxy) 1-propényl/ cyclopropane carboxylate de (R) α-cyano 3-phénoxy benzyle.

$\alpha_D = + 117°,5 \pm 3°$ (c = 0,6% $CHCl_3$)

Exemple 15:

(1R cis) 2,2-diméthyl-3-/(Z)-3-oxo 3-(2,2-difluoroéthoxy) 1-propényl/ cyclopropane carboxylate de/3-propargyl-2,5-dioxoimidazolidinyl/ méthyle.

$\alpha_D = + 18° \pm 2°$ (c = 1% $CHCl_3$)

Exemple 16:

(1R cis) 2,2-diméthyl-3-/(Z)-3-oxo 3-(2,2-difluoroéthoxy) 1-propényl/ cyclopropane carboxylate de (R) α-éthynyle 3-phénoxy benzyle.

$α_D$ = + 47° ± 1,5 (c = 1% $CHCl_3$)

L'acide (1R cis) 2,2-diméthyl-3-/(Z)-3-oxo 3-(2,2-difluoro éthoxy) 1-propényl/ cyclopropane carboxylique a été préparé d'une manière analogue à celle décrite dans la préparation VI à partir de 2,2-difluoroéthanol.

2°) de l'acide (1R cis) 2,2-diméthyl 3-/(Z)-3-oxo 3-(2-fluoroéthoxy) 1-propényl/ cyclopropane carboxylique et de l'alcool correspondant.

Exemple 17:

(1R cis) 2,2-diméthyl-3-/(Z)-3-oxo 3-(2-fluoroéthoxy) 1-propényl/ cyclopropane carboxylate de (3-propargyl 2,5-dioxoimidazolidinyl) méthyle.

$α_D$ = + 18° ± 2° (c = 1% $CHCl_3$)

Exemple 18:

(1R cis) 2,2-diméthyl-3-/(Z)-3-oxo 3-(2-fluoroéthoxy)-1-propényl/ cyclopropane carboxylate de (RS) α-cyano (6-phénoxy 2-pyridyl) méthyle.

$α_D$ = + 49,5° ± 2,5° (c = 0,5% $CHCl_3$)

Exemple 19:

(1R cis) 2,2-diméthyl-3-/(Z)-3-oxo 3-(2-fluoroéthoxy)-1-propényl/ cyclopropane carboxylate de (R) α-méthyl 3-phénoxy benzyle.

$α_D$ = + 123° ± 1,5 (c = 1% $CHCl_3$)

Exemple 20:

(1R cis) 2,2-diméthyl-3-/(Z)-3-oxo 3-(2-fluoroéthoxy)-1-propényl/ cyclopropane carboxylate de α-éthynyl 3-phénoxy benzyle.

$α_D$ = + 47° ± 1,5° (c = 1% $CHCl_3$)

L'acide (1R cis) 2,2-diméthyl-3-/(Z)-3-oxo 3-(2-fluoro éthoxy) 1-propényl/ cyclopropane carboxylique à été préparé d'une manière analogue à celle décrite dans la préparation VI à partir du 2-fluoro éthanol.

3°) de l'acide (1R cis) 2,2-diméthyl-3-/(Z)-3-oxo 3-(2-(1,1,1,3,3,3-hexafluoro) propoxy) 1-propényl/ cyclopropane carboxylique (préparation VI) et de l'alcool correspondant.

Exemple 21:

(1R cis) 2,2-diméthyl-3-/(Z)-3-oxo 3-(2-(1,1,1,3,3,3-hexafluoro) propoxy) 1-propényl/ cyclopropane carboxylate de (R) α-éthynyl 3-phénoxy benzyle.

$α_D$ = + 31,5° ± 1,5° (c = 1% $CHCl_3$)

Exemple 22:

(1R cis) 2,2-diméthyl-3-/(Z)-3-oxo 3-(2-(1,1,1,3,3,3-hexafluoro) propoxy) 1-propényl/ cyclopropane carboxylate de (R) α-méthyl 3-phénoxy benzyle.

$α_D$ = + 97° ± 2 (c = 1% $CHCl_3$)

Exemple 23:

(1R cis) 2,2-diméthyl-3-/(Z)-3-oxo 3-(2-(1,1,1,3,3,3-hexafluoro) propoxy) 1-propényl/ cyclopropane carboxylate de (S) α-cyano 3-phénoxy benzyle.

$α_D$ = + 23,5° ± 2° (c = 0,5% benzène).

Exemple 24:

(1R cis) 2,2-diméthyl-3-/(Z)-3-oxo 3-(2-(1,1,1,3,3,3-hexafluoro) propoxy) 1-propényl/ cyclopropane carboxylate de 3,4,5,6-tétrahydrophtalimido méthyle.

$α_D$ = − 30° ± 1 (c = 1% $CHCl_3$)

Exemple 25:

(1R cis) 2,2-diméthyl-3-/(Z)-3-oxo 3-(2-(1,1,1,3,3,3-hexafluoro) propoxy) 1-propényl/ cyclopropane carboxylate de (RS) cyano 6-phénoxy 2-pyridyl méthyle.

$α_D$ = + 33,5° ± 2,5° (c = 0,2% $CHCl_3$)

4°) de l'acide (1R cis) 2,2-diméthyl-3-/(Z)-3-oxo 3-(2,2,2-trifluoro éthoxy) 1-propényl/ cyclopropane carboxylique (préparation I) et de l'alcool correspondant.

Exemple 26:

(1R cis) 2,2-diméthyl-3-/(Z)-3-oxo 3-(2,2,2-trifluoro éthoxy) 1-propényl/ cyclopropane carboxylate de (3-propargyl 2,5-dioxoimidazolidinyl) méthyle.

$α_D$ = − 4° ± 1° (c = 1% benzène)

Exemple 27:

(1R cis) 2,2-diméthyl-3-/(Z)-3-oxo 3-(2,2,2-trifluoro éthoxy) 1-propényl/ cyclopropane carboxylate de (R) α-méthyl 3-phénoxy benzyle.

$\alpha_D$ = + 108°,5 ± 2° (c = 1% $CHCl_3$)

Exemple 28:

(1R cis) 2,2-diméthyl-3-/(Z)-3-oxo 3-(2,2,2-trifluoro éthoxy) 1-propényl/ cyclopropane carboxylate de 3,4,5,6-tétrahydrophthalimido méthyle.

$\alpha_D$ = + 2,5° ± 2 (c = 0,5% $CHCl_3$)

Exemple 29:

(1R cis) 2,2-diméthyl-3-/(Z)-3-oxo 3-(2,2,2-trifluoro éthoxy) 1-propényl/ cyclopropane carboxylate de (R) α-éthynyl 3-phénoxy benzyle.

$\alpha_D$ = + 42° ± 1,5 (c = 1% $CHCl_3$)

Exemple 30:

(1R cis) 2,2-diméthyl-3-/(Z)-3-oxo 3-(2,2,2-trifluoro éthoxy) 1-propényl/ cyclopropane carboxylate de (RS) α-cyano 6-phénoxy 2-pyridyl méthyle.

$\alpha_D$ = + 46,5° ± 2° (c = 0,7% $CHCl_3$)

5°) du (1R cis) 2,2-diméthyl-3-/(Z) 3-hydroxy-3-oxo 1-propényl/ cyclopropane carboxylate de (S) α-cyano 3-phénoxy benzyle (préparation VII) et l'alcool correspondant.

Exemple 31:

(1R cis) 2,2-diméthyl-3-/(Z)-3-oxo 3-(2-trichloroéthoxy) 1-propényl/ cyclopropane carboxylate de (S) α-cyano 3-phénoxy benzyle.

$\alpha_D$ = + 42°5 ± 2 (c = 0,5% benzène)

Exemple 32:

(1R cis) 2,2-diméthyl-3-/(Z)-3-oxo 3-(2-chloroéthoxy) 1-propényl/ cyclopropane carboxylate de (S) α-cyano 3-phénoxy benzyle.

$\alpha_D$ = + 39° ± 4 (c = 0,25% benzène)

Exemple 33:

(1R cis) 2,2-diméthyl-3-/(Z)-3-oxo 3-(2-méthoxyéthoxy) 1-propényl/ cyclopropane carboxylate de (S) α-cyano 3-phénoxy benzyle.

$\alpha_D$ = + 37°5 ± 2 (c = 1% $CHCl_3$)

Exemple 34:

(1R cis) 2,2-diméthyl-3-/(Z)-3-oxo 3-(RS) 1-cyano éthoxy) 1-propényl/ cyclopropane carboxylate de (S) α-cyano 3-phénoxy benzyle.

$\alpha_D$ = + 64,5 ± 3° (c = 0,3% $CHCl_3$)

Exemple 35:

(1R cis) 2,2-diméthyl-3-/(Z)-3-oxo 3-(2-fluoroéthoxy) 1-propényl/ cyclopropane carboxylate de (S) α-cyano 3-phénoxy benzyle.

$\alpha_D$ = + 48° (c = 0,25% benzène)

Exemple 36:

(1R cis) 2,2-diméthyl-3-/(Z)-3-oxo 3-phénéthoxy 1-propényl/ cyclopropane carboxylate de (S) α-cyano 3-phénoxy benzyle.

$\alpha_D$ = + 46° ± 2°5 (c = 0,5% benzène)

Exemple 37:

(1R cis) 2,2-diméthyl-3-/(Z)-3-oxo 3-(2,2-diméthyl dioxolanyl 4-(RS)-méthoxy) 1-propényl/ cyclopropane carboxylate de (S) α-cyano 3-phénoxy benzyle.

$\alpha_D$ = + 46° ± 2 (c = 0,75% benzène)

6°) du (1R cis) 2,2-diméthyl-3-/(Z)-3-hydroxy 3-oxo 1-propényl/ cyclopropane carboxylate de (RS) α-cyano 3-phénoxy benzyle et de l'alcool correspondant.

Exemple 38:

(1R cis) 2,2-diméthyl-3-/(Z)-3-oxo 3-(2-diméthylamino éthoxy) 1-propényl/ cyclopropane carboxylate de (RS) α-cyano 3-phénoxy benzyle.

$\alpha_D$ = + 23° ± 3 (c = 0,25% $CHCl_3$)

Le (1R cis) 2,2-diméthyl-3-/(Z)-3-hydroxy 3-oxo 1-propényl/ cyclopropane carboxylate de (RS) α-cyano 3-phénoxy benzyle utilisé au début de l'exemple précédent a été préparé d'une manière analogue à celle de l'ester (S) α-cyano 3-phénoxy benzyle des préparations IV et VII.

7°) du (1R cis) 2,2-diméthyl-3-/(Z)-3-hydroxy 3-oxo 1-propényl/ cyclopropane carboxylate de (R) α-méthyl 3-phénoxy benzyle (préparation VIII) et de l'alcool correspondant.

Exemple 39:

(1R cis) 2,2-diméthyl-3-/(Z)-3-oxo 3-(2-méthoxy éthoxy) 1-propényl/ cyclopropane carboxylate de (R) α-méthyl 3-phénoxy benzyle.

RMN $CDCl_3$ ppm

1,22—1,25 protons des méthyles en 2 du cyclopropane
1,45—1,55 protons du méthyle de l'ester en 1 du cyclopropane
5,7 à 6,0 proton du benzyle
3,42 protons du méthoxyle
4,22 à 4,38 protons en 1 de l'ester en 3 du cyclopropane
3,55 à 3,72 protons en 2 de l'ester en 3 du cyclopropane
5,85—6,05 et 6,32 à 6,8 protons éthyléniques

Exemple 40:

(1R cis) 2,2-diméthyl-3-/(Z)-3-oxo 3-(1-méthoxy 1-trifluorométhyl éthoxy) 1-propényl/ cyclopropane carboxylate de (S) α-cyano 3-phénoxy benzyle.

Sous atmosphère inerte on agite 1,02 g de (1R cis) 2,2-diméthyl-3-/(Z)-3-chloro 3-oxo 1-propényl) cyclopropane carboxylate de (S) α-cyano 3-phénoxy benzyle et 9 cm3 de chlorure de méthylène. On ajoute 1,12 g de 1-méthyl 1-trifluorométhyl éthanol et maintient l'agitation pendant 48 heures à température ambiante. On concentre à sec sous pression réduite, chromatographie le résidu sur silice, élue par un mélange: hexane-éther éthylique (8—2) et obtient 250 mg de produit attendu F ~ 59°C.

$\alpha_D = + 57° \pm 2°$ (c = 0,4% benzène)

Le (1R, cis) 2,2-diméthyl-3-/(Z)-3-chloro 3-oxo 1-propényl/ cyclopropane carboxylate de (S) α-cyano 3-phénoxy benzyle utilisé au départ de l'exemple 40 a été préparé par action du chlorure de thionyle sur le (1R cis) 2,2-diméthyl-3-/(Z)-3-hydroxy-3-oxo 1-propényl/ cyclopropane carboxylate de (S) α-cyano 3-phénoxy benzyle préparation VII.

Exemple 41:

(1R cis) 2,2-diméthyl-3-/(Z)-3-oxo 3-(1-trifluorométhyl 1-méthyl propyloxy) 1-propényl/ cyclopropane carboxylate de (S) α-cyano 3-phénoxy benzyle.

On dissout 900 mg de (1R cis) 2,2-diméthyl-3-/(Z)-3-chloro-3-oxo 1-propényl/ cyclopropane carboxylate de (S) α-cyano 3-phénoxy benzyle dans 3 cm3 de chlorure de méthylène, ajoute 1 cm3 de 1-trifluorométhyl 1-méthyl propanol et agite pendant 16 heures à température ambiante sous atmosphère inerte et à l'abri de l'humidité. Après 3 jours à température ambiante on lave le mélange réactionnel avec une solution aqueuse saturée de bicarbonate de sodium puis à l'eau, sèche et concentre à sec. On chromatographie le résidu sur silice élue par un mélange hexane-éther éthylique (8—2) et obtient 570 mg de produit attendu.

Exemple 42:

(1R cis) 2,2-diméthyl-3-/(Z)-3-oxo 3-(2,3-dihydroxy propyloxy) 1-propényl/ cyclopropane carboxylate de (S) α-cyano 3-phénoxy benzyle.

On chauffe au reflux 4,65 g de (1R cis) 2,2-diméthyl-3-/(Z)-3-oxo 3-(2,2-diméthyl dioxolanyl 4-(RS)-méthoxy) 1-propényl/ cyclopropane carboxylate de (S) α-cyano 3-phénoxy benzyle (exemple 37), 46 cm3 de dioxanne, 9 cm3 d'eau et 0,45 g d'acide paratoluène sulfonique pendant 45 minutes. On élimine la majorité du dioxanne par distillation à 40°C sous pression réduite, reprend le résidu par 150 cm3 de chlorure de méthylène et 25 cm3 d'eau. On agite, décante, lave la phase organique à l'eau, sèche et concentre à sec sous pression réduite. On chromatographie le résidu sur silice, élue par un mélange cyclohexane-acétate d'éthyle (3—7) et obtient 3,85 g de produit attendu.

$\alpha_D = + 53° \pm 2,5°$ (c = 0,5% $CHCl_3$)

Exemple 43:

(1R cis) 2,2-diméthyl-3-/(Z)-3-oxo 3-(2-tétrahydropyranyloxyéthoxy) 1-propényl/ cyclopropane carboxylate de (RS) α-cyano 3-phénoxy benzyle.

*Stade A: (1R cis) 2,2-diméthyl-3-/(Z)-3-oxo 3-(2-tétrahydropyranyloxyéthoxy) 1-propynyl/ cyclopropane carboxylate de (RS) α-cyano 3-phénoxy benzyle.*

On opère de manière analogue à cell décrite au stade A de l'exemple 7, au départ de 2,3 g de (1R cis) 2,2-diméthyl-3-/(Z)-3-hydroxy-3-oxo 1-propynyl/ cyclopropane carboxylate de (RS) α-cyano 3-phénoxy benzyle et de 7,5 g de 1-bromo 2-(2-tétrahydropyranyl) oxy éthane. On obtient 1,8 g de produit attendu après chromatographie sur silice dans le mélange cyclohexane-acétate d'éthyle (75—25).

*Stade B: (1R cis) 2,2-diméthyl-3-/(Z)-3-oxo 3-(2-tétrahydropyranyloxyéthoxy) 1-propényl/ cyclopropane carboxylate de (RS) α-cyano 3-phénoxy benzyle.*

On opère de manière analogue à celle décrite au stade B de l'exemple 7, au départ du produit obtenu au stade A di-dessus. On obtient après purification par chromatographie dans le mélange cyclohexane-acétate d'éthyle (80—20), 1,3 g de produit attendu.

$\alpha_D = + 33° \pm 1°$ (c = 1% $CHCl_3$)

le (1R cis) 2,2-diméthyl-3-/(Z)-3-hydroxy 3-oxo 1-propynyl/ cyclopropane carboxylate de (RS) α-cyano 3-phénoxy benzyle utilisé au début de l'exemple précédent a été préparé d'une manière analogue à celle de l'ester (S) α-cyano 3-phénoxy benzyle de la préparation IV.

Exemple 44:

(1R cis) 2,2-diméthyl-3-/(Z)-3-oxo 3-(2-hydroxyéthoxy) 1-propényl/ cyclopropane carboxylate de (RS) α-cyano 3-phénoxy benzyle.

On mélange 0,85 g de produit obtenu à l'exemple 43, 17 cm3 d'éthanol, 5 cm3 de dioxanne, 1 cm3 d'eau et 4 cm3 d'acide chlorhydrique 2N puis agite à 20°C pendant 3 heures. On ajoute ensuite 1 cm3 de triéthylamine, évapore à sec, reprend par un mélange eau-glace, extrait au chlorure de méthylène, lave l'extrait à l'eau, le sèche et évapore le solvant. On chromatographie le rédidu sur silice en éluant au mélange cyclohexane-acétate d'éthyle (65—35) et obtient 0,65 g de produit attendu.

$\alpha_D = + 42,5° \pm 2,5°$ (c = 0,5% $CHCl_3$)

Les composés suivants peuvent aussi être obtenus selon le procédé de l'invention, au départ des acides et alcools correspondants:

le (1R cis) 2,2-diméthyl-3-/(Z)-3-oxo 3-(1,1,1,3,3,3-hexafluoro propoxy) propényl/ cyclopropane carboxylate de 3-phénoxy benzyle.

$\alpha_D = + 26,5° \pm 2,5°$ (c = 0,5% $CHCl_3$)

le (1R cis) 2,2-diméthyl-3-/(Z)-3-oxo 3-(1,1,1,3,3,3-hexafluoro propoxy) propényl/ cyclopropane carboxylate de (S) α-cyano 3-phénoxy 4-fluoro benzyle.

$\alpha_D = + 27° \pm 2°$ (c = 0,8% benzène).

Préparation IV:

(1R cis) 2,2-diméthyl-3-(3-oxo 3-hydroxy 1-propynyl) cyclopropane carboxylate de (S) α-cyano 3-phénoxy benzyle.

*Stade A: (1R cis) 2,2-diméthyl 3/2-carboxy éthynyl/ cyclopropane carboxylate de terbutyle:*

On introduit 26 g de (1R cis) 2,2-diméthyl 3-(2,2-dibromovinyl) cyclopropane carboxylate de terbutyle dans 175 cm3 de tétrahydrofurane anhydre. On ajoute ensuite à −65°C 60 cm3 d'une solution de butyl lithium à 20% dans le cyclohexane. On agite 1 heure à −60°C puis fait barboter un courant de gaz carbonique pendant 1 heure et demie, verse le mélange réactionnel dans l'eau glacée additionnée de soude N. On lave à l'éther. La phase aqueuse alcaline est acidifiée à pH 4 et extraite à l'éther. On sèche les phases organiques, amène à sec sous pression réduite. On obtient ainsi un produit que l'on recristallise dans l'éther de pétrole (Eb 60—80°C). On obtient alors 8,3 g du produit recherché fondant à 144°C.

RMN $CDCl_3$ ppm

1,22 et 1,37 protons des méthyles en 2 du cyclopropane
1,78 proton en 1 et 3 du cyclopropane
1,47 protons du terbutyle
8,25 proton du groupement

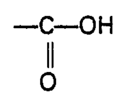

*Stade B: (1R cis) 2,2-diméthyl 3-(2,2,2-trichloroéthoxycarbonyléthynyl) cyclopropane carboxylate de terbutyle.*

On introduit 6,2 g de dicyclohexylcarbodiimide dans une solution renfermant 7,15 g de (1R cis) 2,2-diméthyl 3-(2-carboxyéthynyl) cyclopropane carboxylate de terbutyle et 80 mg de diméthylaminopyridine dans 35 cm3 de chlorure de méthylène. On agite le mélange réactionnel pendant 10 minutes et ajoute 4,5 g de 2,2,2-trichloroéthanol. On maintient sous agitation pendant une heure et élimine par filtration le précipité formé. On lave le filtrat à l'acide chlorhydrique N puis à l'eau jusqu'à neutralité, le sèche et l'amène à sec. On obtient 14 g d'une huile que l'on chromatographie sur silice en éluant par le mélange benzène-acétate d'éthyle (97—3). On isole ainsi 9 g du produit recherché fondant à 70—71°C.

*Stade C: acide (1R cis) 2,2-diméthyl 3-(2,2,2-trichloroéthoxycarbonyl éthynyl) cyclopropane carboxylique.*

On porte au reflux pendant une heure un mélange renfermant 11,4 g du produit préparé selon le stade B, 120 cm3 de toluène et 300 mg d'acide paratoluène sulfonique. On laisse revenir à la température ambiante, lave le mélange réactionnel à l'eau, le sèche, et l'amène à sec. On obtient ainsi 9,5 g du produit recherché que l'on utilise tel quel pour le stade suivant.

*Stade D: (1R cis) 2,2-diméthyl 3-(2,2,2-trichloro éthoxy carbonyl éthynyl) cyclopropane carboxylate de (S) α-cyano 3-phénoxy benzyle.*

On ajoute 6,2 g de dicyclohexylcarbodiimide dans une solution renfermant 9,5 g du produit préparé au stade C, 30 cm3 de chlorure de méthylène et 3 cm3 de pyridine. On agite le mélange réactionnel pendant une demi-heure et ajoute 6,8 g d'alcool (S) α-cyano 3-phénoxy benzylique. On maintient sous agitation pendant une heure et demie. On élimine par filtration, l'insoluble formé. On lave le filtrat à l'acide chlorhydrique N puis à l'eau jusqu'à neutralité. On le sèche, le filtre et l'amène à sec. On obtient 16,3 g d'une huile que l'on chromatographie sur silice en éluant par le mélange benzèneacétate d'éthyle (97—3). On isole ainsi 12 g du produit recherché fondant à 101°C.

*Stade E: (1R cis) 2,2-diméthyl 3-(3-hydroxy 3-oxo 1-propynyl) cyclopropane carboxylate de (S) α-cyano 3-phénoxy benzyle.*

On introduit 5,9 g de poudre de zinc dans une solution renfermant 6,5 g du produit préparé au stade D, 23,4 cm3 d'acide acétique et 2,6 cm3 d'eau. On maintient le mélange sous agitation pendant une heure. On filtre et décante le filtrat. On lave la phase organique à l'eau et extrait la phase aqueuse au chlorure de méthylène. On réunit les solutions chlorométhyléniques, les sèche, les filtre et les amène à sec. On obtient ainsi 4,7 g de produit brut utilisé tel quel.

Préparation V:

(1R cis) 2,2-diméthyl 3-/(Z) (3-hydroxy 3-oxo 1-propényl/ cyclopropane carboxylate de terbutyle:

On hydrogène 2 g de (1R cis) 2,2-diméthyl 3/2-carboxy éthynyl/ cyclopropane carboxylate de terbutyle préparé au stade A de la préparation IV dans 40 cm3 d'acétate d'éthyle en présence de 0,38 g d'hydroxyde de palladium à 10% sur sulfate de baryum et 0,4 cm3 de quinoléïne. On filtre, lave le filtrat à l'acide chlorhydrique 0,5 N puis à l'eau jusqu'à neutralité, sèche, concentre à sec sous pression réduite et obtient 2 g du produit recherché fondant à 94°C.

Préparation VI:

L'acide (1R cis) 2,2-diméthyl 3-/(Z) 3-oxo 3-(-2(1,1,1,3,3,3-hexafluoro) propoxy) 1-propényl/ cyclopropane carboxylique.

*Stade A: (1R cis) 2,2-diméthyl 3-/(Z) 3-oxo 3-(2-(1,1,1,3,3,3-hexafluoro propoxy) 1-propényl/ cyclopropane carboxylate de terbutyle.*

On opère comme au stade A de l'exemple 9, en utilisant 3,6 g de ((1R cis) 2,2-diméthyl 3-/(Z) 3-hydroxy 3-oxo 1-propényl/ cyclopropane carboxylate de terbutyle (préparation V) et 3 g d'hexafluoro isopropanol. Après élution par un mélange: benzène-cyclohexane (4—6) on obtient, 4,9 g de produit attendu F = 92°C.

*Stade B: l'acide (1R cis) 2,2-diméthyl-3-/(Z) 3-oxo 3-(-2(1,1,1,3,3,3-hexafluoro) propoxy) 1-propényl/ cyclopropane carboxylique*

On opère comme à l'exemple 11 stade B, à partir de 4,9 g du produit obtenu ci-dessus et obtient 4,2 g de produit attendu.

IR $CHCl_3$)

OH acide mono + dimère } 3500 $cm^{-1}$

C=O

ester conj { 1755 $cm^{-1}$ épaulement; 1744 $cm^{-1}$ maxi

acide 1695 $cm^{-1}$

C=C conj 1627 $cm^{-1}$

gem. diméthyl 1380 $cm^{-1}$ épaulement

Préparation VII:

(1R cis) 2,2-diméthyl 3/(Z) 3-hydroxy 3-oxo 1-propényl cyclopropane carboxylate de (S) α-cyano 3-phénoxy benzyle.

On hydrogène 4,7 g de (1R cis) 2,2-diméthyl 3-(2-carboxy éthynyl) cyclopropane carboxylate de (S) α-cyano 3-phénoxy benzyle (préparation IV) dans 45 cm3 d'acétate d'éthyle en présence de 500 mg d'hydroxyde de palladium à 10% sur sulfate de baryum et 6,5 cm3 de quinoléïne. On filtre, lave le filtrat à l'acide chlorhydrique N, puis à l'eau jusqu'à neutralité, sèche et amène à sec. On obtient 5,1 g d'une huile que l'on chromatographie sur silice en éluant par le mélange hexane, acétate d'éthyle, acide acétique (70—30—1). On obtient ainsi 3,8 g du produit recherché.

Préparation VIII:

(1R cis) 2,2-diméthyl 3-/(Z) 3-hydroxy 3-oxo 1-propényl/ cyclopropane carboxylate de (R) α-méthyl 3-phénoxy benzyle.

*Stade A: (1R cis) 2,2-diméthyl 3-/3-oxo 3-(2,2,2-trichloro éthoxy) propynyle/ cyclopropane carboxylate de (R) α-méthyl 3-phénoxy benzyle.*

On opère comme au stade D de la préparation IV, en utilisant 6 g d'acide (1R cis) 2,2-diméthyl 3-/3-oxo 3-(2,2,2-trichloroéthoxy) propynyle/ cyclopropane carboxylate et 4,1 g de 1-(R) (-3-phénoxy phényl) éthanol. On obtient après chromatographie dans un mélange cyclohexane-acétate d'éthyle (8—2), 4,38 g de produit attendu.

*Stade B: (1R cis) 2,2-diméthyl 3-/3-oxo 3-hydroxy propynyle/ cyclopropane carboxylate de (R) α-méthyl 3-phénoxy benzyle.*

A 4,16 g de produit obtenu ci-dessus dissous dans 4 cm3 de chlorure de méthylène on ajoute 45 cm3 d'acide acétique à 10% d'eau et 0,53 g de poudre de zinc et agite 30 minutes à température ambiante, ajoute à nouveau 0,53 g de poudre de zinc jusqu'à fin de réaction (4 fois). Après 3 heures de contact on filtre et extrait au chlorure de méthylène. On lave la phase organique à l'eau, sèche, concentre à sec par entrainement azéotropique au toluène et obtient 3,05 g de produit attendu.

*Stade C: (1R cis) 2,2-diméthyl 3-/(Z) 3-hydroxy 3-oxo 1-propényl/ cyclopropane carboxylate de (R) α-méthyl 3-phénoxy benzyle.*

*On opère comme au stade B de l'exemple 7 au départ du produit obtenu au stade B ci-dessus et obtient 2,9 g de produit attendu brut, utilisé tel quel.*

*Exemples de Compositions*

*Exemple A: Préparation d'un concentré soluble.*

*On effectue un mélange homogène de:*

| | |
|---|---|
| *Produit de l'exemple 1* | *0,25 g* |
| *Butoxyde de pipéronyle* | 1 g |
| *Tween 80®* | 0,25 g |
| Topanol A | 0,1 g |
| Eau | 98,4 g |

Exemple B: Préparation d'un concentré émulsifiable.

On mélange intimement:

| | |
|---|---|
| Produit de l'exemple 1 | 0,015 g |
| Butoxyde de pipéronyle | 0,5 g |
| Topanol A | 0,1 g |
| Tween 80® | 3,5 g |
| Xylène | 95,885 g |

Exemple C: Préparation d'un concentré émulsifiable.

On effectue un mélange homogène de:

| | |
|---|---|
| Produit de l'exemple 1 | 1,5 g |
| Tween 80 | 20 g |
| Topanol A | 0,1 g |
| Xylène | 78,4 g |

Exemple D: Préparation d'une composition fumigène.

On mélange d'une façon homogène:

| | |
|---|---|
| Produit de l'exemple 1 | 0,25 g |
| Poudre de tabu | 25 g |
| Poudre de feuilles de cedre | 40 g |
| Poudre de bois de pin | 33,75 g |
| Vert brillant | 0,5 g |
| p-nitrophénol | 0,5 g |

Etude de l'activité des composés selon l'invention sur les parasites.

*1°): Etude de l'effet létal sur mouches domestiques.*

Les insectes tests sont des mouches domestiques femelles âgées de 4/5 jours. On opère par application topique de 1 µl de solution acétonique du produit sur le thorax dorsal des insectes à l'aide du micro manipulateur d'Arnold. On utilise 50 individus par dose et par traitement. On effectue le contrôle de mortalité vingt-quatre heures après traitement.

Le résultat obtenu exprimé en DL 50 ou dose (en nanogrammes) nécessaire pour tuer 50% des insectes, est le suivant:

| Produit de l'exemple | DL 50 (ng par individu) |
|---|---|
| 1 | 1,115 |
| 23 | 0,962 |
| 29 | 0,825 |

Conclusion: sur le test utilisé, les produits des exemples 1, 23 et 29 présentent une activité remarquable.

*2°): Etude de l'effet sur larves de Spodoptera littoralis.*

Les essais sont effectués par application topique d'une solution acétonique du produit à tester à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65% d'humidité relative. Après traitement, les individus sont placés sur un milieu nutritif artificiel (milieu de Poitout).

On effectue le contrôle des mortalités 48 heures après traitement.

Le résultat expérimental obtenu est le suivant:

| Composé de l'exemple | DL 50 (ng par individu) |
|---|---|
| 1 | 4,699 |
| 35 | 0,544 |

Conclusion: sur le test utilisé, les produits des exemples 1 et 35 présentent une bonne activité.

*3°): Etude de l'activité de choc sur mouche domestique.*

Les insectes tests sont des mouches domestiques femelles âgées de 4/5 jours. On opère par pulvérisation directe en chambre de Kearns et March en utilisant comme solvant un mélange en volumes égaux d'acétone et d'isopar L (quantité de solution utilisée 2 × 0,2 cm3). On utilise environ 50 insectes par dose de traitement. On effectue les contrôles toutes les minutes jusqu'à 10 minutes, puis à 15 minutes et l'on détermine le KT 50 par les méthodes habituelles.

Les résultats obtenus sont les suivants:

| Produit de l'exemple | KT 50 en mn | Concentration g/l |
|---|---|---|
| 1 | 3,534 | 1 |
| 2 | 3,608 | 0,25 |
| 26 | 1,550 | 0,25 |
| 30 | 3,498 | 1 |
| 35 | 2,894 | 1 |

Conclusion: sur le test utilisé, les produits des exemples 1, 2, 26, 30 et 35 présentent une bonne activité.

*4°): Etude de l'activité par contact tarsal sur blatte germanique. (composé de l'exemple 1)*

Les insectes testés sont des mâles de blatte germanique (*Blatella germanica*). On opère par dépôt d'une solution acétonique de concentration déterminée sur le fond d'une boîte de Petri de 20 cm de diamètre. Après séchage on laisse séjourner 20 mâles de blattes par concentration durant 1 heure puis on transfère les insectes sur milieu sain et on contrôle leur mortalité à 24 h, 48 h, 3 et 5 jours.

Le résultat exprimé en concentration létale 50 (CL 50) donne, pour le produit considéré, 0,216 mg/m2.
Conclusion: d'après le test utilisé le produit présente une très bonne activité.

*5°): Activité sur Tétranychus urticae.* (composé de l'exemple 1)
*Essai adulticide*

On utilise des plants de haricot comportant deux feuilles cotylédonaires. Ces plants sont traités au pistolet Fisher avec une solution acétonique du produit. Après séchage 25 femelles de l'acarien Tétranychus urticae sont disposées par feuilles soit 50 individus par dose expérimentée par plant. Les contrôles d'efficacité sont effectués après 1, 24, 48 et 72 heures de contact.

A la dose de 2,8 mg/l le produit présente une très bonne activité.

**Revendications pour les Etats contractants: BE CH DE FR GB IT LI LU NL SE**

1. Sous toutes les formes isomères possibles ou sous forme de mélanges, les composés de formule (I')

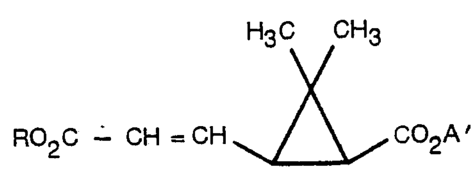

(I')

dans laquelle la copule cyclopropanique est de structure 1R cis et la double liaison de géométrie Z et dans laquelle A' représente

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

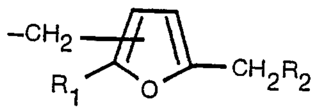

dans lequel le substituant $R_1$ représente un atome d'hydrogène ou un radical méthyle et le substituant $R_2$ un aryle monocyclique ou un groupement $—CH_2—C≡CH$

soit un groupement

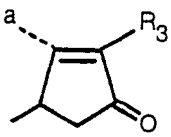

dans lequel a représente un atome d'hydrogène ou un radical méthyle et $R_3$ représente le radical $—CH_2—CH=CH_2$, $—CH_2—CH=CH—CH_3$, $—CH_2—CH=CH—CH=CH_2$, $—CH_2—CH=CH—CH_2—CH_3$,

soit un groupement

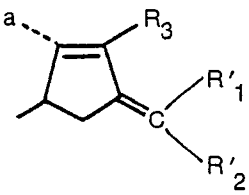

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano.

soit un groupement

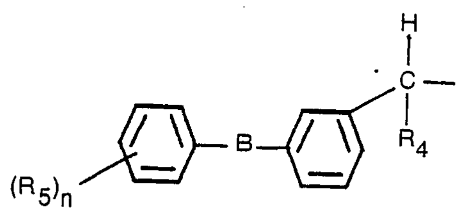

dans lequel B représente un atome d'oxygène ou de soufre ou un groupement

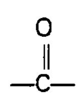

ou $—CH_2—$ et $R_4$ représente un atome d'hydrogène, un radical $—C≡N$, un radical méthyle, un radical $—CONH_2$, un radical $—CSNH_2$ ou un radical $—C≡CH$, $R_5$ représente un atome d'halogène ou un radical méthyle et n représente un nombre égal à 0, 1 ou 2,

soit un groupement

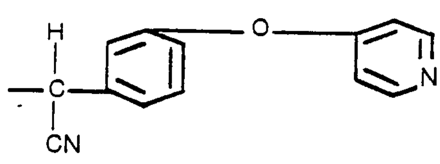

soit un groupement

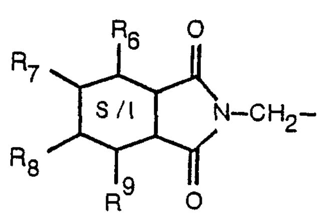

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tètrahydro,

soit un groupement

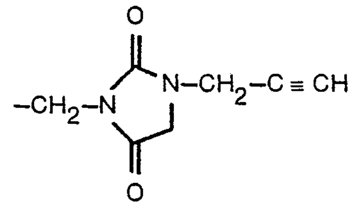

soit un groupement

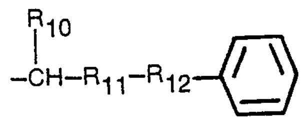

dans lequel $R_{10}$ représente un atome d'hydrogène ou un radical CN, $R_{12}$ représente un radical $—CH_2—$ ou un atome d'oxygène, $R_{11}$ représente un radical thiazolyle ou thiadiazolyle dont la liaison avec

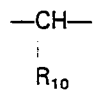

peut se trouver à l'une quelconque des positions disponibles, $R_{12}$ étant lié à $R_{11}$ par l'atome de carbone compris entre l'atome de soufre et un atome d'azote,

soit un groupement

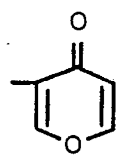

soit un groupement

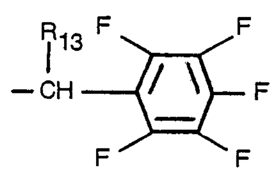

dans lequel $R_{13}$ représente un atome d'hydrogène ou un radical CN,

soit un groupement

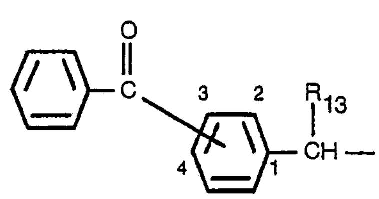

dans lequel $R_{13}$ est défini comme ci-dessus, et le radical benzoyle est en position 3 ou 4,

soit un groupement

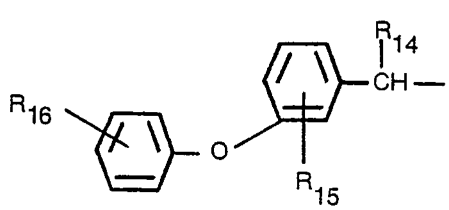

dans lequel $R_{14}$ représente un atome d'hydrogène, un radical méthyle, éthynyle ou cyano et $R_{15}$ et $R_{16}$, différents, représentent un atome d'hydrogène, de flour ou de brome,

soit un groupement

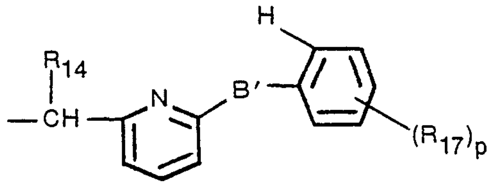

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre et R représente un radical alcoyle renfermant de 1 à 18 atomes de carbone substitué par un ou plusieurs groupements identiques ou différents choisis dans le groupe constitué par les atomes d'halogène, les groupements OH ou SH, les groupements OR' ou SR' dans lesquels R' représente un radical alcoyle renfermant de 1 à 8 atomes de carbone, les groupements $NO_2$ ou

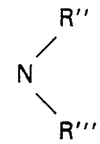

dans lesquels R'' et R''', identiques ou différents, représentent un atome d'hydrogène, ou un radical alcoyle renfermant de 1 à 8 atomes de carbone, les groupements C≡N, $SO_3H$ ou $PO_4H_2$ ou les groupements $COalc_1$, $SO_2alc_2$, ou $SO_3alc_3$ dans lesquels $alc_1$, $alc_2$ et $alc_3$ représentent des radicaux alcoyle renfermant de 1 à 18 atomes de carbone, ou R représente un radical alcoyle renfermant de 1 à 18 atomes de carbone substitué par un radical aryle lui-même éventuellement substitué par un ou plusieurs groupements OH, Oalc ou alc renfermant de 1 à 8 atomes de carbone, par un ou plusieurs groupements $CF_3$, $OCF_3$, $SCF_3$, ou par un groupement (G):

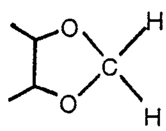

(G)

ou R représente un radical alcoyle renfermant de 1 à 18 atomes de carbone substitué sur deux carbones adjacents par un groupement ($G_1$)

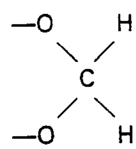

($G_1$)

ou substitué par un groupement

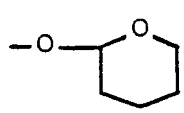

ou R représenté un groupement aryle renfermant de 6 à 14 atomes de carbone éventuellement substitué par un ou plusieurs groupements OH, Oalc ou alc renfermant de 1 à 8 atomes de carbone ou par un groupement $CF_3$, $OCF_3$ ou $SCF_3$, ou R représente un radical pyridinyle, furanyle, thiophényle, oxazolyle ou thiazolyl.

2. Sous toutes les formes isomères possibles ou sous forme de mélanges, les composés de formule (I'), tels que définis à la revendication 1, répondant à la formule (I)

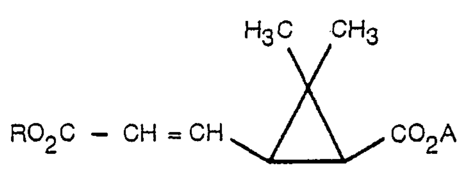

dans laquelle la structure de la copule cyclopropanique est 1R cis et la géométrie de la double liaison est Z et dans laquelle A représente

soit un radical alcoyle renfermant de 1 à 18 atomes de carbone,

soit un radical benzyle éventuellement substitué tel que défini à la revendication 1,

soit un groupement

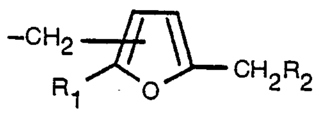

dans lequel les substituants $R_1$ et $R_2$ sont tels que définis à la revendication 1,

soit un groupement

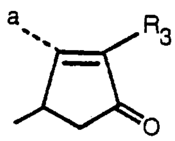

dans lequel a et $R_3$ sont tels que définis à la revendication 1

soit un groupement

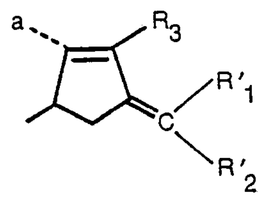

dans lequel a, $R'_1$, $R'_2$ et $R_3$ sont tels que définis à la revendication 1,

soit un groupement

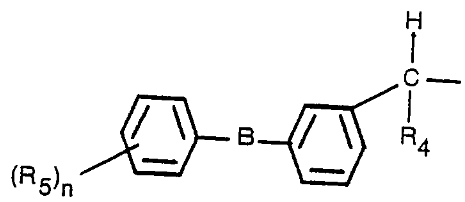

dans lequel B représente un atome d'oxygène, un groupement

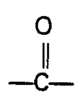

ou $—CH_2—$, $R_4$ est tel que défini à la revendication 1, $R_5$ représente un atome de chlore ou un radical méthyle et n représente un nombre égal à 0, 1 ou 2,

soit un groupement

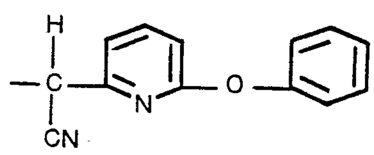

soit un groupement

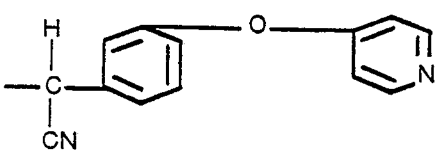

soit un groupement

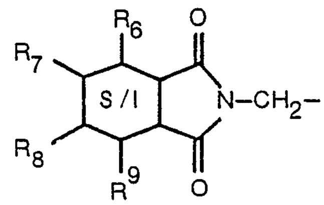

dans lequel les substituants $R_6$, $R_7$, $R_8$, $R_9$ et S/I soit tels que définis à la revendication 1, soit un groupement

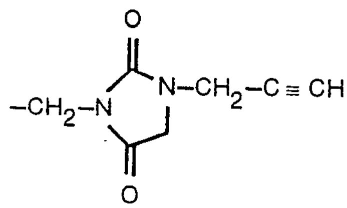

et R est tel que défini à la revendication 1.

3. Les composés de formule (I'), tels que définis à l'une quelconque des revendications 1 et 2, pour lesquels A représente un groupement α-cyano-3-phénoxy benzyle sous forme S, R ou RS.

4. Les composés de formule (I'), tels que définis à l'une quelconque des revendications 1 et 2, pour lesquels A représente un groupement 2-méthyl-4-oxo-3-(2-propényl) 2-cyclopentèn-1-yle, sous forme S, R ou RS.

5. Les composés de formule (I'), tels que définis à l'une quelconque des revendications 1 à 4, pour lesquels R représente un radical alcoyle renfermant de 1 à 18 atomes de carbone substitué par un ou plusieurs groupements fonctionnels mentionnés à la revendication 1.

6. Les composés de formule (I'), tels que définis à la revendication 5, caractérisés en ce que R représente un radical alcoyle substitué par un ou plusieurs atomes d'halogène.

7. Les composés de formule (I'), tels que définis à la revendication 6, caractérisés en ce que R est substitué par un ou plusieurs atomes de fluor.

8. Les composés de formule (I'), tels que définis à la revendication 7, pour lesquels R représente un groupement $CH_2CF_3$.

9. Le composé de formule (I') dont le nom suit:
le (1R cis) 2,2-diméthyl 3/(Z) 3-oxo 3-(2-(1,1,1,3,3,3-hexafluoro)propoxy) 1-propényl/cyclopropane carboxylate de (S) α-cyano 3-phénoxy benzyle.

10. Procédé de préparation des composés de formule (I') tels que définis à l'une quelconque des revendications 1 à 9, caractérisé en ce que l'on fait réagir un acide de formule (II)

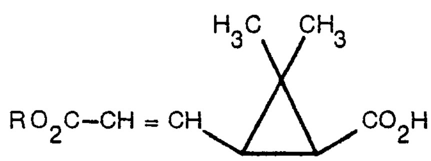

(II)

dans laquelle la copule cyclopropanique est de structure 1R cis et la double liaison de géométrie Z et dans laquelle R conserve la même signification que dans la revendication 1, ou un dérivé fonctionnel de cet acide, avec un alcool de formule (III)

$$A'—OH \qquad (III)$$

dans laquelle A' conserve la même signification que dans la revendication 1, pour obtenir le composé de formule (I') correspondant.

11. Procédé selon la revendication 10, caractérisé en ce que l'on prépare les composés de formule (II) en soumettant au sein d'un solvant organique, un composé de formule ($B_1$)

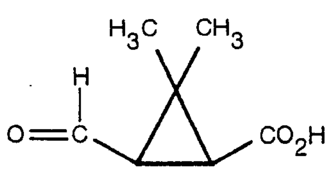

($B_1$)

sous forme de lactone cis, à l'action d'un composé de formule ($B_2$)

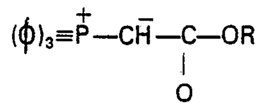

($B_2$)

dans laquelle R conserve sa signification précédente, pour obtenir le composé de formule (II) correspondant

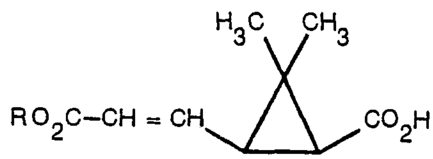

(II)

dans laquelle la copule cyclopropanique est de structure 1R cis sous forme d'un mélange d'isomère E et Z, que l'on sépare, en chacun des isomères.

12. Procédé selon la revendication 11, caractérisé en ce que l'on prépare les composés de formule (II) en faisant réagir un composé de formule (IV)

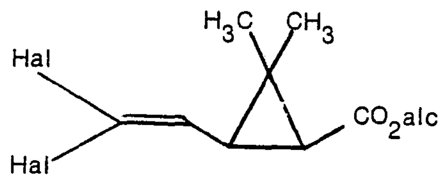

(IV)

dans laquelle la copule cyclopropanique est de structure 1R cis et dans laquelle Hal représente un atome d'halogène et alc représente un radical alcoyle renfermant de 1 à 20 atomes de carbone, dans un premier temps avec un agent alcalin capable d'arracher les atomes d'halogène, puis, dans un deuxième temps, soit avec un agent capable d'introduire le groupement carboxylique pour obtenir le composé de formule (V)

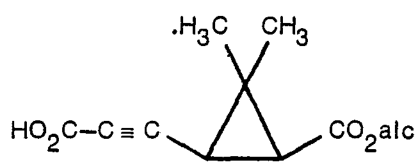

(V)

dans laquelle la copule cyclopropanique est de structure 1R cis que l'on soumet à l'action d'un agent d'estérification pour obtenir le composé de formule (VI)

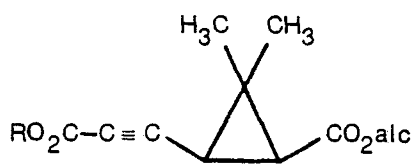

(VI)

dans laquelle la copule cyclopropanique est de structure 1R cis et dans laquelle R conserve la même signification que précédemment,

soit avec un dérivé de formule

$$Hal—CO_2R$$

dans laquelle Hal représente un atome d'halogène et R conserve sa signification précédente, pour obtenir directement le composé de formule (VI)

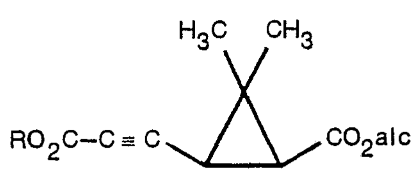

(VI)

dans laquelle la copule cyclopropanique est de structure 1R cis puis soumet le composé de formule (VI) à l'action d'un agent d'hydrogénation ménagée, pour obtenir le composé de formule (VII)

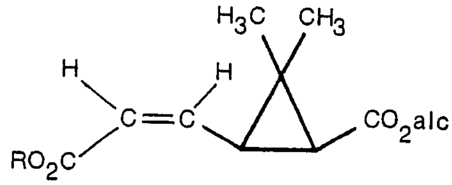 , (VII)

dans laquelle la copule cyclopropanique est de structure 1R cis et dans laquelle la double liaison a la géométrie Z, que l'on soumet à l'action d'un agent d'hydrolyse acide capable de cliver sélectivement la fonction ester sur le carbone en 1 du cyclopropane, pour obtenir le composé de formule (II) correspondant.

13. Procédé selon la revendication 12, caractérisé en ce que l'on soumet d'abord le composé de formule (V) à l'action d'un agent d'hydrogénation ménagée pour obtenir le composé de formule (VIII)

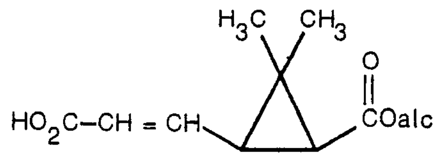 (VIII)

dans laquelle la copule cyclopropanique est de structure 1R cis dans laquelle la double liaison a la géométrie Z, que l'on soumet à l'action d'un agent d'estérification, pour obtenir le composé de formule (VII) correspondant

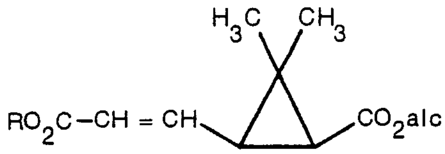 (VII)

dans laquelle la copule cyclopropanique est de structure 1R cis et dans laquelle R conserve sa signification précédente, puis poursuit la synthèse comme décrit à la revendication 12.

14. Procédé selon les revendications 10 et 12, caractérisé en ce que l'on soumet un composé de formule (VI)

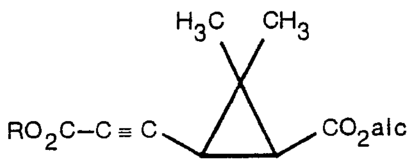 (VI)

dans laquelle la copule cyclopropanique est de structure 1R cis et dans laquelle R et alc, sont définis comme aux revendications 10 et 12, à l'action d'un agent d'hydrolyse acide capable de cliver sélectivement la fonction ester en 1 du cyclopropane, pour obtenir le composé de formule (IX)

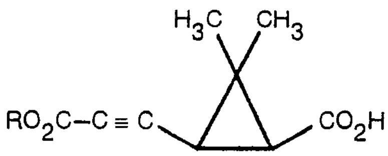 (IX)

dans laquelle la copule cyclopropanique est de structure 1R cis et dans laquelle R est défini comme précédemment, que

soit l'on soumet, le cas échéant sous forme d'un dérivé fonctionnel, à l'action d'un alcool de formule (III)

$$A'—OH \quad (III)$$

dans laquelle A' conserve la même signification qu'à la revendication 1, pour obtenir le composé de formule (X)

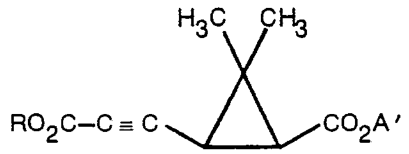

(X)

dans laquelle la copule cyclopropanique est de structure 1R cis et dans laquelle R et A' conservent la même signification que précédemment, que l'on soumet à l'action d'un agent d'hydrogénation ménagée, pour obtenir le composé de formule (I'),

soit l'on soumet d'abord à l'action d'un agent d'hydrogénation ménagée, pour obtenir le composé de formule (II)

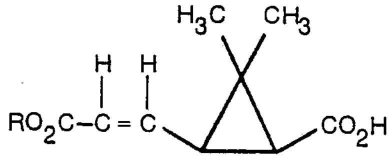

(II)

dans laquelle R est défini comme précédemment, la copule cyclopropanique est de structure 1R cis et la double liaison à la géométrie Z, puis, le cas échéant sous forme d'un dérivé fonctionnel, à l'action d'un alccol (III), pour obtenir le composé de formule (I').

15. Procédé de préparation des composés de formule (I'), telle que définie à la revendication 1, caractérisé en ce que l'on soumet un composé de formule (XI)

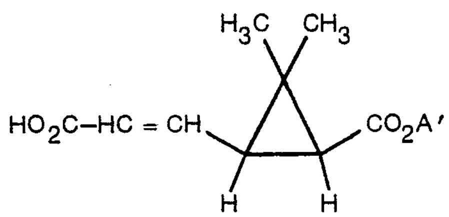

(XI)

dans laquelle la copule cyclopropanique est de structure 1R cis et dans laquelle la double liaison a la géométrie Z, à l'action d'un agent d'estérification, pour obtenir le composé de formule (I') correspondant.

16. Procédé selon la revendication 15, caractérisé en ce que le composé de formule (XI) est préparé en soumettant un acide de formule (V)

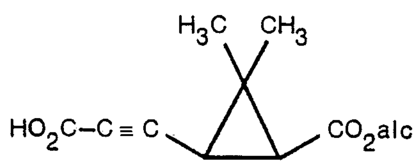

(V)

dans laquelle la copule cyclopropanique est de structure 1R cis et dans laquelle alc représente un radical alcoyle renfermant de 1 à 8 atomes de carbone, à l'action du 2,2,2-trichloroéthanol pour obtenir le composé de formule (XII)

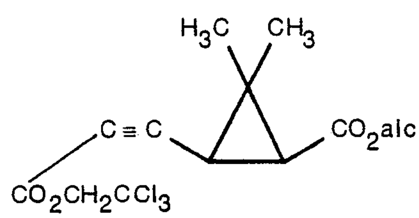

(XII)

dans laquelle la copule cyclopropanique est de structure 1R cis que l'on soumet à l'action d'un agent d'hydrolyse acide pour obtenir le composé de formule (XIII)

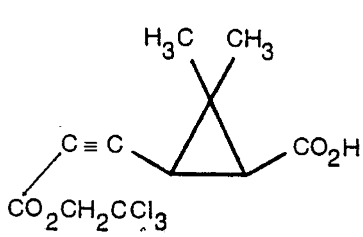

(XIII)

dans laquelle la copule cyclopropanique est de structure 1R cis que l'on soumet à l'action d'un alcool de formule (III)

A'—OH (III)

dans laquelle A' conserve la même signification que dans la revendication 1, pour obtenir le composé de formule (XIV)

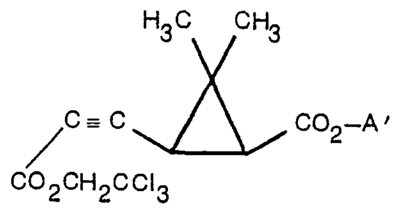

(XIV)

dans laquelle la copule cyclopropanique est de structure 1R cis que l'on soumet à l'action d'un agent de clivage de la fonction ester porté par le carbone acétylénique pour obtenir le composé de formule (XV)

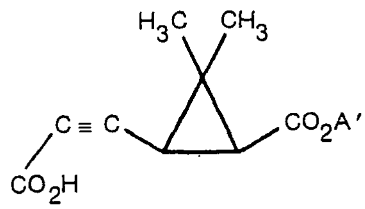

(XV)

dans laquelle la copule cyclopropanique est de structure 1R cis que l'on soumet à l'action d'un agent d'hydrogénation ménagée pour obtenir le composé de formule (XI).

17. Procédé selon les revendications 15 et 16, caractérisé en ce que l'on soumet un composé de formule (XV)

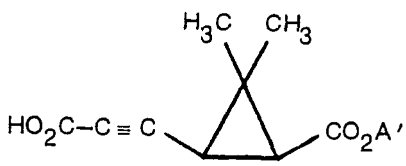

(XV)

dans laquelle la copule cyclopropanique est de structure 1R cis et dans laquelle A' est défini comme à la revendication 1, à l'action d'un agent d'estérification, pour obtenir le composé de formule (X)

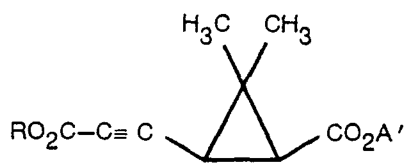

(X)

dans laquelle la copule cyclopropanique est de structure 1R cis et dans laquelle R et A' sont définis comme à la revendication 1, que l'on soumet à l'action d'un agent d'hydrogénation ménagée, pour obtenir le composé de formule (I'), dans laquelle la copule cyclopropanique est de structure 1R cis et la géométrie de la double liaison est Z.

18. Procédé de préparation des composés de formule (I'), dans laquelle R représente un radical alcoyle substitué par un ou plusieurs radicaux hydroxy, selon lequel l'on prépare d'abord par un procédé selon l'une quelconque des revendications 10 à 17, des composés de formule (I') dans laquelle R représente un radical alcoyle substitué par un ou plusieurs radicaux hydroxy protégés, et caractérisé en ce que l'on soument ces composés à l'action d'un agent d'hydrolyse acide.

19. Application des composés de formule (I'), tels que définis à l'une quelconque des revendications 1 à 9, à la lutte contre les parasites des végétaux, les parasites des locaux et les parasites des animaux à sang chaud.

20. Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 9.

21. Les compositions insecticides renfermant comme principe actif au moins l'un des produits définis à l'une quelconque des revendications 1 à 8.

22. Les compositions insecticides renfermant comme principe actif le produit défini à la revendication 9.

23. Les compositions destinées à l'alimentation animale renfermant comme principe actif au moins l'un des produits définis à l'une quelconque des revendications 1 à 9.

24. Associations douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I') peuvent exister sous toutes leurs formes stéréoisomères possibles, de même que les copules acides et alcools des esters pyréthrinoîdes ci-dessus.

25. A titre de médicaments, les composés définis à l'une quelconque des revendications 1 à 9.

26. Les compositions pharmaceutiques renfermant comme principe actif, l'un au moins des médicaments définis à la revendication 25.

27. A titre de produits intermédiaires nécessaires à la preparation des composés de formule (I') tels que définis à la revendication 1, les composés de formule II

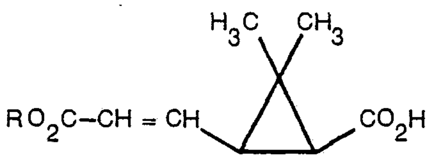

(II)

dans laquelle la copule cyclopropanique est de structure 1R cis et la double liaison de géométrie Z, tels que définis à la revendication 1 et les composés de formule VI

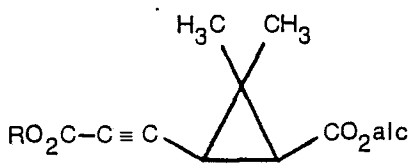

(VI)

dans laquelle la copule cyclopropanique est de structure 1R cis, tels que définis à la revendication 12.

28. A titre de produits intermédiaires nécessaires à la préparation des composés de formule I' tels que définis à la revendication 1, les composés de formule IX:

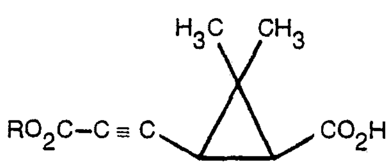

(IX)

et les composés de formule X

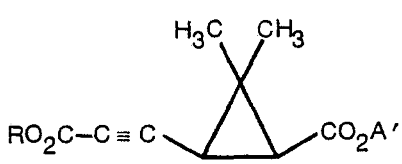

(X)

tels que définis à la revendication 14.

**Revendications pour l'Etat contractant: AT**

1. Procédé pour préparer sous toutes les formes isomères possibles ou sous forme de mélanges, les composés de formule (I'):

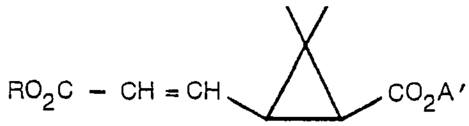

(I')

dans laquelle la copule cyclopropanique est de structure 1R cis et la double liaison de géométrie Z et dans laquelle A' représente

soit un radical alcoyle renfermant de 1 à 18 atomes de carbone,

soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxy et les atomes d'halogène,

soit un groupement

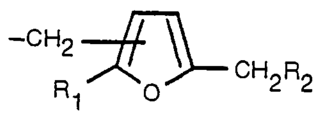

dans lequel le substituant $R_1$ représente un atome d'hydrogène ou un radical méthyle et le substituant $R_2$ un aryle monocyclique ou un groupement $—CH_2—C{\equiv}CH$

soit un groupement

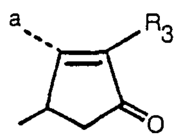

dans lequel a représente un atome d'hydrogène ou un radical méthyle et $R_3$ représente le radical $—CH_2—CH{=}CH_2$, $—CH_2—CH{=}CH—CH_3$, $—CH_2—CH{=}CH—CH{=}CH_2$, $—CH_2—CH{=}CH—CH_2—CH_3$,

soit un groupement

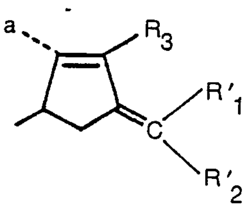

dans lequel a représente un atome d'hydrogène ou un radical méthyle, $R_3$ conserve la même signification que précédemment, $R'_1$ et $R'_2$, inditiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano.

soit un groupement

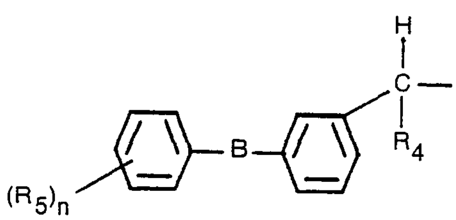

dans lequel B représente un atome d'oxygène ou de soufre ou un groupement

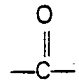

ou $—CH_2—$ et $R_4$ représente un atome d'hydrogène, un radical $—C{\equiv}N$, un radical méthyle, un radical

—$CONH_2$, un radical —$CSNH_2$ ou un radical —C≡CH, $R_5$ représente un atome d'halogène ou un radical méthyle et n représente un nombre égal à 0, 1 ou 2, et

soit un groupement

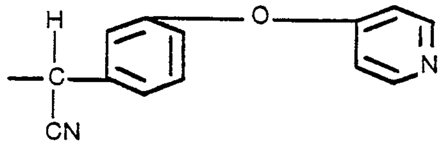

soit un groupement

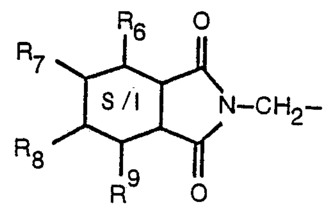

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tètrahydro,

soit un groupement

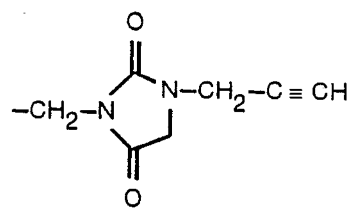

soit un groupement

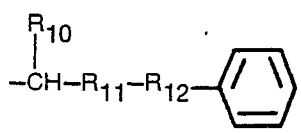

dans lequel $R_{10}$ représente un atome d'hydrogène ou un radical CN, $R_{12}$ représente un radical —$CH_2$— ou un atome d'oxygène, $R_{11}$ représente un radical thiazolyle ou thiadiazolyle dont la liaison avec

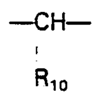

peut se trouver à l'une quelconque des positions disponibles, $R_{12}$ étant lié à $R_{11}$ par l'atome de carbone compris entre l'atome de soufre et un atome d'azote,

soit un groupement

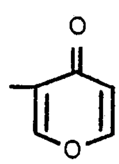

soit un groupement

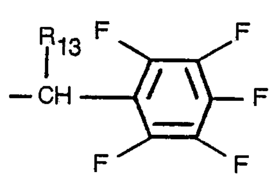

dans lequel $R_{13}$ représente un atome d'hydrogène ou un radical CN,

soit un groupement

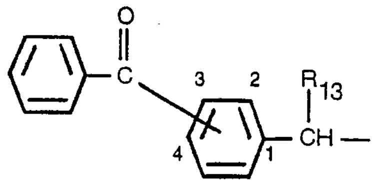

dans lequel $R_{13}$ est défini comme ci-dessus, et le radical benzoyle est en position 3 ou 4,

soit un groupement

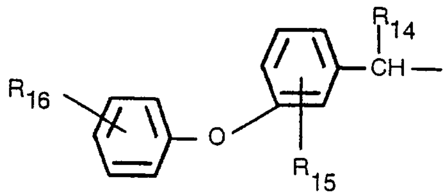

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

soit un groupement

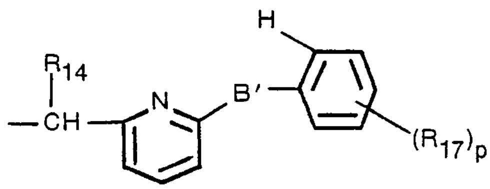

dans lequel $R_{14}$ est défini comme ci-dessus, chacun des $R_{17}$ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoyl sulfonyl renfermant de 1 à 4 atomes de carbone, trifluorométhyl, 3,4-méthylène dioxy, chloro, fluoro ou bromo, p représente un nombre égal à 0, 1 ou 2 et B' représente un atome d'oxygène ou un atome de soufre et R représente un radical alcoyle renfermant de 1 à 18 atomes de carbone substitué par un ou plusieurs groupements fonctionnels identiques ou différents choisis dans le groupe constitué par les atomes d'halogène, les groupements OH ou SH, les groupements OR' ou SR' dans lesquels R' représente un radical alcoyle renfermant de 1 à 8 atomes de carbone, les groupements $NO_2$ ou

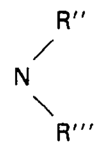

dans lesquels R'' et R''', identiques ou différents, représentent un atome d'hydrogène, ou un radical alcoyle renfermant de 1 à 8 atomes de carbone, les groupements C≡N, $SO_3H$ ou $PO_4H_2$ ou les groupements $COalc_1$, $SO_2alc_2$, ou $SO_3alc_3$ dans lesquels $alc_1$, $alc_2$ et $alc_3$ représentent des radicaux alcoyle renfermant de 1 à 18 atomes de carbone, ou R représente un radical alcoyle renfermant de 1 à 18 atomes de carbone substitué par un radical aryle lui-même éventuellement substitué par un ou plusieurs groupements OH, Oalc ou alc renfermant de 1 à 8 atomes de carbone, par un ou plusieurs groupements $CF_3$, $OCF_3$, $SCF_3$, ou par un groupement (G):

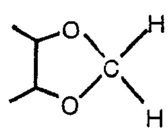

(G)

ou R représente un radical alcoyle renfermant de 1 à 18 atomes de carbone substitué sur deux carbones adjacents par un groupement ($G_1$)

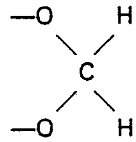

(G₁)

ou substitué par un groupement

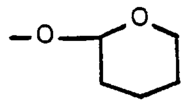

ou R représenté un groupement aryle renfermant de 6 à 14 atomes de carbone éventuellement substitué par un ou plusieurs groupements OH, Oalc ou alc renfermant de 1 à 18 atomes de carbone ou par un groupement $CF_3$, $OCF_3$ ou $SCF_3$, ou R représente un radical pyridinyle, furanyle, thiophényle, oxazolyle ou thiazolyle, caractérisé en ce que l'on fait réagir un acide de formule (II):

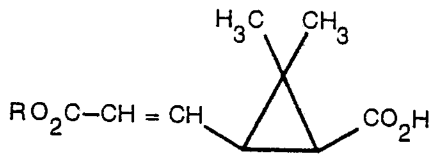

(II)

dans laquelle la copule cyclopropanique est de structure 1RS cis et dans laquelle R conserve la mëme signification que précédemment, ou un dérivé fonctionnel de cet acide, avec un alcool de formule (III)

A'—OH (III)

dans laquelle A' conserve la même signification que précédemment pour obtenir le composé de formule (I') correspondant.

2. Procédé selon la revendication 1, caractérisé en ce que l'on prépare les composés de formule (II) en faisant réagir au sein d'un solvant organique, un composé de formule ($B_1$)

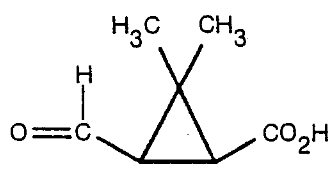

($B_1$)

sous forme de lactone cis, à l'action d'un composé de formule ($B_2$)

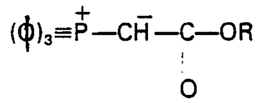

($B_2$)

dans laquelle R conserve sa signification précédente, pour obtenir le composé de formule (II) correspondant

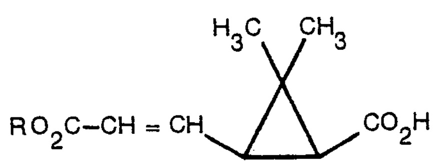

(II)

dans laquelle la copule cyclopropanique est de structure 1R cis sous forme d'un mélange d'isomères E et Z, que l'on sépare, en chacun des isomères.

3. Procédé selon la revendication 1, caractérisé en ce que l'on prépare les composés de formule (II) en faisant réagir un composé de formule (IV)

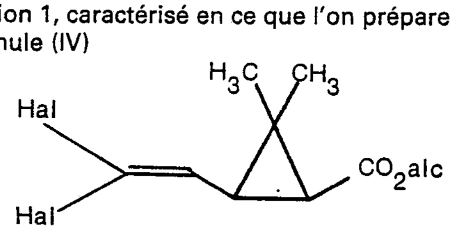

(IV)

dans laquelle la copule cyclopropanique est de structure 1R cis et dans laquelle Hal représente un atome d'halogène et alc représente un radical alcoyle renfermant de 1 à 20 atomes de carbone, dans un premier temps avec un agent alcalin capable d'arracher les atomes d'halogène, puis, dans un deuxième temps, soit avec un agent capable d'introduire le groupement carboxylique pour obtenir le composé de formule (V)

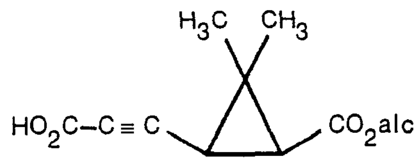

(V)

dans laquelle la copule cyclopropanique est de structure 1R cis que l'on soumet à l'action d'un agent d'estérification pour obtenir le composé de formule (VI)

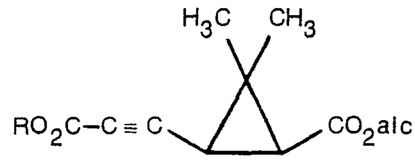

(VI)

dans laquelle la copule cyclopropanique est de structure 1R cis et dans laquelle R conserve la même signification que précédemment,

soit avec un dérivé de formule

$$Hal—CO_2R$$

dans laquelle Hal représente un atome d'halogène et R conserve sa signification précédente, pour obtenir directement le composé de formule (VI)

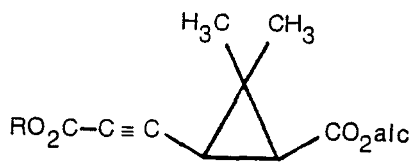

(VI)

dans laquelle la copule cyclopropanique est de structure 1R cis, puis soumet le composé de formule (VI) à l'action d'un agent d'hydrogénation ménagée, pour obtenir le composé de formule (VII)

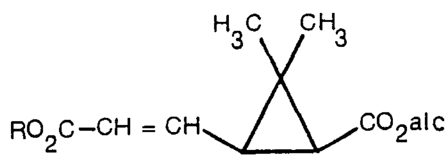

(VII)

dans laquelle la copule cyclopropanique est de structure 1R cis et dans laquelle la double liaison a la géométrie Z, que l'on soumet à l'action d'un agent d'hydrolyse acide capable de cliver sélectivement la fonction ester sur le carbone en 1 du cyclopropane, pour obtenir le composé de formule (II) correspondant.

4. Procédé selon la revendication 3, caractérisé en ce que l'on soumet d'abord le composé de formule (V) à l'action d'un agent d'hydrogénation ménagée pour obtenir le composé de formule (VIII)

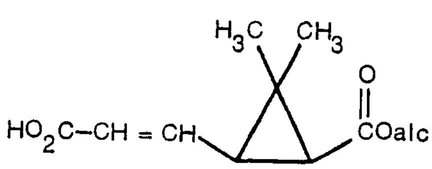

(VIII)

dans laquelle la copule cyclopropanique est de structure 1R cis dans laquelle la double liaison a la géométrie Z, que l'on soumet à l'action d'un agent d'estérification, pour obtenir le composé de formule (VII) correspondant

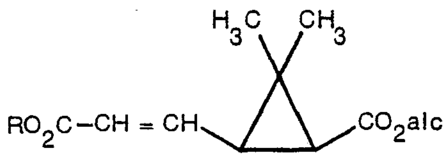

(VII)

dans laquelle la copule cyclopropanique a la structure 1R cis et al la double liaison la géométrie Z dans lequel R conserve sa signification précédente, puis poursuit la synthèse comme décrit à la revendication 3.

5. Procédé selon les revendications 1 et 3, caractérisé en ce que l'on soumet un composé de formule (VI)

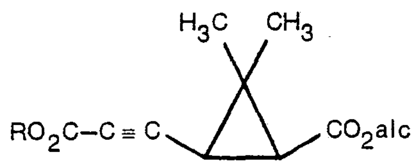

(VI)

dans laquelle la copule cyclopropanique est de structure 1R cis et dans laquelle R et alc, sont définis comme aux revendications 1 et 3, à l'action d'un agent d'hydrolyse acide capable de cliver sélectivement la fonction ester en 1 du cyclopropane, pour obtenir le composé de formule (IX)

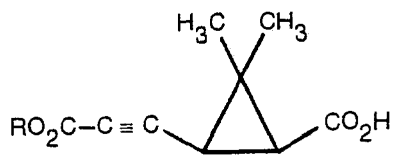

(IX)

dans laquelle la copule cyclopropanique est de structure 1R cis et dans laquelle R est défini comme précédemment, que

soit l'on soumet, le cas échéant sous forme d'un dérivé fonctionnel, à l'action d'un alcool de formule (III)

A'—OH (III)

dans laquelle A' conserve la même signification qu'à la revendication 1, pour obtenir le composé de formule (X)

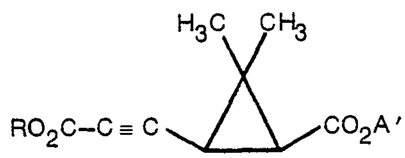

(X)

dans laquelle la copule cyclopropanique est de structure 1R cis et dans laquelle R et A' conservent la même signification que précédemment, que l'on soumet à l'action d'un agent d'hydrogénation ménagée, pour obtenir le composé de formule (I'),

soit l'on soumet d'abord à l'action d'un agent d'hydrogénation ménagée, pour obtenir le composé de formule (II)

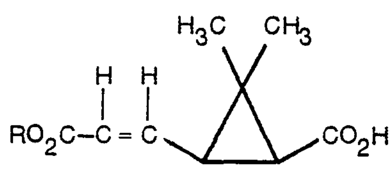

(II)

dans laquelle R est défini comme précédemment, la copule cyclopropanique a la structure R et la double liaison à la géométrie Z, puis, le cas échéant sous forme d'un dérivé fonctionnel, à l'action d'un alcool (III), pour obtenir le composé de formule (I').

6. Procédé de préparation des composés de formule (I'), telle que définie à la revendication 1, caractérisé en ce que l'on soumet un composé de formule (XI)

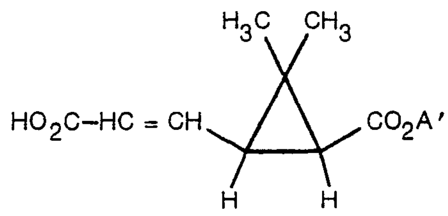

(XI)

dans laquelle la copule cyclopropanique est de structure 1R cis et la double liaison la géométrie Z, à l'action d'un agent d'estérification, pour obtenir le composé de formule (I') correspondant.

7. Procédé selon la revendication 6, caractérisé en ce que le composé de formule (XI) est préparé en soumettant un acide de formule (V)

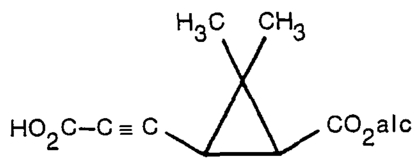

(V)

dans laquelle la copule cyclopropanique a la structure 1R cis et dans laquelle alc représente un radical alcoyle renfermant de 1 à 8 atomes de carbone, à l'action du 2,2,2-trichloroéthanol pour obtenir le composé de formule (XII)

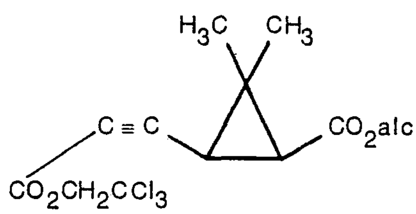

(XII)

dans laquelle la copule cyclopropanique est de structure 1R cis que l'on soumet à l'action d'un agent d'hydrolyse acide pour obtenir le composé de formule (XIII)

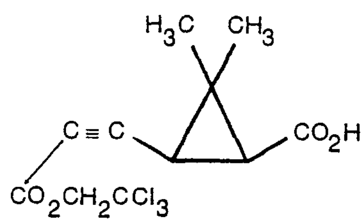

(XIII)

dans laquelle la copule cyclopropanique est de structure 1R cis que l'on soumet à l'action d'un alcool de formule (III)

A'—OH (III)

dans laquelle A' conserve la même signification que dans la revendication 1, pour obtenir le composé de formule (XIV)

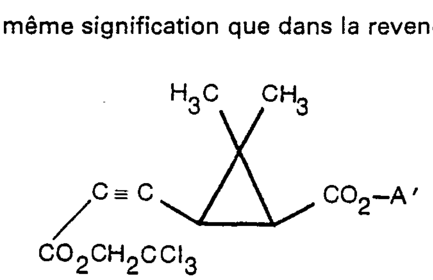

(XIV)

dans laquelle la copule cyclopropanique est de structure 1R cis que l'on soumet à l'action d'un agent de clivage de la fonction ester porté par le carbone acétylénique pour obtenir le composé de formule (XV)

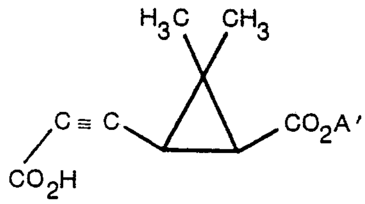

(XV)

dans laquelle la copule cyclopropanique est de structure 1R cis que l'on soumet à l'action d'un agent d'hydrogénation ménagée pour obtenir le composé de formule (XI).

8. Procédé selon les revendications 6 et 7, caractérisé en ce que l'on soumet un composé de formule (XV)

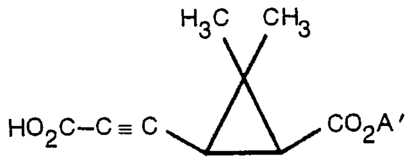

(XV)

dans laquelle la copule cyclopropanique est de structure 1R cis et A' est défini comme à la revendication 1, à l'action d'un agent d'estérification, pour obtenir le composé de formule (X)

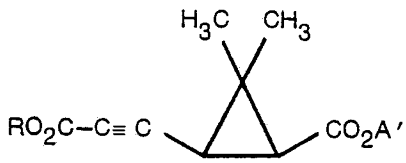

(X)

dans laquelle la copule cyclopropanique est de structure 1R cis et R et A' sont définis comme à la revendication 1, que l'on soumet à l'action d'un agent d'hydrogénation ménagée, pour obtenir le composé de formule (I'), dans laquelle la copule cyclopropanique est de structure 1R cis et la géométrie de la double liaison est Z.

9. Procédé de préparation des composés de formule (I'), dans laquelle R représente un radical alcoyle substitué par un ou plusieurs radicaux hydroxy, selon lequel l'on prépare d'abord par un procédé selon l'une quelconque des revendications 1 à 8, des composés de formule (I') dans laquelle R représente un radical alcoyle substitué par un ou plusieurs radicaux hydroxy protégés, et caractérisé en ce que l'on soument ces composés à l'action d'un agent d'hydrolyse acide.

10. Procédé selon l'une quelconque des revendications 1 à 9, pour préparer sous toutes les formes stéréoisomères possibles ou sous forme de mélanges, les composés de formule (I'), tels que définis à la revendication 1, répondant à la formule (I):

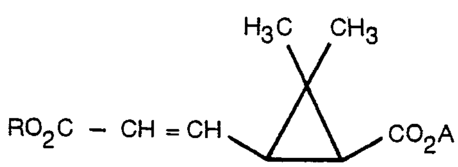

(I)

dans laquelle la copule cyclopropanique est de structure 1R cis et la double liaison de géométrie Z, dans laquelle A représente

soit un radical alcoyle renfermant de 1 à 18 atomes de carbone,

soit un radical benzyle éventuellement substitué tel que défini à la revendication 1,

soit un groupement

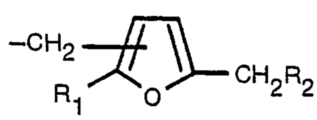

dans lequel les substituants $R_1$ et $R_2$ sont tels que définis à la revendication 1,

soit un groupement

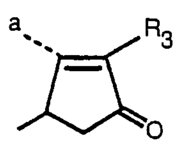

dans lequel a et $R_3$ sont tels que définis à la revendication 1

soit un groupement

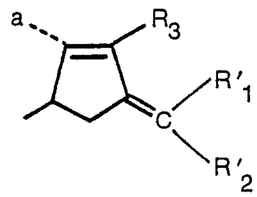

dans lequel a, $R'_1$, $R'_2$ et $R_3$ sont tels que définis à la revendication 1,

soit un groupement

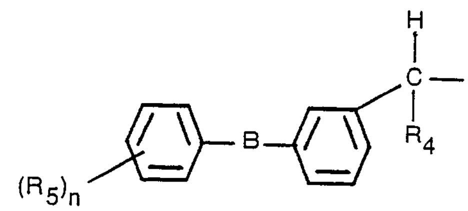

dans lequel B représente un atome d'oxygène, un groupement

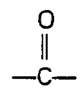

ou $—CH_2—$, $R_4$ est tel que défini à la revendication 1, $R_5$ représente un atome de chlore ou un radical méthyle et n représente un nombre égal à 0, 1 ou 2,

soit un groupement

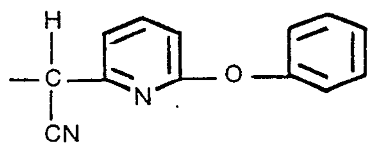

soit un groupement

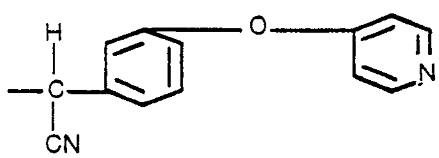

soit un groupement

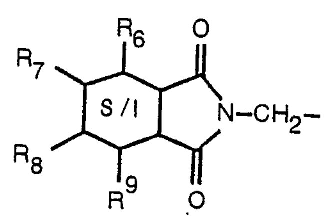

dans lequel les substituants $R_6$, $R_7$, $R_8$, $R_9$ et S/I sont tels que définis à la revendication 1,

soit un groupement

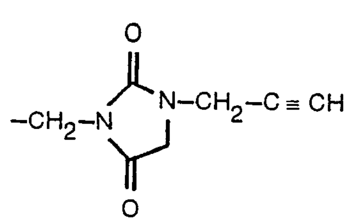

et R est tel que défini à la revendication 1, caractérisé en ce que l'on que l'on utilise au départ des composés de formule III, XI ou XV dans lesquelles A' a les valeurs de A indiquées précédemment.

11. Procédé selon l'une quelconque des revendications 1 à 10, caractérisé en ce que l'on utilise au départ des composés de formule III, IX ou XV dans lesquelles A' représente un groupement α-cyano-3-phénoxybenzyle sous forme S, R ou RS ou un groupement 2-méthyl-4-oxo-3-(2-propényl) 2-cyclopentèn-1-yle, sous forme S, R ou RS.

12. Procédé selon l'une quelconque des revendications 1 à 11, caractérisé en ce que l'on utilise au départ des composés de formule II, $B_2$, VI ou des agents d'estérification comportant un groupement R que est un radical alcoyle renfermant de 1 à 18 atomes de carbone, substitué par un ou plusieurs groupements fonctionnels mentionnés à la revendication 1.

13. Procédé selon la revendication 12, caractérisé en ce que R représente un radical alcoyle substitué par un ou plusieurs atomes d'halogène.

14. Procédé selon la revendication 12, caractérisé en ce que R est substitué par un ou plusieurs atomes de fluor.

15. Procédé selon la revendication 14, caractérisé en ce que R représente un groupement $—CH_2—CF_3$.

16. Procédé selon l'une quelconque des revendications 1 à 8 10 à 12 et 14 à 15, caractérisé en ce que l'on prépare
le (1R cis) 2,2-diméthyl 3/(Z) 3-oxo 3-(2-(1,1,1,3,3,3-hexafluoro)propoxy) 1-propényl/cyclopropane carboxylate de (S) α-cyano 3-phénoxy benzyle.

17. Application des composés de formule (I'), tels que définis à la revendication 1, à la lutte contre les parasites des végétaux, les parasites des locaux et les parasites des animaux à sang chaud.

18. Les compositions destinées à la lutte contre les parasites des végétaux, les parasites des locaux et les parasites des animaux à sang chaud, caractérisées en ce qu'elles renferment comme principe actif au moins un des products définis à la revendication 1.

19. Les compositions insecticides renfermant comme principe actif au moins l'un des produits définis à la revendication 1.

20. Les compositions insecticides renfermant comme principe actif le produit de la revendication 16.

21. Les compositions destinées à l'alimentation animale renfermant comme principe actif au moins l'un des produits définis à la revendication 1.

22. Compositions douées d'activité insecticide, acaricide ou nématicide, caractérisées en ce qu'elles contiennent comme matière active, d'une part un au moins des composés de formule générale (I'), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels "halo" represénte un atome de fluor, de chlore ou de brome, étant entendu que les composés (I') peuvent exister sous toutes leurs formes stéréoisomères possibles, de même que les copules acides et alcools des esters pyréthrinoïdes ci-dessus.

23. Compositions selon l'une quelconque des revendications 18 à 21, caractérisées en ce que les composés de formule (I') répondent à la formule (I) telle que définie à la revendication 20.

**Patentansprüche für die Vertragsstaaten: BE CH DE FR GB IT LI LU NL SE**

In sämtlichen ihrer möglichen isomeren Formen oder in Form von Gemischen die Verbindungen der Formel (I')

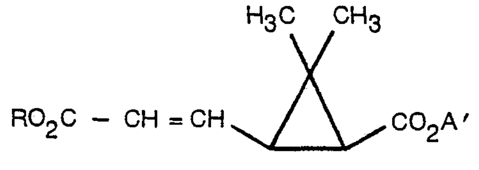

(I')

worin die Cyclopropan-Verknüpfung die 1R, cis-Struktur besitzt und die Doppelbindung die Z-Geometrie aufweist und worin A' bedeutet:

entweder einen Alkylrest mit 1 bis 18 Kohlenstoffatomen

oder einen Benzylrest, der gegebenenfalls durch ein oder mehrere Reste substituiert ist, ausgewählt unter den Alkylresten mit 1 bis 4 Kohlenstoffatomen, den Alkenylresten mit 2 bis 6 Kohlenstoffatomen, den

Alkenyloxyresten mit 2 bis 6 Kohlenstoffatomen, den Alkadienylresten mit 4 bis 8 Kohlenstoffatomen, dem Methylendioxyrest und den Halogenatomen,

oder eine Gruppe

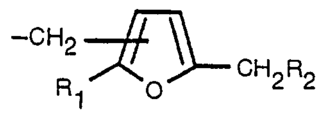

worin der Substituent $R_1$ ein Wasserstoffatom oder einen Methylrest bedeutet und der Substituent $R_2$ einen monocyclischen Arylrest oder eine Gruppe $-CH_2-C{\equiv}CH$ darstellt, oder eine Gruppe

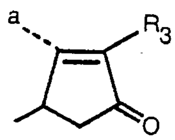

worin a ein Wasserstoffatom oder einen Methylrest bedeutet und $R_3$ den Rest $-CH_2-CH{=}CH_2$, $-CH_2-CH{=}CH-CH_3$, $-CH_2-CH{=}CH-CH{=}CH_2$ oder $-CH_2-CH{=}CH-CH_2-CH_3$ darstellt, oder eine Gruppe

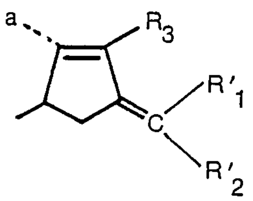

worin a ein Wasserstoffatom oder einen Methylrest bedeutet, $R_3$ die vorstehende Bedeutung besitzt, $R_1'$ und $R_2'$, die gleich oder verschieden sein können, ein Wasserstoffatom, ein Halogenatom, einen Alkkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten, oder eine Gruppe

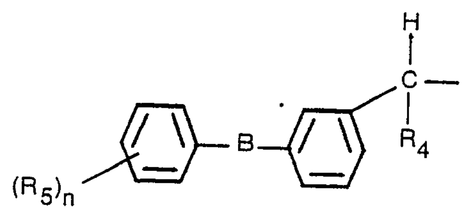

worin B ein Sauerstoff- oder Schwefelatom oder eine Gruppe

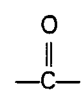

oder $-CH_2-$ bedeutet und $R_4$ ein Wasserstoffatom, einen Rest $-C{\equiv}N$, einen Methylrest, einen Rest $-CONH_2$, einen Rest $-CSNH_2$ oder einen Rest $-C{\equiv}CH$ bedeutet, $R_5$ ein Halogenatom oder einen Methylrest bedeutet und n eine Zahl entsprechend 0, 1 oder 2 darstellt, oder eine Gruppe

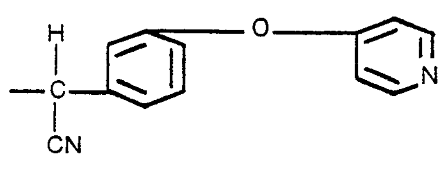

oder eine Gruppe

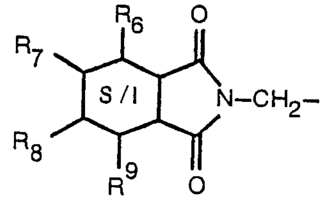

worin die Substituenten $R_6$, $R_7$, $R_8$ und $R_9$ ein Wasserstoffatom, ein Chloratom oder einen Methylrest bedueten und worin S/I einen aromatischen Ring oder einen analogen Dihydro- oder Tetrahydroring symbolysiert, oder eine Gruppe

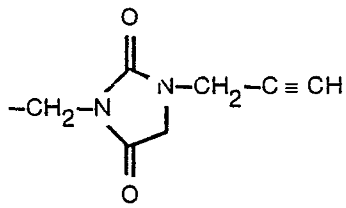

oder eine Gruppe

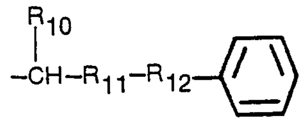

worin $R_{10}$ ein Wasserstoffatom oder eien CN-Rest bedeutet, $R_{12}$ einen Rest $—CH_2—$ oder ein Sauerstoffatom bedeutet, $R_{11}$ einen Thiazolyl- oder Thiazolylrest bedeutet, dessen Bindung mit

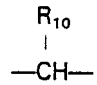

sich in irgendeiner der verfügbaren Positionen befinden kann, wobei $R_{12}$ an $R_{11}$ über das zwischen dem Schwefelatom und einem Stickstoffatom befindliche Kohlenstoffatom gebunden ist, oder eine Gruppe

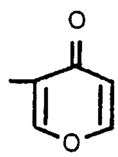

oder eine Gruppe

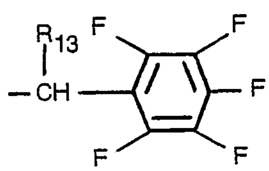

worin $R_{13}$ ein Wasserstoffatom oder einen CN-Rest bedeutet, oder eine Gruppe

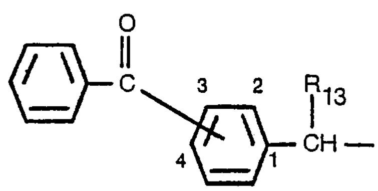

worin $R_{13}$ wie vorstehend definiert ist und der Benzoylrest sich in 3- oder 4-Stellung befindet, oder eine Gruppe

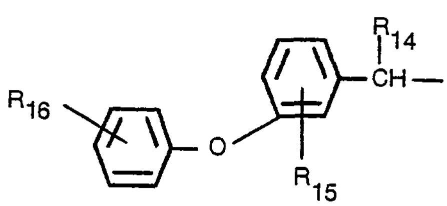

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten, oder eine Gruppe

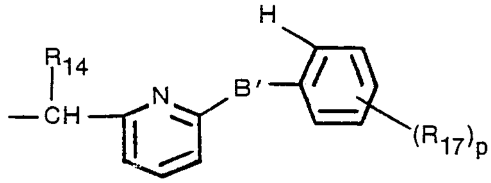

worin $R_{14}$ wie vorstehend definiert ist, ein jeder der Reste $R_{17}$ unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, eine Trifluormethylgruppe, eine 3,4-Methylendioxygruppe, Chlor, Fluor oder Brom bedeutet, p eine Zahl entsprechend 0, 1 oder 2 darstellt und B' ein Sauerstoffatom oder ein Schwefelatom bedeutet, und R einen Alkylrest mit 1 bis 18 Kohlenstoffatomen darstellt, der durch eine oder mehrere gleiche oder verschiedene Gruppen, ausgewählt unter den Halogenatomen, den OH— oder SH-Gruppen, den OR'- oder SR'-Gruppen, worin R' einen Alkylrest mit 1 bis 8 Kohlenstoffatomen bedeutet, den $NO_2$— oder

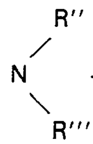 -Gruppen,

worin R'' und R''', die gleich oder verschieden sind, ein Wasserstoffatom oder einen Alkylrest mit 1 bis 8 Kohlenstoffatomen darstellen, den Gruppen C≡N, $SO_3H$ oder $PO_4H_2$ oder den Gruppen $COalc_1$, $SO_2alc_2$ oder $SO_3alc_3$, worin $alc_1$, $alc_2$ und $alc_3$ Alkylreste mit 1 bis 18 Kohlenstoffatomen darstellen, substituiert ist, oder R einen Alkylrest mit 1 bis 18 Kohlenstoffatomen darstellt, der durch einen Arylrest substituiert ist, der seinerseits gegebenenfalls durch eine oder mehrere OH—, Oalc oder alc-Gruppen mit 1 bis 8 Kohlenstoffatomen, durch eine oder mehrere Gruppen $CF_3$, $OCF_3$, $SCF_3$ oder durch eine Gruppe (G)

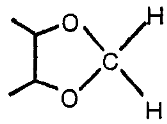

(G)

substituiert ist, oder R einen Alkylrest mit 1 bis 18 Kohlenstoffatomen darstellt, der an zwei benachbarten Kohlenstoffatomen durch eine Gruppe ($G_1$)

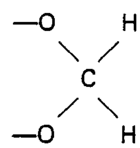

($G_1$)

substituiert ist oder der substituiert ist durch eine Gruppe

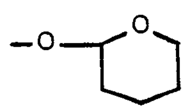

oder R eine Arylgruppe mit 6 bis 14 Kohlenstoffatomen darstellt, die gegebenenfalls durch eine oder mehrere Gruppen OH, Oacl oder alc mit 1 bis 8 Kohlenstoffatomen oder durch eine Gruppe $CF_3$, $OCF_3$ oder $SCF_3$ substituiert ist, oder R einen Pyridinyl-, Furanyl-, Thiophenyl-, Oxazolyl- oder Thiazolyl-Rest darstellt.

2. In sämtlichen inhrer möglichen isomeren Formen oder in Form von Gemischen die Verbindungen der Formel (I') gemäß Anspruch 1 der Formel (I)

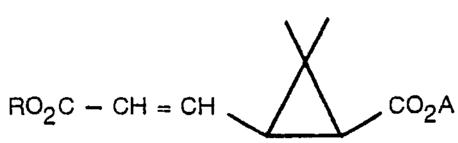

(I)

worin die Struktur der Cyclopropanverknüpfung die 1R, cis-Struktur ist und die Geometrie der Doppelbindung die Z-Geometrie ist und worin A bedeutet:

entweder einen Alkylrest mit 1 bis 18 Kohlenstoffatomen, oder einen gegebenenfalls substituierten Benzylrest, wie in Anspruch 1 definiert, oder eine Gruppe

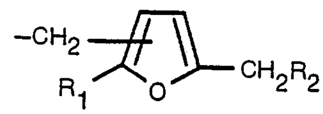

worin die Substituenten $R_1$ und $R_2$ wie in Anspruch 1 definiert sind, oder eine Gruppe

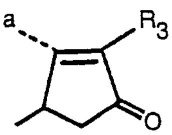

worin a und $R_3$ wie in Anspruch 1 definiert sind, oder eine Gruppe

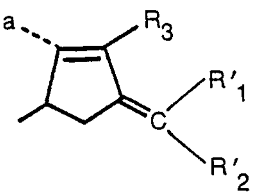

worin a, $R_1'$, $R_2'$ und $R_3$ wie in Anspruch 1 definiert sind, oder eine Gruppe

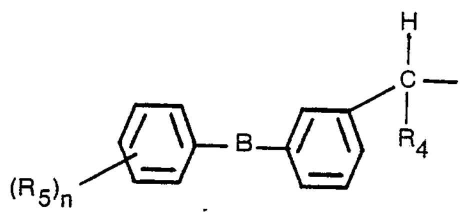

worin B ein Sauerstoffatom, eine Gruppe

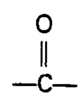

oder $-CH_2-$ bedeutet, $R_4$ wie in Anspruch 1 definiert ist, $R_5$ ein Chloratom oder einen Methylrest bedeutet und n eine Zahl entsprechend 0, 1 oder 2 darstellt, oder eine Gruppe

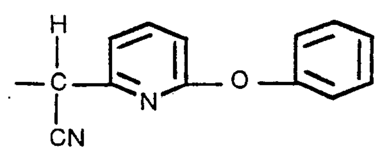

oder eine Gruppe

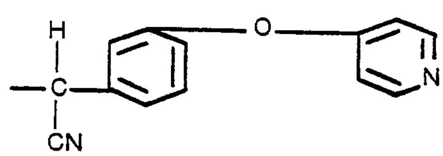

oder eine Gruppe

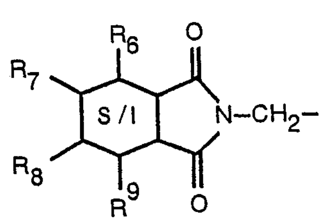

worin die Substituenten $R_6$, $R_7$, $R_8$, $R_9$ und S/I wie in Anspruch 1 definiert sind, oder eine Gruppe

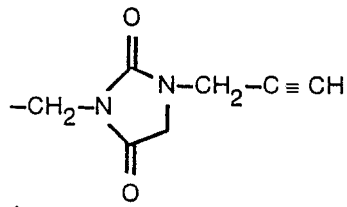

und R wie in Anspruch 1 definiert ist.

3. Die Verbindungen der Formel (I') gemäß einem der Ansprüche 1 und 2, worin A eine α-Cyano-3-phenoxybenzyl-Gruppe in der S—, R— oder RS-Form bedeutet.

4. Die Verbindungen der Formel (I') gemäß einem der Ansprüche 1 und 2, worin A eine 2-Methyl-4-oxo-3-(2-propenyl)-2-cyclopenten-1-yl-Gruppe in der S—, R— oder RS-Form bedeutet.

5. Die Verbindungen der Formel (I') gemäß einem der Ansprüche 1 bis 4, worin R einen Alkylrest mit 1 bis 18 Kohlenstoffatomen bedeutet, der durch eine oder mehrere in Anspruch 1 angagebene, funktionelle Gruppen substituiert ist.

6. Die Verbindungen der Formel (I') gemäß Ansprüche 5, dadurch gekennzeichnet, daß R einen Alkylrest bedeutet, der durch ein oder mehrere Halogenatome substituiert ist.

7. Die Verbindungen der Formel (I') gemäß Ansprüche 6, dadurch gekennzeichnet, daß R durch ein oder mehrere Fluoratome substituiert ist.

8. Die Verbindungen der Formel (I') gemäß Ansprüche 7, worin R eine Gruppe $CH_2CF_3$ bedeutet.

9. Die Verbindungen der Formel (I') mit der folgenden Bezeichnung:
(S)-α-Cyano-3-phenoxy-benzyl-(1R,cis)-2,2-dimethyl-3-[(Z)-3-oxo-3-(2-(1,1,1,3,3,3-hexafluor)-propoxy)-1-propenyl]-cyclopropan-carboxylat.

10. Verfahren zur Herstellung der Verbindungen der Formel (I') gemäß einem der Ansprüche 1 bis 9, dadurch gekennzeichnet, daß man eine Säure der Formel (II)

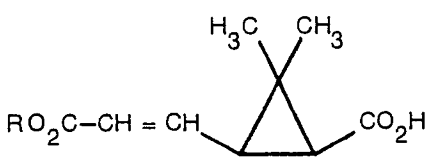

(II)

worin die Cyclopropan-Verknüpfung die 1R,cis-Struktur besitzt und die Doppelbindung die Z-Geometrie aufweist und worin R die in Anspruch 1 angegebene Bedeutung besitzt, oder ein funktionelles Derivat dieser Säure mit einem Alkohol der Formel (III)

$$A'—OH \qquad (III)$$

worin A' die in Anspruch 1 angegebene Bedeutung besitzt, umsetzt, um die entsprechende Verbindung der Formel (I') zu erhalten.

11. Verfahren gemäß Anspruch 10, dadurch gekennzeichnet, daß man die Verbindungen der Formel (II) herstellt, indem man in dem Medium eines organischen Lösungsmittels eine Verbindung der Formel ($B_1$)

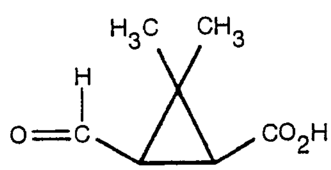

($B_1$)

in Form des cis-Lactons der Einwirkung einer Verbindung der Formel ($B_2$)

$$(\phi)_3\equiv\overset{+}{P}—\overset{-}{C}H—\underset{\underset{O}{\|}}{C}—OR \qquad (B_2)$$

worin R die vorstehende Bedeutung besitzt, unterzieht, um die entsprechende Verbindung der Formel (II)

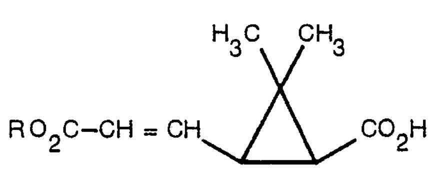

(II)

worin die Cyclopropan-Verknüpfung die 1R,cis-Struktur besitzt, in Form eines Gemisches der E- und Z-Isomeren zu erhalten, das man in ein jedes der Isomeren auftrennt.

12. Verfahren gemäß Anspruch 11, dadurch gekennzeichnet, daß man die Verbindungen der Formel (II) herstellt, indem man eine Verbindung der Formel (IV)

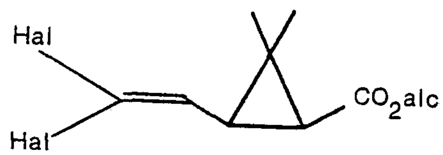

(IV)

worin die Cyclopropan-Verknüpfung die 1R,cis-Struktur besitzt und worin Hal ein Halogenatom bedeutet und alc einen Alkylrest mit 1 bis 20 Kohlenstoffatomen darstellt, in einer ersten Stufe mit einem alkalischen Mittel umsetzt, das befähigt ist, die Halogenatome an sich zu reißen, und danach in einer zweiten Stufe

entweder mit einem Mittel umsetzt, das befähigt ist, die Carboxylgruppe einzuführen, um die Verbindung der formel (V)

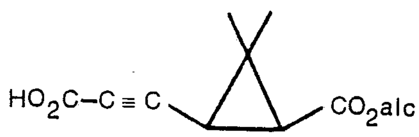

(V)

worin die Cyclopropan-Verknüpfung die 1R,cis-Struktur besitzt, zu erhalten, die man der Einwirkung eines Veresterungsmittels unterzieht, um eine Verbindung der Formel (VI)

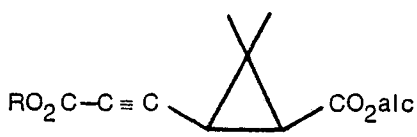

(VI)

zu erhalten, worin die Cyclopropan-Verknüpfung die 1R,cis-Struktur besitzt und worin R die vorstehend angegebene Bedeutung hat, oder mit einem Derivat der Formel

$$Hal—CO_2—R$$

worin Hal ein Halogenatom bedeutet und R die vorstehende Bedeutung besitzt, umsetzt, um direkt die Verbindung der Formel (VI)

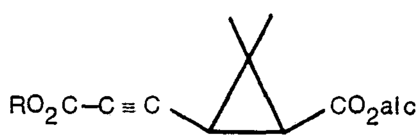

(VI)

worin die Cyclopropan-Verknüpfung die 1R,cis-Struktur besitzt, zu erhalten, danach die Verbindung der Formel (VI) der Einwirkung eines milden Hydrierungsmittels unterzieht, um die Verbindung der Formel (VII)

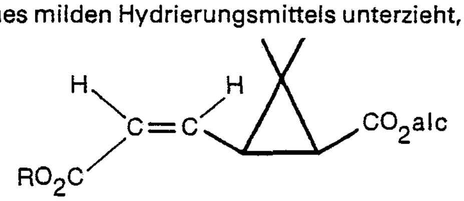

(VII)

worin die Cyclopropan-Verknüpfung die 1R,cis-Struktur besitzt und die Doppelbindung die Z-Geometrie aufweist,.zu erhalten, die man der Einwirkung eines sauren Hydrolysemittels unterzieht, das befähigt ist, selektiv die Esterfunktion an dem Kohlenstoffatom in 1-Stellung des Cyclopropans zu spalten, um die entsprechende Verbindung der Formel (II) zu erhalten.

13. Verfahren gemäß Anspruch 12, dadurch gekennzeichnet, daß man zunächst die Verbindung der Formel (V) der Einwirkung eines milden Hydrierungsmittels unterzieht, um die Verbindung der Formel (VIII)

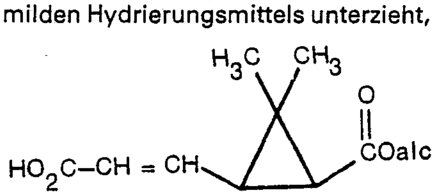

(VIII)

zu erhalten, worin die Cyclopropan-Verknüpfung die 1R,cis-Struktur besitzt und worin die Doppelbindung die Z-Geometrie aufweist, welche man der Einwirkung eines Veresterungsmittels unterzieht, um die entsprechende Verbindung der Formel (VII)

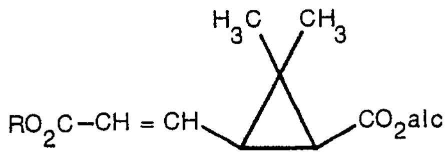

(VII)

zu erhalten, worin die Cyclopropan-Verknüpfung die 1R,cis-Struktur besitzt und worin R die vorstehende Bedeutung hat, und anschließend die Synthese, wie in Anspruch 12 angegeben, fortsetzt.

14. Verfahren gemäß Ansprüchen 10 und 12, dadurch gekennzeichnet, daß man eine Verbindung der formel (VI)

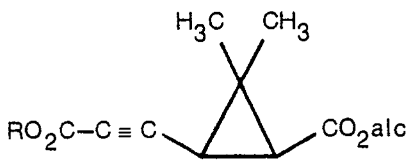

(VI)

worin die Cyclopropan-Verknüpfung die 1R,cis-Struktur besitzt und worin R und alc wie in den Ansprüchen 10 und 12 definiert sind, der Einwirkung eines sauren Hydrolysemittels unterzieht, das befähigt ist, selektiv die Esterfunktion in der 1-Stellung des Cyclopropans zu spalten, um die Verbindung der Formel (IX)

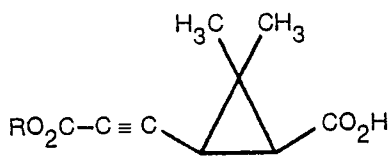

(IX)

zu erhalten, worin die Cyclopropan-Verknüpfung die 1R,cis-Struktur besitzt und worin R wie vorstehend definiert ist, welche man

entweder gegebenenfalls in Form eines funktionellen Derivats, der Einwirkung eines Alkohols der Formel (III)

$$A'\text{—}OH \qquad \text{(III)}$$

unterzieht, worin A' die in Anspruch 1 angegebene Bedeutung besitzt, um die Verbindung der Formel (X)

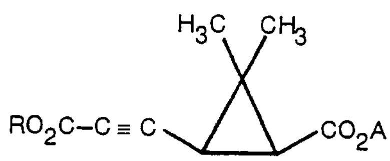

(X)

zu erhalten, worin die Cyclopropan-Verknüpfung die 1R,cis-Struktur besitzt und worin R und A' die vorstehend angegebene Bedeutung besitzen, welche man der Einwirkung eines milden Hydrierungsmittels unterzieht, um die Verbindung der Formel (I') zu erhalten, oder zunächst der Einwirkung eines milden Hydrierungsmittels unterzieht, um die Verbindung der Formel (II)

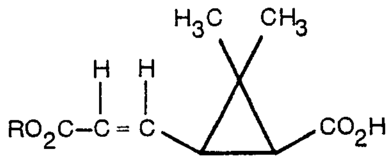

(II)

zu erhalten, worin R wie vorstehend definiert ist, die Cyclopropan-Verknüpfung die 1R,cis-Struktur besitzt und die Doppelbindung die Z-Geometrie aufweist, und danach, gegebenenfalls in Form eines funktionellen Derivats, der Einwirkung eines Alkohols (III) unterzieht, um die Verbindung der Formel (I') zu erhalten.

15. Verfahren zur Herstellung der Verbindungen der Formel (I') gemäß Anspruch 1, dadurch gekennzeichnet, daß man eine Verbindung der Formel (XI)

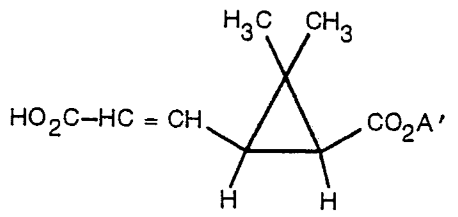

(XI)

worin die Cyclopropan-Verknüpfung die 1R,cis-Struktur besitzt und worin die Doppelbindung die Z-Geometrie aufweist, der Einwirkung eines Veresterungsmittels unterzieht, um die entsprechende Verbindung der Formel (I') zu erhalten.

16. Verfahren gemäß Anspruch 15, dadurch gekennzeichnet, daß die Verbindung der Formel (XI) hergestellt wird, indem man eine Säure der Formel (V)

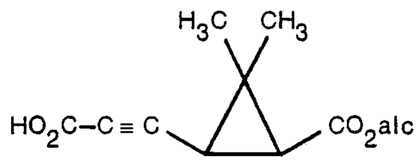

(V)

worin die Cyclopropan-Verknüpfung die 1R,cis-Struktur besitzt und worin alc einen Alkylrest mit 1 bis 8 Kohlenstoffatomen bedeutet, der Einwirkung von 2,2,2-Trichlorethanol unterzieht, um die Verbindung der Formel (XII)

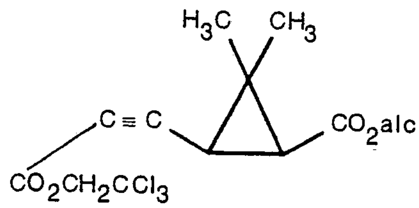

(XII)

worin die Cyclopropan-Verknüpfung die 1R,cis-Struktur besitzt, zu erhalten, die man der Einwirkung eines sauren Hydrolysemittels unterzieht, um die Verbindung der Formel (XIII)

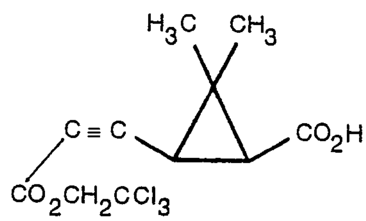

(XIII)

worin die Cyclopropan-Verknüpfung die 1R,cis-Struktur besitzt, zu erhalten, die man der Einwirkung eines Alkohols der Formel (III)

$$A'—OH \qquad (III)$$

worin A' die in Anspruch 1 angegebene Bedeutung besitzt, unterzieht, um die Verbindung der Formel (XIV)

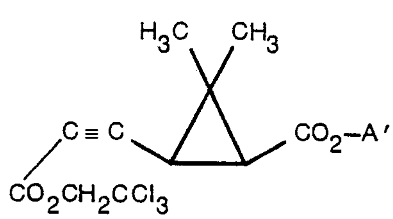

(XIV)

worin die Cyclopropan-Verknüpfung die 1R,cis-Struktur besitzt, zu erhalten, die man der Einwirkung eines Mittels zur Spaltung der an dem acetylenischen Kohlenstoff gebundenen Esterfunktion unterzieht, um die Verbindung der Formel (XV)

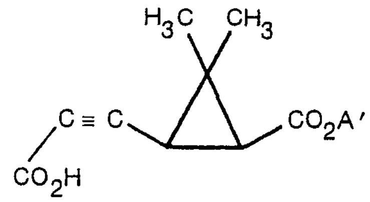

(XV)

worin die Cyclopropan-Verknüpfung die 1R,cis-Struktur besitzt, zu erhalten, die man der Einwirkung eines milden Hydrierungsmittels unterzieht, um die Verbindung der Formel (XI) zu erhalten.

17. Verfahren gemäß den Ansprüchen 15 und 16, dadurch gekennzeichnet, daß man eine Verbindung der Formel (XV)

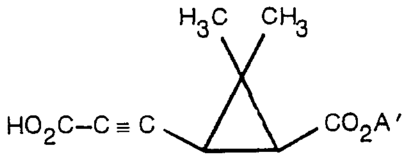

(XV)

worin die Cyclopropan-Verknüpfung die 1R,cis-Struktur besitzt, und worin A' wie in Anspruch 1 definiert ist, der Einwirkung eines Veresterungsmittels unterzieht, um die Verbindung der Formel (X)

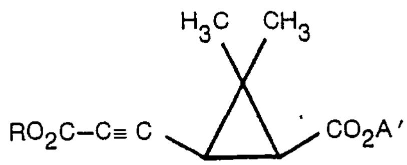

(X)

zu erhalten, worin die Cyclopropan-Verknüpfung die 1R,cis-Struktur besitzt, und worin R und A' wie in Anspruch 1 definiert sind, welche man der Einwirkung eines milden Hydrierungsmittels unterzieht, um die Verbindung der Formel (I')

zu erhalten, worin die Cyclopropan-Verknüpfung die 1R,cis-Struktur besitzt, zu erhalten, und die Geometrie der Doppelbindung die Z-Geometrie ist.

18. Verfahren zur Herstellung der Verbindungen der Formel (I'), worin R einen Alkylrest bedeutet, der durch einen oder mehrere Hydroxyreste substituiert ist, bei dem man zunächst nach einem Verfahren gemäß einem der Ansprüche 10 bis 17 Verbindungen der Formel (I') herstellt, worin R einen Alkylrest bedeutet, der durch einen oder mehrere geschützte Hydroxyreste substituiert ist, und dadurch gekennzeichnet, daß man diese Verbindungen der Einwirkung eines sauren Hydrolysemittels unterzieht.

19. Verwendung der Verbindungen der Formel (I') gemäß einem der Ansprüche 1 bis 9 zur Bekämpfung von Parasiten der Pflanzen, Parasiten von Räumlichkeiten und Parasiten warmblütiger Tiere.

20. Zusammensetzungen zur Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 9 enthalten.

21. Insektizide Zusammensetzungen, enthaltend als Wirkstoff zumindest eines der Produkte gemäß einem der Ansprüche 1 bis 8.

22. Insektizide Zusammensetzungen, enthaltend als Wirkstoff das Produkt gemäß Anspruch 9.

23. Zusammensetzungen für die tierische Ernährung, enthaltend als Wirkstoff zumindest eines der Produkte gemäß einem der Ansprüche 1 bis 9.

24. Mit insektizider, akarizider oder nematizider Aktivität ausgestattete Assoziationen, dadurch gekennzeichnet, daß sie als Wirkstoff einesteils zumindest eine der Verbindungen der allgemeinen Formel (I') und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, unter den Estern des 5-Benzyl-3-furyl-methylalkohols der 2,2-Dimethyl-3-(2-oxo-3-tetrahydrothiophenyliden-methyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropyl-essigsäuren, unter den Estern der Allethrolone, des 3,4,5,6-Tetrahydrophthalimidomethylalkohols, des 5-Benzyl-3-furyl-methylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin "halo" ein Fluor-, Chlor- oder Bromatom bedeutet, wobei die Verbindungen (I') in sämtlichen ihrer möglichen stereoisomeren Formen vorliegen können, ebenso wie die sauren Verknüpfungs-komponenten und die Alkohole der vorstehenden Pyrethrinoidester.

25. Als Arzneimittel die Verbindungen gemäß einem der Ansprüche 1 bis 9.

26. Pharmazeutische Zusammensetzungen, enthaltend als Wirkstoff zumindest eines der Arzneimittel gemäß Anspruch 25.

27. Als Zwischenprodukte, die für die Herstellung der Verbindungen der Formel (I') gemäß Anspruch 1 erforderlich sind, die Verbindungen der Formel (II)

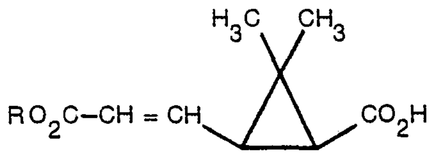

(II)

worin die Cyclopropan-Verknüpfung die 1R,cis-Struktur besitzt, und die Doppelbindung die Z-Geometrie aufweist, wie in Anspruch 1 definiert, und die Verbindungen der Formel VI

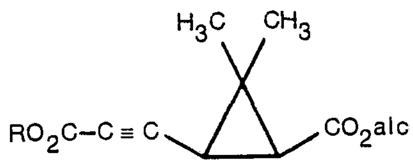

(VI)

worin die Cyclopropan-Verknüpfung die 1R,cis-Struktur besitzt, wie in Anspruch 12 definiert.

28. Als Zwischenprodukte, die für die Herstellung der Verbindungen der Formel (I'), wie in Anspruch 1 definiert, erforderlich sind, die Verbindungen der Formel IX

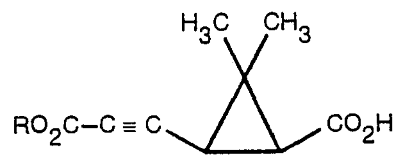

(IX)

und die Verbindungen der Formel X

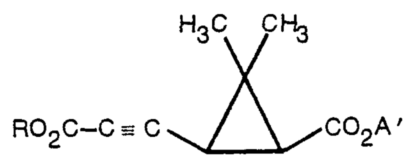

(X)

wie in Anspruch 14 definiert.

## Patentansprüche für den Vertragsstaat: AT

1. Verfahren zur Herstellung in sämtlichen ihrer möglichen isomeren Formen oder in Form von Gemischen der Verbindungen der Formel (I')

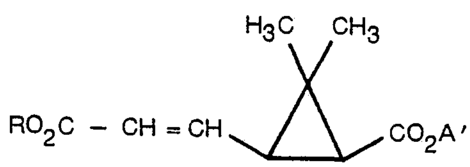

(I')

worin die Cyclopropan-Verknüpfung die 1R,cis-Struktur besitzt und die Doppelbindung die Z-Geometrie aufweist und worin A' bedeutet:

entweder einen Alkylrest mit 1 bis 18 Kohlenstoffatomen

oder einen Benzylrest, der gegebenenfalls durch ein oder mehrere Reste substituiert ist, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,

oder eine Gruppe

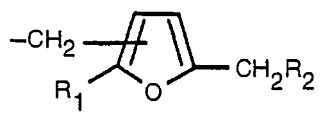

worin der Substituent $R_1$ ein Wasserstoffatom oder einen Methylrest bedeutet und der Substituent $R_2$ einen monocyclischen Arylrest oder eine Gruppe $—CH_2—C{\equiv}CH$ darstellt, oder eine Gruppe

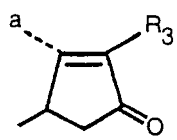

worin a ein Wasserstoffatom oder einen Methylrest bedeutet und $R_3$ den Rest $—CH_2—CH{=}CH_2$, $—CH_2—CH{=}CH—CH_3$, $—CH_2—CH{=}CH—CH{=}CH_2$ oder $—CH_2—CH{=}CH—CH_2—CH_3$ darstellt, oder eine Gruppe

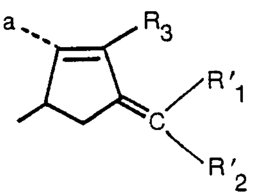

worin a ein Wasserstoffatom oder einen Methylrest bedeutet, $R_3$ die vorstehende Bedeutung besitzt, $R'_1$ und $R'_2$, die gleich oder verschieden sein können, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten, oder eine Gruppe

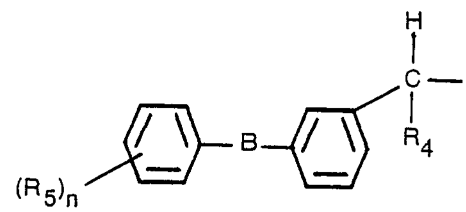

worin B ein Sauerstoff- oder Schwefelatom oder eine Gruppe

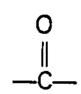

oder $—CH_2—$ bedeutet und $R_4$ ein Wasserstoffatom, einen Rest $—C{\equiv}N$, einen Methylrest, einen Rest $—CONH_2$, einen Rest $—CSNH_2$ oder einen Rest $—C{\equiv}CH$ bedeutet, $R_5$ ein Halogenatom oder einen Methylrest bedeutet und n eine Zahl entsprechend 0, 1 oder 2 darstellt, oder eine Gruppe

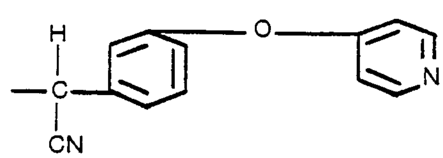

oder eine Gruppe

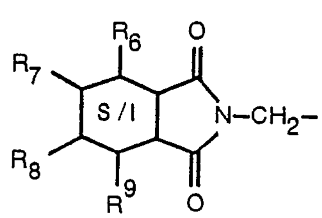

worin die Substituenten $R_6$, $R_7$, $R_8$ und $R_9$ ein Wasserstoffatom, ein Chloratom oder einen Methylrest

bedueten und worin S/I einen aromatischen Ring oder einen analogen Dihydro- oder Tetrahydroring symbolysiert, oder eine Gruppe

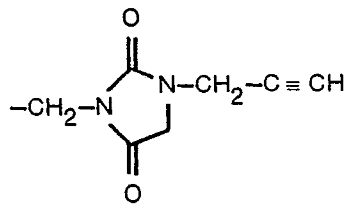

oder eine Gruppe

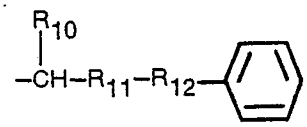

worin $R_{10}$ ein Wasserstoffatom oder einen CN-Rest bedeutet, $R_{12}$ einen Rest $-CH_2-$ oder ein Sauerstoffatom bedeutet, $R_{11}$ einen Thiazolyl- oder Thiazolylrest bedeutet, dessen Bindung mit

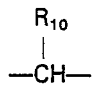

sich in irgendeiner der verfügbaren Positionen befinden kann, wobei $R_{12}$ an $R_{11}$ über das zwischen dem Schwefelatom und einem Stickstoffatom befindliche Kohlenstoffatom gebunden ist, oder eine Gruppe

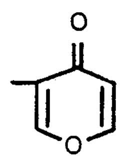

oder eine Gruppe

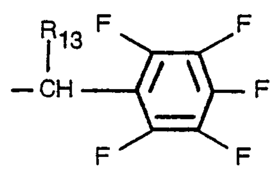

worin $R_{13}$ ein Wasserstoffatom oder einen CN-Rest bedeutet, oder eine Gruppe

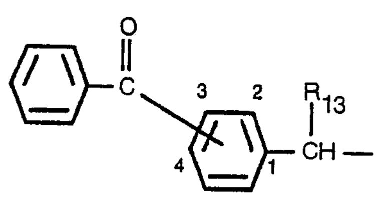

worin $R_{13}$ wie vorstehend definiert ist und der Benzoylrest sich in 3- oder 4-Stellung befindet, oder eine Gruppe

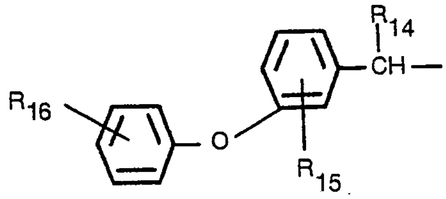

worin $R_{14}$ ein Wasserstoffatom, einen Methyl-, Ethinyl- oder Cyanorest bedeutet und $R_{15}$ und $R_{16}$, die voneinander verschieden sind, ein Wasserstoff-, Fluor- oder Bromatom bedeuten, oder eine Gruppe

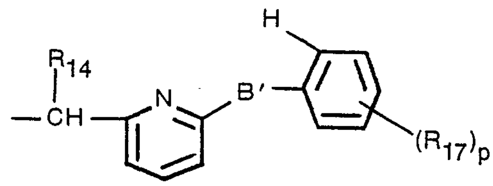

worin $R_{14}$ wie vorstehend definiert ist, ein jeder der Reste $R_{17}$ unabhängig eine Alkylgruppe mit 1 bis 4 Kohlenstoffatomen, eine Alkoxygruppe mit 1 bis 4 Kohlenstoffatomen, eine Alkylthiogruppe mit 1 bis 4 Kohlenstoffatomen, eine Alkylsulfonylgruppe mit 1 bis 4 Kohlenstoffatomen, eine Trifluormethylgruppe, eine 3,4-Methylendioxygruppe, Chlor, Fluor oder Brom bedeutet, p eine Zahl entsprechend 0, 1 oder 2 darstellt und B' ein Sauerstoffatom oder ein Schwefelatom bedeutet, und R einen Alkylrest mit 1 bis 18 Kohlenstoffatomen darstellt, der durch eine oder mehrere gleiche oder verschiedene Gruppen, ausgewählt unter den Halogenatomen, den OH— oder SH-Gruppen, den OR'- oder SR'-Gruppen, worin R' einen Alkylrest mit 1 bis 8 Kohlenstoffatomen bedeutet, den $NO_2$— oder

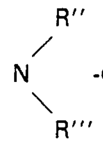 -Gruppen,

worin R'' und R''', die gleich oder verschieden sind, ein Wasserstoffatom oder einen Alkylrest mit 1 bis 8 Kohlenstoffatomen darstellen, den Gruppen C≡N, $SO_3H$ oder $PO_4H_2$ oder den Gruppen $COalc_1$, $SO_2alc_2$ oder $SO_3alc_3$, worin $alc_1$, $alc_2$ und $alc_3$ Alkylreste mit 1 bis 18 Kohlenstoffatomen darstellen, substituiert ist, oder R einen Alkylrest mit 1 bis 18 Kohlenstoffatomen darstellt, der durch einen Arylrest substituiert ist, der seinerseits gegebenenfalls durch eine oder mehrere OH—, Oalc oder alc-Gruppen mit 1 bis 8 Kohlenstoffatomen, durch eine oder mehrere Gruppen $CF_3$, $OCF_3$, $SCF_3$ oder durch eine Gruppe (G)

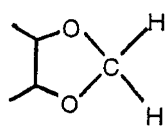

(G)

substituiert ist, oder R einen Alkylrest mit 1 bis 18 Kohlenstoffatomen darstellt, der an zwei benachbarten Kohlenstoffatomen durch eine Gruppe ($G_1$)

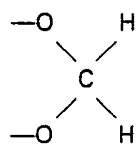

($G_1$)

substituiert ist oder der substituiert ist durch eine Gruppe

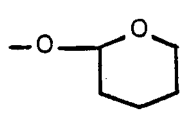

oder R eine Arylgruppe mit 6 bis 14 Kohlenstoffatomen darstellt, die gegebenenfalls durch eine oder mehrere Gruppen OH, Oacl oder alc mit 1 bis 8 Kohlenstoffatomen oder durch eine Gruppe $CF_3$, $OCF_3$ oder $SCF_3$ substituiert ist, oder R einen Pyridinyl-, Furanyl-, Thiophenyl-, Oxazolyl- oder Thiazolyl-Rest darstellt, dadurch gekennzeichnet, daß man eine Säure der Formel (II)

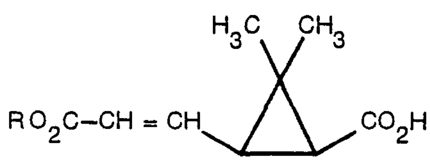

(II)

worin die Cyclopropan-Verküpfung die 1R,cis-Struktur besitzt und worin R die vorstehende Bedeutung besitzt, oder ein funktionelles Derivat dieser Säure mit einem Alkohol der Formel (III)

A'—OH (III)

worin A' die vorstehende Bedeutung besitzt, umsetzt, um die entsprechende Verbindung der Formel (I') zu erhalten.

2. Verfahren gemäß Anspruch 1, dadurch gekennzeichnet, daß man die Verbindungen der Formel (II) herstellt, indem man in dem Medium eines organischen Lösungsmittels eine Verbindung der Formel ($B_1$)

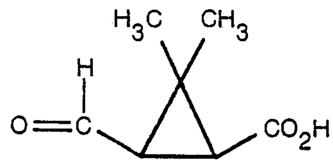

($B_1$)

in Form des cis-Lactons der Einwirkung einer Verbindung der Formel ($B_2$)

$$(\phi)_3{\equiv}\overset{+}{P}-\overset{-}{C}H-\underset{\underset{O}{\|}}{C}-OR \qquad (B_2)$$

worin R die vorstehende Bedeutung besitzt, unterzieht, um die entsprechende Verbindung der Formel (II)

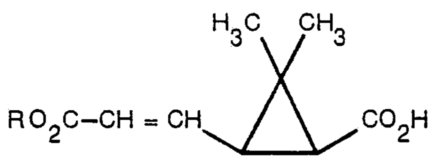

(II)

worin die Cyclopropan-Verknüpfung die 1R,cis-Struktur besitzt, in Form eines Gemisches der E- und Z-Isomeren zu erhalten, das man in ein jedes der Isomeren auftrennt.

3. Verfahren gemäß Anspruch 1, dadurch gekennzeichnet, daß man die Verbindungen der Formel (II) herstellt, indem man eine Verbindung der Formel (IV)

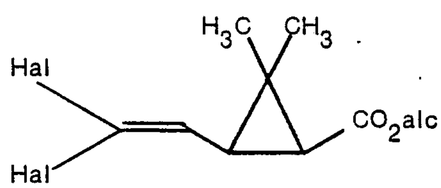

(IV)

worin die Cyclopropan-Verknüpfung die 1R,cis-Struktur besitzt und worin Hal ein Halogenatom bedeutet und alc einen Alkylrest mit 1 bis 20 Kohlenstoffatomen darstellt, in einer ersten Stufe mit einem alkalischen Mittel umsetzt, das befähigt ist, die Halogenatome an sich zu reißen, und danach in einer zweiten Stufe

entweder mit einem Mittel umsetzt, das befähigt ist, die Carboxylgruppe einzuführen, um die Verbindung der formel (V)

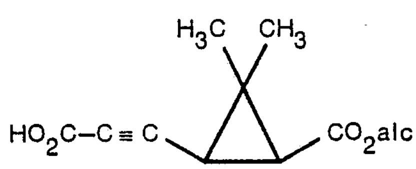

(V)

worin die Cyclopropan-Verknüpfung die 1R,cis-Struktur besitzt, zu erhalten, die man der Einwirkung eines Veresterungsmittels unterzieht, um eine Verbindung der Formel (VI)

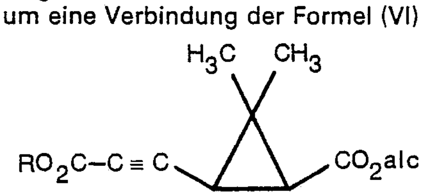

(VI)

zu erhalten, worin die Cyclopropan-Verknüpfung die 1R,cis-Struktur besitzt und worin R die vorstehend angegebene Bedeutung hat, oder mit einem Derivat der Formel

$$Hal-CO_2-R$$

worin Hal ein Halogenatom bedeutet und R die vorstehende Bedeutung besitzt, umsetzt, um direkt die Verbindung der Formel (VI)

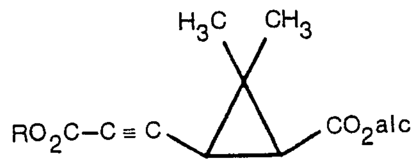

(VI)

worin die Cyclopropan-Verknüpfung die 1R,cis-Struktur besitzt, zu erhalten, danach die Verbindung der Formel (VI) der Einwirkung eines milden Hydrierungsmittels unterzieht, um die Verbindung der Formel (VII)

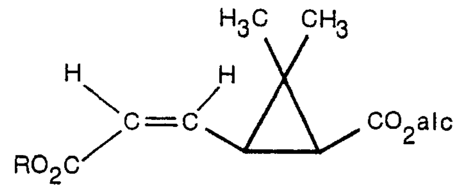

(VII)

worin die Cyclopropan-Verknüpfung die 1R,cis-Struktur besitzt und die Doppelbindung die Z-Geometrie aufweist, zu erhalten, die man der Einwirkung eines sauren Hydrolysemittels unterzieht, das befähigt ist, selektiv die Esterfunktion an dem Kohlenstoffatom in 1-Stellung des Cyclopropans zu spalten, um die entsprechende Verbindung der Formel (II) zu erhalten.

4. Verfahren gemäß Anspruch 3, dadurch gekennzeichnet, daß man zunächst die Verbindung der Formel (V) der Einwirkung eines milden Hydrierungsmittels unterzieht, um die Verbindung der Formel (VIII)

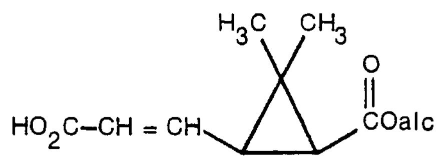

(VIII)

zu erhalten, worin die Cyclopropan-Verknüpfung die 1R,cis-Struktur besitzt und worin die Doppelbindung die Z-Geometrie aufweist, welche man der Einwirkung eines Veresterungsmittels unterzieht, um die entsprechende Verbindung der Formel (VII)

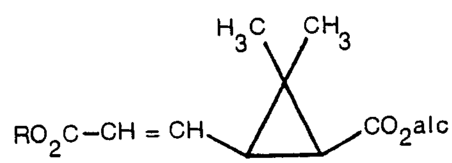

(VII)

zu erhalten, worin die Cyclopropan-Verknüpfung die 1R,cis-Struktur besitzt und die Doppelbindung die Z-Geometrie aufweist und worin R die vorstehend Bedeutung besitzt, und anschließend die Synthese, wie in Anspruch 3 angageben, fortsetzt.

5. Verfahren gemäß Ansprüchen 1 und 3, dadurch gekennzeichnet, daß man eine Verbindung der formel (VI)

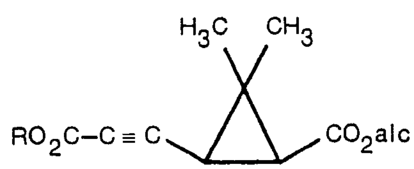

(VI)

worin die Cyclopropan-Verknüpfung die 1R,cis-Struktur besitzt und worin R und alc wie in den Ansprüchen 1 und 3 definiert sind, der Einwirkung eines sauren Hydrolysemittels unterzieht, das befähigt ist, selektiv die Esterfunktion in der 1-Stellung des Cyclopropans zu spalten, um die Verbindung der Formel (IX)

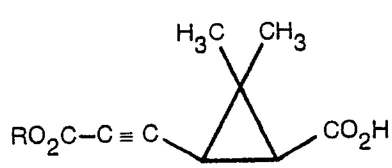

(IX)

zu erhalten, worin die Cyclopropan-Verknüpfung die 1R,cis-Struktur besitzt und worin R wie vorstehend definiert ist, welche man

entweder gegebenenfalls in Form eines funktionellen Derivats, der Einwirkung eines Alkohols der Formel (III)

$$A'—OH \qquad (III)$$

unterzieht, worin A' die in Anspruch 1 angegebene Bedeutung besitzt, um die Verbindung der Formel (X)

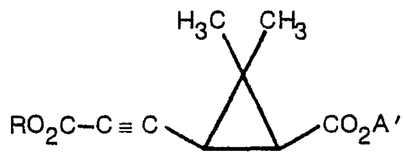

(X)

zu erhalten, worin die Cyclopropan-Verknüpfung die 1R,cis-Struktur besitzt und worin R und A' die vorstehend Bedeutung besitzen, welche man der Einwirkung eines milden Hydrierungsmittels unterzieht, um die Verbindung der Formel (I') zu erhalten, oder zunächst der Einwirkung eines milden Hydrierungsmittels unterzieht, um die Verbindung der Formel (II)

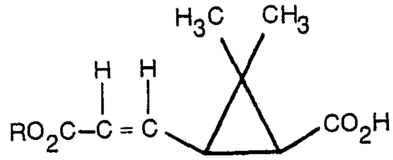

(II)

zu erhalten, worin R wie vorstehend definiert ist, die 1R,cis-Struktur besitzt und die Doppelbindung die Z-Geometrie aufweist, und danach, gegebenenfalls in Form eines funktionellen Derivats, der Einwirkung eines Alkohols (III) unterzieht, um die Verbindung der Formel (I') zu erhalten.

6. Verfahren zur Herstellung der Verbindungen der Formel (I') gemäß Anspruch 1, dadurch gekennzeichnet, daß man eine Verbindung der Formel (XI)

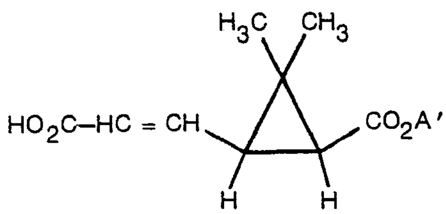

(XI)

worin die Cyclopropan-Verknüpfung die 1R,cis-Struktur besitzt und worin die Doppelbindung die Z-Geometrie aufweist, der Einwirkung eines Veresterungsmittels unterzieht, um die entsprechende Verbindung der Formel (I') zu erhalten.

7. Verfahren gemäß Anspruch 6, dadurch gekennzeichnet, daß die Verbindung der Formel (XI) hergestellt wird, indem man eine Säure der Formel (V)

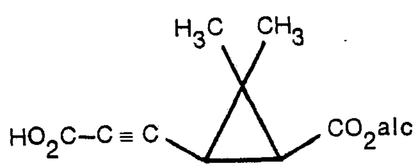

(V)

worin die Cyclopropan-Verknüpfung die 1R,cis-Struktur besitzt und worin alc einen Alkylrest mit 1 bis 8 Kohlenstoffatomen bedeutet, der Einwirkung von 2,2,2-Trichlorethanol unterzieht, um die Verbindung der Formel (XII)

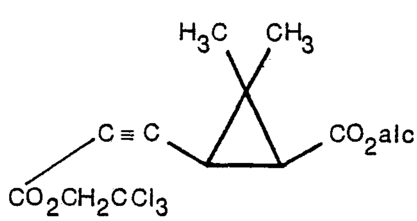

(XII)

worin die Cyclopropan-Verknüpfung die 1R,cis-Struktur besitzt, zu erhalten, die man der Einwirkung eines sauren Hydrolysemittels unterzieht, um die Verbindung der Formel (XIII)

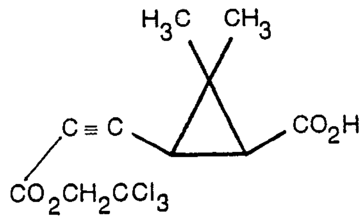

(XIII)

worin die Cyclopropan-Verknüpfung die 1R,cis-Struktur besitzt, zu erhalten, die man der Einwirkung eines Alkohols der Formel (III)

$$A'\text{—}OH \qquad (III)$$

worin A' die in Anspruch 1 angegebene Bedeutung besitzt, unterzieht, um die Verbindung der Formel (XIV)

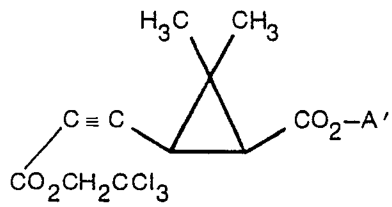

(XIV)

worin die Cyclopropan-Verknüpfung die 1R,cis-Struktur besitzt, zu erhalten, die man der Einwirkung eines Mittels zur Spaltung der von dem acetylenischen Kohlenstoff getragenen Esterfunktion unterzieht, um die Verbindung der Formel (XV)

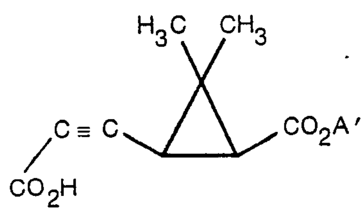

worin die Cyclopropan-Verknüpfung die 1R,cis-Struktur besitzt, zu erhalten, die man der Einwirkung eines milden Hydrierungsmittels unterzieht, um die Verbindung der Formel (XI) zu erhalten.

8. Verfahren gemäß den Ansprüchen 6 und 7, dadurch gekennzeichnet, daß man eine Verbindung der Formel (XV)

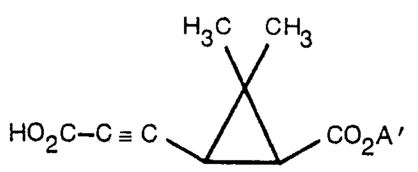

(XV)

worin die Cyclopropan-Verknüpfung die 1R,cis-Struktur besitzt, und A' wie in Anspruch 1 definiert ist, Einwirkung eines Veresterungsmittels unterzieht, um die Verbindung der Formel (X)

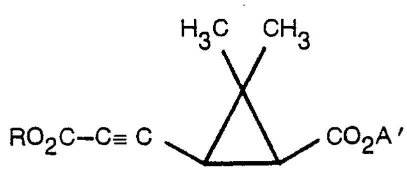

(X)

zu erhalten, worin die Cyclopropan-Verknüpfung die 1R,cis-Struktur besitzt, und worin R und A' wie in Anspruch 1 definiert sind, welche man der Einwirkung eines milden Hydrierungsmittels unterzieht, um die Verbindung der Formel (I') zu erhalten, worin die Cyclopropan-Verknüpfung die 1R,cis-Struktur besitzt, und die Geometrie der Doppelbindung die Z-Geometrie ist.

9. Verfahren zur Herstellung der Verbindungen der Formel (I'), worin R einen Alkylrest bedeutet, der durch einen oder mehrere Hydroxyrests substituiert ist, bei dem man zunächst nach einem Verfahren gemäß einem der Ansprüche 1 bis 8 Verbindungen der Formel (I') herstellt, worin R einen Alkylrest bedeutet, der durch einen oder mehrere geschützte Hydroxyreste substituiert ist, dadurch gekennzeichnet, daß man diese Verbindungen der Einwirkung eines sauren Hydrolysemittels unterzieht.

10. Verfahren gemäß einem der Ansprüche 1 bis 9 zur Herstellung in sämtlichen ihrer möglichen stereoisomeren Formen oder in Form von Gemischen der Verbindungen der Formel (I'), wie in Anspruch 1 definiert, der Formel (I)

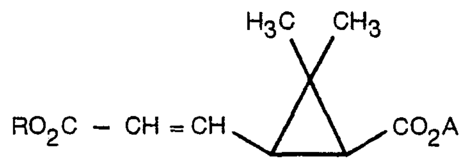

(I)

worin die Cyclopropan-Verknüpfung die 1R,cis-Struktur besitzt und die Doppelbindung die Z-Geometrie aufweist und worin A bedeutet:

entweder einen Alkylrest mit 1 bis 18 Kohlenstoffatomen, oder einen Benzylrest, der gegebenenfalls, wie in Anspruch 1 definiert, substituiert ist, oder eine Gruppe

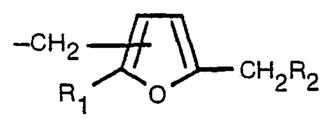

worin die Substituenten $R_1$ und $R_2$ wie in Anspruch 1 definiert sind, oder eine Gruppe

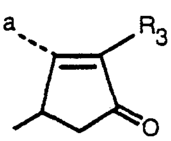

worin a und $R_3$ wie in Anspruch 1 definiert sind, oder eine Gruppe

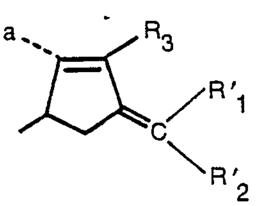

worin a, $R'_1$, $R'_2$ und $R_3$ wie in Anspruch 1 definiert sind, oder eine Gruppe

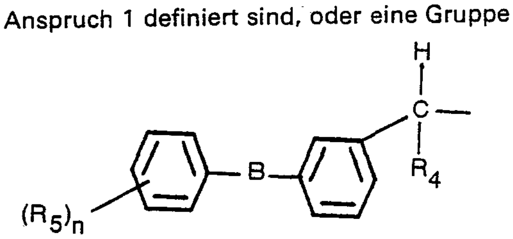

worin B ein Sauerstoffatom, eine Gruppe

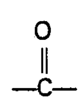

oder $-CH_2-$ bedeutet, $R_4$ wie in Anspruch 1 definiert ist, $R_5$ ein Chloratom oder einen Methylrest bedeutet und n eine Zahl entsprechend 0, 1 oder 2 darstellt, oder eine Gruppe

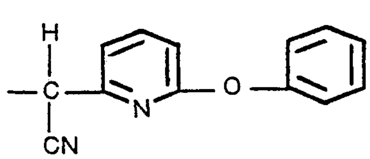

oder eine Gruppe

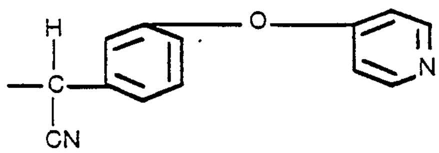

oder eine Gruppe

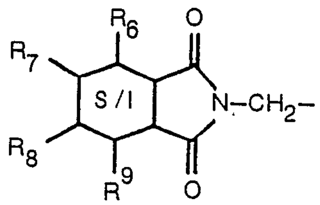

worin die Substituenten $R_6$, $R_7$, $R_8$, $R_9$ und S/I wie in Anspruch 1 definiert sind, oder eine Gruppe

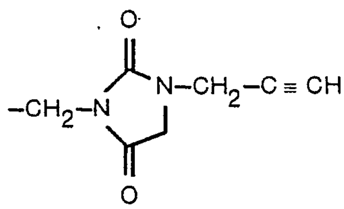

und R wie in Anspruch 1 definiert ist, dadurch gekennzeichnet, daß man von Verbindungen der Formel III, XI oder XV ausgeht, worin A' die vorstehend angegebenen Bedeutungen von A besitzt.

11. Verfahren gemäß einem der Ansprüche 1 bis 10, dadurch gekennzeichnet, daß man von Verbindungen der Formel III, XI oder XV ausgeht, worin A' eine α-Cyano-3-phenoxybenzylgruppe in der S-, R- oder RS-Form oder eine 2-Methyl-4-oxo-3-(2-propenyl)-2-cyclopenten-1-yl-Gruppe in der S-, R- oder RS-Form bedeutet.

12. Verfahren gemäß einem der Ansprüche 1 bis 11, dadurch gekennzeichnet, daß man von Verbindungen der Formel II, $B_2$, VI oder Veresterungsmitteln mit einer Gruppe R ausgeht, die ein Alkylrest mit 1 bis 18 Kohlenstoffatomen ist, der durch eine oder mehrere, in Anspruch 1 angegebene funktionelle Gruppen substituiert ist.

13. Verfahren gemäß Anspruch 12, dadurch gekennzeichnet, daß R einen Alkylrest bedeutet, der durch ein oder mehrere Halogenatome substituiert ist.

14. Verfahren gemäß Anspruch 13, dadurch gekennzeichnet, daß R durch ein oder mehrere Fluoratome substituiert ist.

15. Verfahren gemäß Anspruch 14, dadurch gekennzeichnet, daß R eine Gruppe $-CH_2-CF_3$ bedeutet.

16. Verfahren gemäß einem der Ansprüche 1 bis 8, 10 bis 12 und 14 bis 15, dadurch gekennzeichnet, daß man das (S) - α - Cyano - 3 - phenoxybenzyl - (1R,cis) - 2,2 - dimethyl - 3 - [(Z) - 3 - oxo - 3 - (2 - (1,1,1,3,3,3 - hexafluor) - propoxy) - 1 - propenyl] - cyclopropan - carboxylat herstellt.

17. Verwendung der Verbindungen der Formel (I') gemäß Anspruch 1 zur Bekämpfung von Parasiten der Pflanzen, Paratiten von Räumlichkeiten und Parasiten warmblütiger Tiere.

18. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß Anspruch 1 enthalten.

19. Insektizide Zusammensetzungen, enthaltend als Wirkstoff zumindest eines der Produkte gemäß Anspruch 1.

20. Insektizide Zusammensetzungen, enthaltend als Wirkstoff das Produkt gemäß Anspruch 16.

21. Zusammensetzungen für die tierische Ernährung, enthaltend als Wirkstoff zumindest eines der Produkte gemäß Anspruch 1.

22. Mit insektizider, akarizider oder nematizider Aktivität ausgestattete Zusammensetzungen, dadurch gekennzeichnet, daß sie als Wirkstoff einesteils zumindest eine der Verbindungen der allgemeinen Formel (I') und anderenteils zumindest einen der Pyrethrinoidester enthalten, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, unter den Estern des 5-Benzyl-3-furyl-methylalkohols der 2,2-Dimethyl-3-(2-oxo-3-tetrahydrothiophenyliden-methyl)-cyclopropan-1-carbonsäuren, unter den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, unter den Estern der α-Cyano-3-phenoxybenzylalkohole der 2,2-Dimethyl-3-(2,2-dibromvinyl)-cyclopropan-1-carbonsäuren, unter den Estern des 3-Phenoxybenzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furyl-methylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, worin "halo" ein Fluor-, Chlor- oder Bromatom bedeutet, wobei die Verbindungen (I') in sämtlichen ihrer möglichen stereoisomeren Formen ebenso wie die sauren Verknüpfungskomponenten und die Alkohole der vorstehenden Pyrethrinoidester vorliegen können.

23. Zusammensetzungen gemäß einem der Ansprüche 18 bis 21, dadurch gekennzeichnet, daß die Verbindungen der Formel (I') der Formel (I) entsprechen, wie in Anspruch 20 definiert.

**Claims for the Contracting States: BE CH DE FR GB IT LI LU NL SE**

1. In all the possible isomeric forms or in the form of mixtures, the compounds with the formula (I')

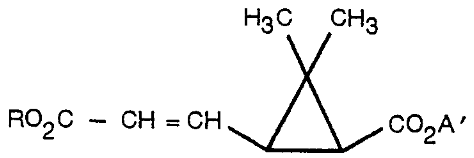

(I')

in which the cyclopropane copula is of IR cis structure and the double bond is of Z geometry and in which A' represents

either an alkyl radical containing from 1 to 18 carbon atoms

or a benzyl radical possibly substituted by one or more radicals chosen from the group composed of the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylene dioxy radical and halogen atoms.

or a group

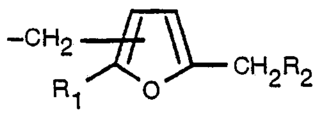

in which the substituent $R_1$ represents a hydrogen atom or a methyl radical and the substituent $R_2$ a monocyclic aryl radical or a group $—CH_2—C{\equiv}CH$

or a group

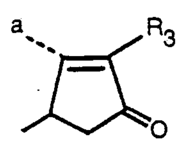

in which *a* represents a hydrogen atom or a methyl radical and $R_3$ represents the radical $—CH_2—CH{=}CH_2$, $—CH_2—CH{=}CH—CH_3$, $—CH_2—CH{=}CH—CH{=}CH_2$, $CH_2—CH{=}CH—CH_2—CH_3$,

or a group

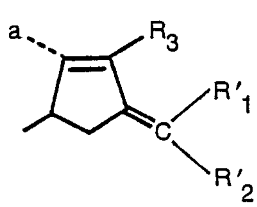

in which *a* represents a hydrogen atom or a methyl radical, $R_3$ retains the same significance as previously, $R'_1$ and $R'_2$, being identical or different, represent a hydrogen atom, a halogen atom, an alkyl radical containing from 1 to 6 carbon atoms, an aryl radical containing from 6 to 10 carbon atoms, an alkyloxycarbonyl group including from 2 to 5 carbon atoms, or a cyano group,

or a group

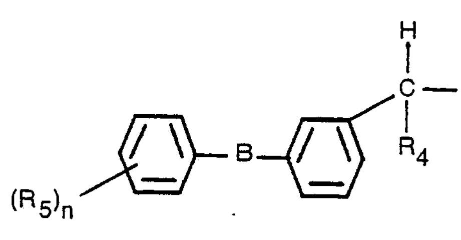

in which B represents an oxygen or sulphur atom or a group

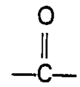

or $—CH_2—$ and $R_4$ represents a hydrogen atom, a —C≡N radical, a methyl radical, a $—CONH_2$ radical, a $—CSNH_2$ radical or a —C≡CH radical, $R_5$ represents a halogen atom or a methyl radical and n represents a number 0, 1 or 2,

or a group

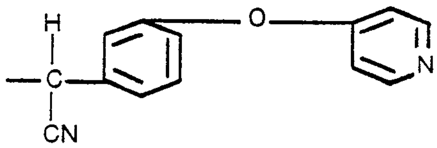

or a group

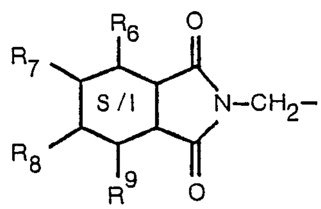

in which the substituents $R_6$, $R_7$, $R_8$, $R_9$ represent a hydrogen atom, a chlorine atom, or a methyl radical and in which S/I symbolises an aromatic ring or a similar dihydro or tetrahydro ring,

or a group

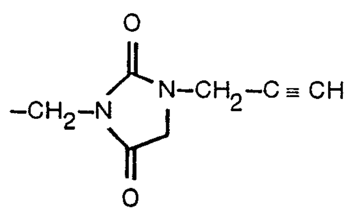

or a group

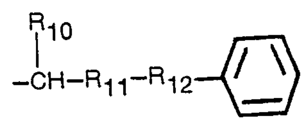

in which $R_{10}$ represents a hydrogen atom or a radical CN, $R_{12}$ represents a radical $—CH_2—$ or an oxygen atom, $R_{11}$ represents a thiazolyl or a thiadiazolyl radical, of which the bond with

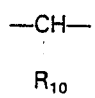

can be found at any one of the available positions $R_{12}$ being attached to $R_{11}$ by the carbon atom included between the sulphur atom and a nitrogen atom,

or a group

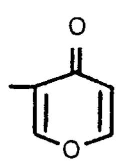

or a group

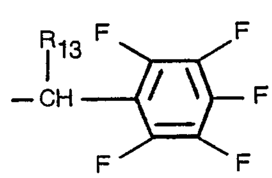

in which $R_{13}$ represents a hydrogen atom or a radical CN,
or a group

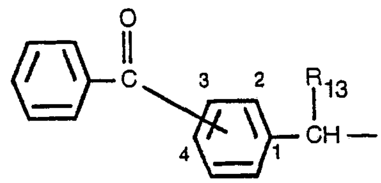

in which $R_{13}$ is defined as above, and the benzoyl radical is in position 3 or 4,
or a group

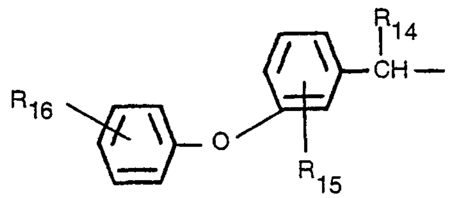

in which $R_{14}$ represents a hydrogen atom, a methyl, ethynyl or cyano radical and $R_{15}$ and $R_{16}$, are different and represent a hydrogen, fluorine or bromine atom,
or a group

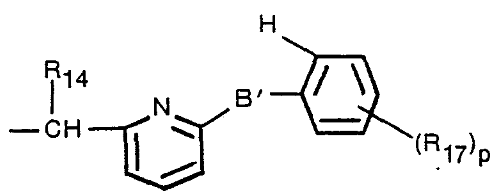

in which $R_{14}$ is defined as above, each of the $R_{17}$'s represents independently an alkyl group containing from 1 to 4 carbon atoms, an alkoxy group containing from 1 to 4 carbon atoms, an alkylthio group containing from 1 to 4 carbon atoms, an alkylsulphonyl group containing from 1 to 4 carbon atoms, a trifluoromethyl, 3,4-methylenedioxy, chloro, fluoro or bromo group, p represents a numeral 0, 1 or 2, and B' represents an oxygen atom or a sulphur atom and R represents an alkyl radical containing from 1 to 18 carbon atoms, substituted by one or more identical or different groups chosen from the group constituted by the halogen atoms, the OH or SH groups, the OR' or SR' groups in which R' represents an alkyl radical containing from 1 to 8 carbon atoms, the groups $NO_2$ or

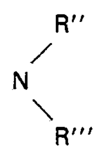

in which R'' and R''', being identical or different, represent a hydrogen atom or an alkyl radical containing from 1 to 8 carbon atoms, the groups C≡N, $SO_3H$ or $PO_4H_2$ or the groups $COalk_1$, $SO_2alk_2$, or $SO_3alk_3$ in which $alk_1$, $alk_2$ and $alk_3$ represent alkyl radicals containing from 1 to 18 carbon atoms, or R represents an alkyl radical containing from 1 to 18 carbon atoms substituted by an aryl radical, itself possibly substituted by one or more OH, Oalk or alk groups containing from 1 to 8 carbon atoms, by one or more $CF_3$, $OCF_3$ $SCF_3$, or by a group (G):

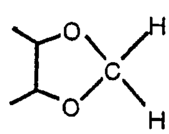

(G)

or R represents an alkyl radical containing from 1 to 18 carbon atoms, substituted on two adjacent carbons by a group ($G_1$)

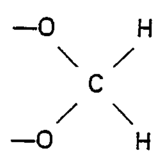

($G_1$)

or substituted by a group

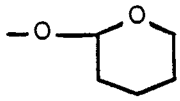

or R represents an aryl group containing from 6 to 14 carbon atoms, possibly substituted by one or more OH, Oalk or alk groups containing from 1 to 8 carbon atoms or by a $CF_3$, $OCF_3$ or $SCF_3$ group, or R represents a pyridinyl, furanyl, thiophenyl, oxazolyl or thiazolyl radical.

2. In all the possible isomeric forms or in the form of mixtures, the compounds with the formula (I'), as defined in Claim 1, answering to the formula (I)

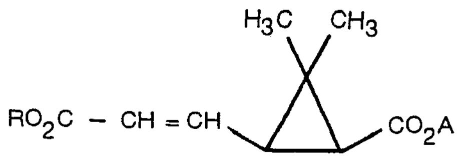

(I)

in which the structure of the cyclopropane copula is 1R cis and the geometry of the double bond is Z and in which A represents

either an alkyl radical containing from 1 to 18 carbon atoms,

or a benzyl radical, possibly substituted, as defined in Claim 1,

or a group

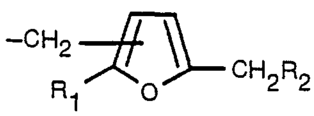

in which the substituents $R_1$ and $R_2$ are as defined in Claim 1

or a group

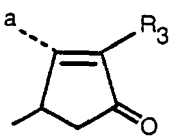

in which *a* and $R_3$ are as defined in Claim 1,

or a group

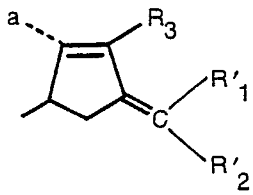

in which *a*, $R'_1$, $R'_3$ are as defined in Claim 1,

or a group

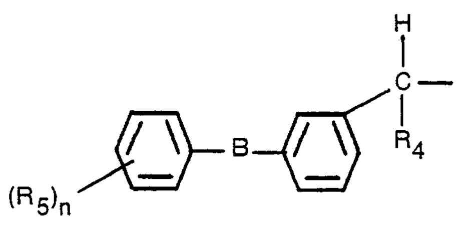

in which B represents an oxygen atom,

a group

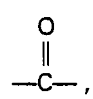

or $-CH_2-$, $R_4$ is as defined in Claim 1, $R_5$ represents a chlorine atom or a methyl radical and n represents a numeral 0, 1 or 2,

or a group

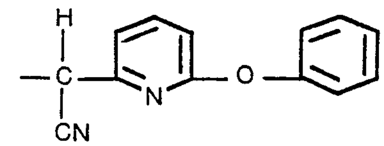

or a group

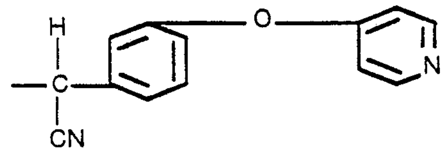

or a group

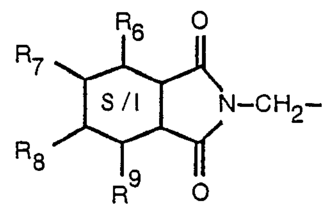

in which the substituents $R_6$, $R_7$, $R_8$, $R_9$ and S/I are as defined in Claim 1,
or a group

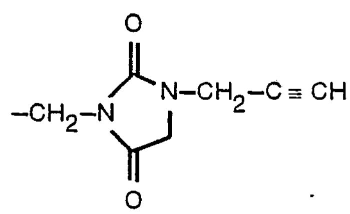

and R is as defined in Claim 1.

3. The compounds with the formula (I'), as defined in one or other of the Claims 1 and 2, for which A represents a α-cyano-3-phenoxybenzyl group in the S, R or RS form.

4. The compounds with the formula (I'), as defined in one or other of the Claims 1 and 2, for which A represents a 2-methyl-4-oxo-3-(2-propenyl)-2-cyclopenten-1-yl group, in the S, R or RS form.

5. The compounds with the formula (I'), as defined in any one of the Claims 1 to 4, for which R represents an alkyl radical containing from 1 to 18 carbon atoms, substituted by one or more functional groups mentioned in Claim 1.

6. The compounds with the formula (I'), as defined in Claim 5, characterized in that R represents an alkyl radical substituted by one or more halogen atoms.

7. The compounds with the formula (I'), as defined in Claim 6, characterized in that R is substituted by one or more fluorine atoms.

8. The compounds with the formula (I'), as defined in Claim 7, for which R represents a $CH_2CF_3$ group.

9. The compound with the formula (I'),the name of which follows:

(IR cis)-2,2-dimethyl-3[(Z)-3-oxo-3-(2-(1,1,1,3,3,3-hexafluoro)-propoxy)-1-propenyl]cyclpropane carboxylate of (S) α-cyano-3-phenoxybenzyl.

10. Preparation process for the compounds with the formula (I') as defined in any of the Claims 1 to 9, characterized in that an acid with the formula (II)

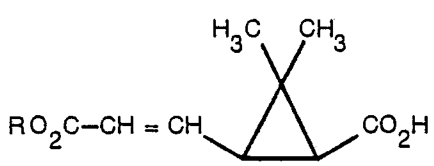

(II)

in which the cyclopropane copula is of 1R cis structure and the double bond of Z geometry and in which R retains the same significance as in Claim 1, or a functional derivative of this acid, is made to react with an alcohol with the formula (III)

$$A'—OH \qquad (III)$$

in which A′ retains the same significance as in Claim 1, in order to obtain the corresponding compound with the formula (I′).

11. Process according to Claim 10, characterized in that the compounds with the formula (II) are prepared by submitting a compound with the formula ($B_1$)

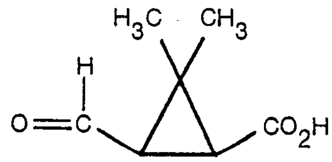

($B_1$)

in the form of the cis lactone, in an organic solvent, to the action of a compound with the formula ($B_2$)

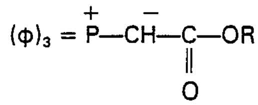

($B_2$)

in which R retains its previous significance, so as to obtain the corresponding compound with the formula (II)

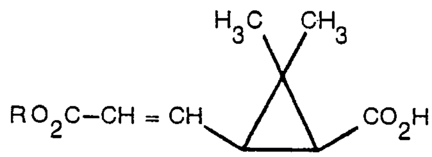

(II)

in which the cyclopropane copula is of 1R cis structure in the form of a mixture of E and Z isomers, which is separated into each of the isomers.

12. Process according to Claim 11, characterized in that the compounds with the formula (II) are prepared by making a compound with the formula (IV)

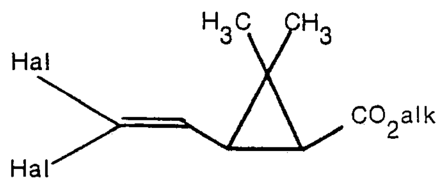

(IV)

in which the cyclopropane copula is of 1R cis structure and in which Hal represents a halogen atom and alk represents an alkyl radical containing from 1 to 20 carbon atoms, react in the first instance with an alkaline agent able to remove the halogen atoms, then, in the second instance,

either with an agent able to introduce the carboxyl group so as to obtain the compound with the formula (V)

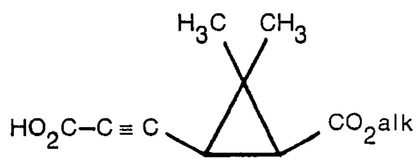

(V)

in which the cyclopropane copula is of 1R cis structure, which is submitted to the action of an esterification agent so as to obtain the compound with the formula (VI)

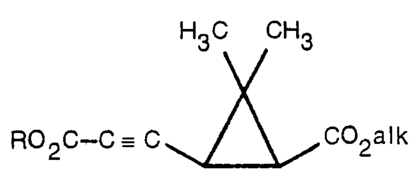

(VI)

in which the cyclopropane copula is of 1R cis structure and in which R retains the same significance as previously,

or with a derivative with the formula

$Hal—CO_2R$

in which Hal represents a halogen atom and R retains its previous significance, so as to obtain directly the compound with the formula (VI)

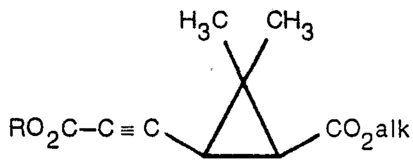

(VI)

in which the cyclopropane copula is of 1R cis structure, then the compound with the formula (VI) is submitted to the action of a mild hydrogenation agent, so as to obtain the compound with the formula (VII)

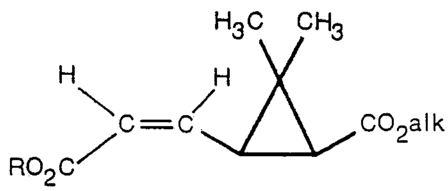

(VII)

in which the cyclopropane copula is of 1R cis structure and in which the double bond has Z geometry, which is submitted to the action of an acid hydrolysis agent able to cleave selectively the ester function on the carbon in position 1 of the cyclopropane, in order to obtain the corresponding compound with the formula (II).

13. Process according to Claim 12, characterized in that the compound with the formula (V) is first submitted to the action of a mild hydrogenation agent in order to obtain the compound with the formula (VIII)

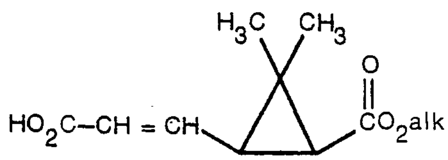

(VIII)

in which the cyclopropane copula is of 1R cis structure in which the double bond has Z geometry, which is submitted to the action of an esterification agent, so as to obtain the corresponding compound with the formula (VII)

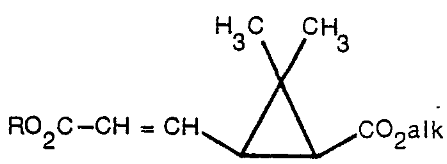

(VII)

in which the cyclopropane copula is of 1R cis structure and in which R retains its previous significance, then the synthesis as described in Claim 12 is continued.

14. Process according to Claims 10 and 12, characterized in that a compound with the formula (VI)

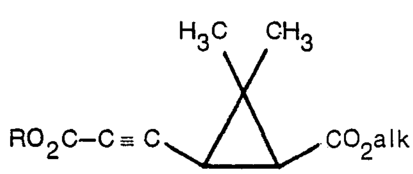

(VI)

in which the cyclopropane copula is of 1R cis structure and in which R and alk are defined as in Claims 10 and 12, is submitted to the action of an acid hydrolysis agent able to cleave selectively the ester function in position 1 of the cyclopropane, so as to obtain the compound with the formula (IX)

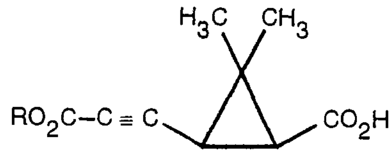

(IX)

in which the cyclopropane copula is of 1R cis structure and in which R is defined as previously, which either is submitted, if appropriate, in the form of a functional derivative, to the action of an alcohol with the formula (III)

$$A'—OH \quad (III)$$

in which A' retains the same significance as in Claim 1, so as to obtain the compound with the formula (X)

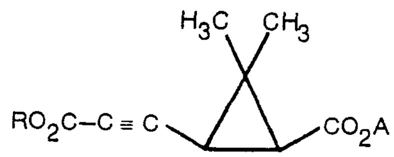

(X)

in which the cyclopropane copula is of 1R cis structure and in which R and A' retain the same significance as previously, which is submitted to the action of a mild hydrogenation agent, so as to obtain the compound with the formula (I'),

or is submitted first to the action of a mild hydrogenation agent, so as to obtain the compound with the formula (II)

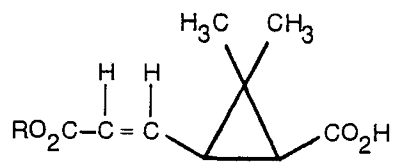

(II)

in which R is defined as previously, the cyclopropane copula is of 1R cis structure and the double bond has Z geometry, then, if appropriate, in the form of a functional derivative, to the action of an alcohol (III) to obtain the compound with the formula (I').

15. Process for the preparation of the compounds with the formula (I'), as defined in Claim 1, characterized in that a compound with the formula (XI)

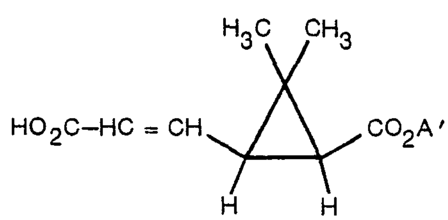

(XI)

in which the cyclopropane copula is of 1R cis structure and in which the double bond has Z geometry is submitted to the action of an esterification agent, so as to obtain the corresponding compound with the formula (I').

16. Process according to Claim 15, characterized in that the compound with the formula (XI) is prepared by submitting an acid with the formula (V)

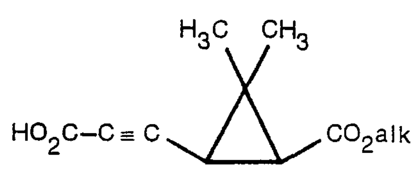

(V)

in which the cyclopropane copula is of 1R cis structure and in which alk represents an alkyl radical

containing from 1 to 8 carbon atoms, to the action of 2,2,2-trichloroethanol, so as to obtain the compound with the formula (XII)

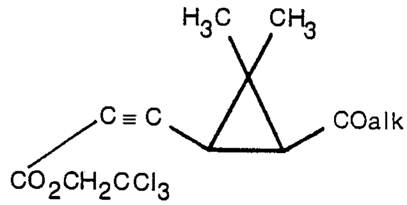

(XII)

in which the cyclopropane copula is of 1R cis structure, which is submitted to the action of an acid hydrolysis agent in order to obtain the compound with the formula (XIII)

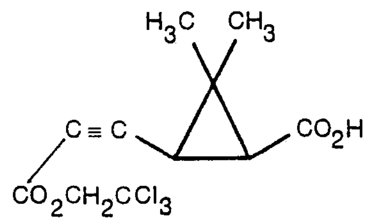

(XIII)

in which the cyclopropane copula is of 1R cis structure which is submitted to the action of an alcohol with the formula (III)

$$A'{-}OH \qquad (III)$$

in which A' retains the same significance as in Claim 1, in order to obtain the compound with the formula (XIV)

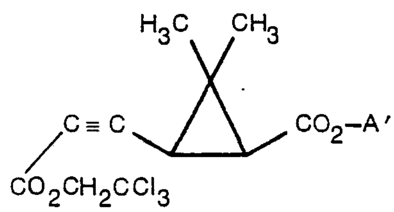

(XIV)

in which the cyclopropane copula is of 1R cis structure, which is submitted to the action of a cleavage agent for the ester function carried by the acetylene carbon, so as to obtain the compound with the formula (XV)

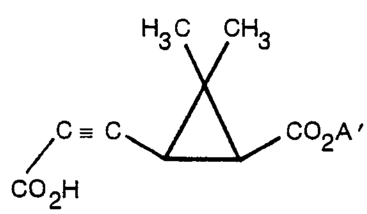

(XV)

in which the cyclopropane copula is of 1R cis structure which is submitted to the action of a mild hydrogenation agent so as to obtain the compound with the formula (XI).

17. Process according to Claims 15 and 16, characterized in that a compound with the formula (XV)

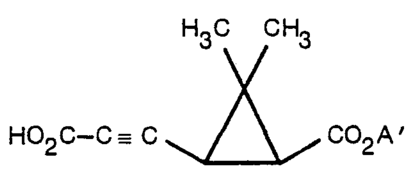

(XV)

in which the cyclopropane copula is of 1R cis structure and in which A' is defined as in Claim 1, is submitted to the action of an esterification agent, so as to obtain the compound with the formula (X)

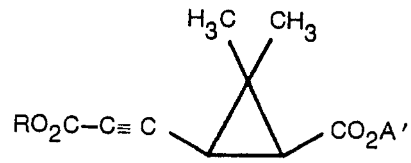

(X)

in which the cyclopropane copula is of 1R cis structure and in which R and A' are defined as in Claim 1, which is submitted to the action of a mild hydrogenation agent, so as to obtain the compound with the formula (I'), in which the cyclopropane copula is of 1R cis structure and the geometry of the double bond is Z.

18. Process for the preparation of the compounds with the formula (I'), in which R represents an alkyl radical substituted by one or more hydroxy radicals, according to which, by a process according to any one of the Claims 10 to 17, there are first prepared compounds with the formula (I') in which R represents an alkyl radical substituted by one or more protected hydroxy radicals, and characterized in that these compounds are submitted to the action of an acid hydrolysis agent.

19. Use of the compounds with the formula (I'), as defined in any one of the Claims 1 to 9, in combatting parasites of vegetation, parasites of premises and parasites of warm-blooded animals.

20. Compositions intended for combatting parasites of vegetation, parasites of premises and parasites of warm-blooded animals, characterized in that they contain as active principle at least one of the products defined in any one of the Claims 1 to 9.

21. Insecticide compositions containing as active principle at least one of the products defined in any one of the Claims 1 to 8.

22. Insecticide compositions containing as active principle the product defined in Claim 9.

23. Compositions intended for animal feeding containing as active principle at least one of the products defined in any one of the Claims 1 to 9.

24. Combinations endowed with insecticide, acaricide or nematocide activity, characterized in that they contain as active material, on the one hand, one at least of the compounds with the general formula (I'), and, on the other hand, one at least of the pyrethrinoid esters chosen from the group constituted by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with chrysanthemic acids, by the esters of 5-benzyl-3-furylmethyl alcohol with 2,2-dimethyl-3-(2-oxo-3-tetrahydrothiophenylidenemethyl)-cyclopropane-1-carboxylic acids, by the esters of 3- phenoxybenzyl alcohol and α-cyano-3-phenoxybenzyl alcohols with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropylacetic acids, by the esters of allethrolones, of 3,4,5,6-tetrahydrophthalimidomethyl alcohol of 5-benzyl-3-furylmethyl alcohol, of 3-phenoxybenzyl alcohol and of α-cyano-3-phenoxybenzyl alcohols with 2,2-dimethyl-3-(1,2,2,2-tetrahaloethyl)cyclopropane-1-carboxylic acids, in which "halo" represents a fluorine, chlorine or bromine atom, it being understood that the compounds (I') can exist in all their possible stereo-isomeric forms, as can also the acid and alcohol copulas of the above pyrethrinoid esters.

25. As medicaments, the compounds defined in any one of the Claims 1 to 9.

26. Pharmaceutical compositions containing as active principle, one at least of the medicaments defined in Claim 25.

27. As intermediate products necessary for the preparation of the compounds with the formula (I') as defined in Claim 1, the compounds with the formula (II)

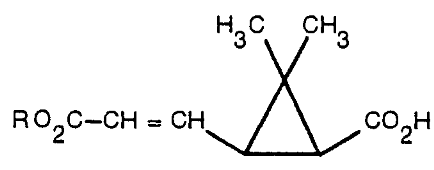

(II)

in which the cyclopropane copula is of 1R cis structure and the double bond of Z geometry, as defined in Claim 1 and the compounds with the formula (VI)

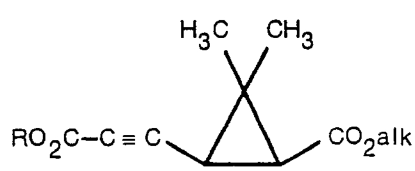

(VI)

in which the cyclopropane copula is of 1R cis structure, as defined in Claim 12.

28. As intermediate products necessary for the preparation of the compounds with the formula I' as defined in Claim 1, the compounds with the formula (IX):

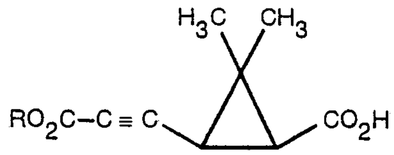

(IX)

and the compounds with the formula (X)

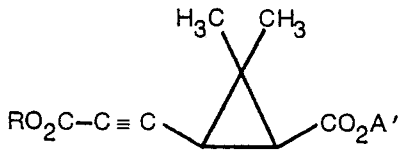

(X)

as defined in Claim 14.

**Claims for the Contracting State: AT**

1. Process for preparing in all the possible isomeric forms or in the form of mixtures, the compounds with the formula (I')

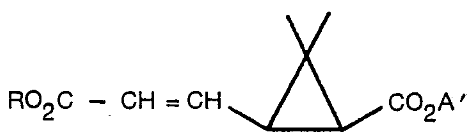

(I')

in which the cyclopropane copula is of IR cis structure and the double bond of Z geometry and in which A' represents

either an alkyl radical containing from 1 to 18 carbon atoms

or a benzyl radical possibly substituted by one or more radicals chosen from the group constructed by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylene dioxy radical and halogen atoms.

or a group

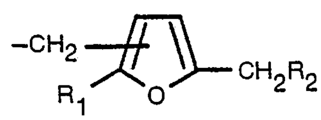

in which the substituent $R_1$ represents a hydrogen atom or a methyl radical and the substituent $R_2$ a monocyclic aryl or a $-CH_2-C{\equiv}CH$ group,

or a group

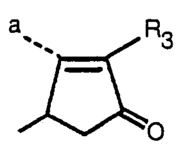

in which *a* represents a hydrogen atom or a methyl radical and $R_3$ represents the radical $-CH_2-CH{=}CH_2$, $-CH_2-CH{=}CH-CH_3$, $-CH_2-CH{=}CH-CH{=}CH_2$, $CH_2-CH{=}CH-CH_2-CH_3$,

or a group

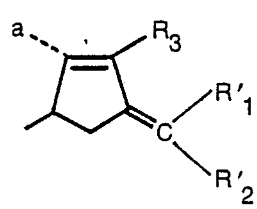

in which *a* represents a hydrogen atom or a methyl radical, $R_3$ retains the same significance as previously, $R'_1$ and $R'_2$, being identical or different, represent a hydrogen atom, a halogen atom, an alkyl radical containing from 1 to 6 carbon atoms, an aryl radical containing from 6 to 10 carbon atoms, an alkyl-oxycarbonyl group containing from 2 to 5 carbon atoms, or a cyano group,

or a group

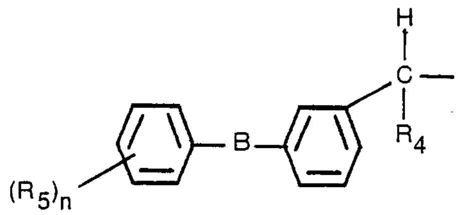

in which B represents an oxygen or sulphur atom or a group

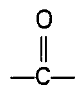

or $—CH_2—$ and $R_4$ represents a hydrogen atom, a $—C{\equiv}N$ radical, a methyl radical, a $—CONH_2$ radical, a $—CSNH_2$ radical or a $—C{\equiv}CH$ radical, $R_5$ represents a halogen atom or a methyl radical and n represents a numeral 0, 1 or 2,

or a group

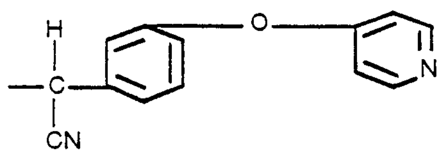

or a group

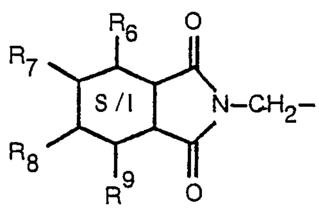

in which the substituents $R_6$, $R_7$, $R_8$, $R_9$ represent a hydrogen atom, a chlorine atom, or a methyl radical and in which S/I symbolises an aromatic ring or a similar dihydro or tetrahydro ring,

or a group

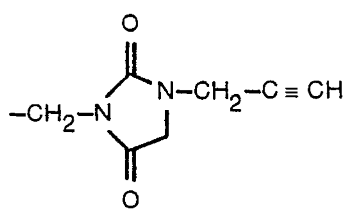

or a group

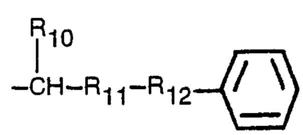

in which $R_{10}$ represents a hydrogen atom or a CN radical, $R_{12}$ represents a $—CH_2—$ radical or an oxygen atom, $R_{11}$ represents a thiazolyl or a thiadiazolyl radical, of which the bond with

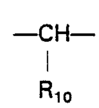

can be found at any one of the available positions, $R_{12}$ being attached to $R_{11}$ by the carbon atom included between the sulphur atom and a nitrogen atom,

or a group

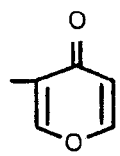

or a group

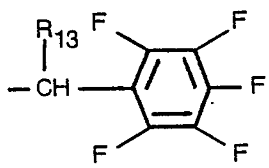

in which $R_{13}$ represents a hydrogen atom or a CN radical,

or a group

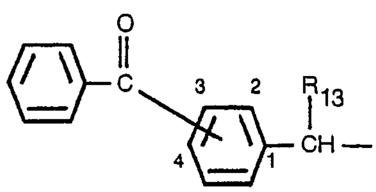

in which $R_{13}$ is defined as above, and the benzoyl radical is in position 3 or 4,

or a group

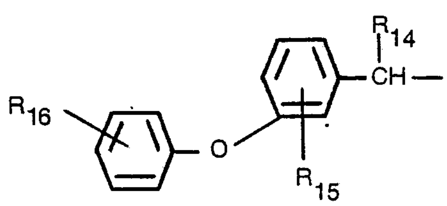

in which $R_{14}$ represents a hydrogen atom, a methyl, ethynyl or cyano radical and $R_{15}$ and $R_{16}$, which are different, represent a hydrogen atom, a fluorine or a bromine atom,

or a group

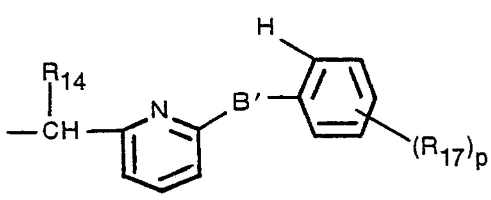

in which $R_{14}$ is defined as above, each of the $R_{17}$'s represents independently an alkyl group containing from 1 to 4 carbon, an alkoxy group containing from 1 to 4 carbon atoms, an alkylthio group containing from 1 to 4 carbon atoms, an alkylsulphonyl group containing from 1 to 4 carbon atoms, a trifluoromethyl, 3,4-methylenedioxy, chloro, fluoro or bromo group, p represents a numeral 0, 1 or 2 and B' represents an oxygen atom or a sulphur atom and R represents an alkyl radical containing from 1 to 18 carbon atoms, substituted by one or more identical or different functional groups chosen from the group constituted by the halogen atoms, the OH or SH groups, the OR' or SR' groups in which R' represents an alkyl radical containing from 1 to 8 carbon atoms, the $NO_2$ or

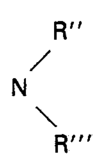

groups in which R'' and R''', which are identical or different, represent a hydrogen atom or an alkyl radical containing from 1 to 8 carbon atoms, the groups C≡N, $SO_3H$ or $PO_4H_2$ groups or the $COalk_1$, $SO_2alk_2$, or $SO_3alk_3$ groups in which $alk_1$, $alk_2$ and $alk_3$ represent alkyl radicals containing from 1 to 18 carbon atoms, or R represents an alkyl radical containing from 1 to 18 carbon atoms substituted by an aryl radical, itself possibly substituted by one or more OH, Oalk or alk groups containing from 1 to 8 carbon atoms, by one or more $CF_3$, $OCF_3$ $SCF_3$, or by a group (G):

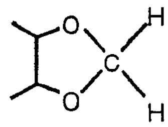

(G)

or R represents an alkyl radical containing from 1 to 18 carbon atoms, substituted on two adjacent carbons by a group ($G_1$)

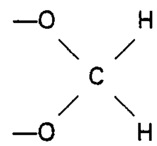

($G_1$)

or substituted by a group

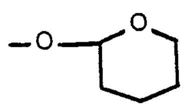

or R represents an aryl group containing from 6 to 14 carbon atoms possibly substituted by one or more OH, Oalk or alk groups containing from 1 to 8 carbon atoms or by a $CF_3$, $OCF_3$ or $SCF_3$ group, or R represents a pyridinyl, furanyl, thiophenyl, oxazolyl or thiazolyl radical, characterized in that an acid with the formula (II):

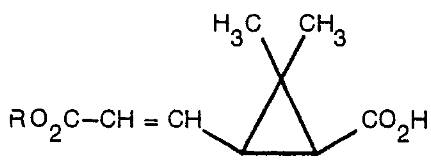

(II)

in which the cyclopropane copula is of 1R cis structure and in which R retains the same significance as previously, or a functional derivative of this acid, is made to react with an alcohol with the formula (III)

A'—OH (III)

in which A' retains the same significance as previously, so as to obtain the corresponding compound with the formula (I').

2. Process according to Claim 1, characterized in that the compounds with the formula (II) are prepared by making a compound with the formula ($B_1$)

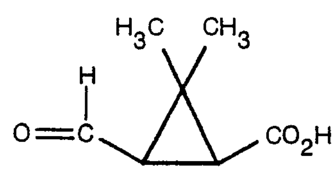

($B_1$)

in the form of the cis lactone, in an organic solvent, to the action of a compound with the formula ($B_2$)

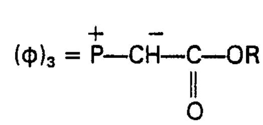

($B_2$)

in which R retains its previous significance, so as to obtain the corresponding compound with the formula (II)

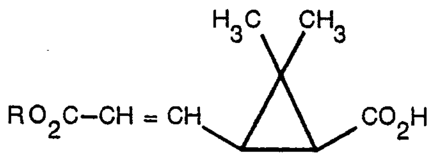

(II)

in which the cyclopropane copula is of 1R cis structure in the form of a mixture of E and Z isomers, which are separated into each of the isomers.

3. Process according to Claim 1, characterized in that the compounds with the formula (II) are prepared by making a compound with the formula (IV)

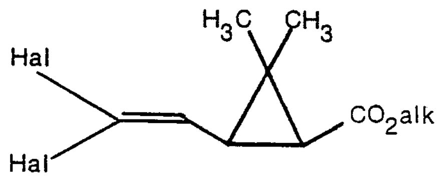

(IV)

in which the cyclopropane copula is of 1R cis structure and in which Hal represents a halogen atom and alk represents an alkyl radical containing from 1 to 20 carbon atoms, react first with an alkaline agent able to remove the halogen atoms, then either with an agent able to introduce the carboxyl group so as to obtain the compound with the formula (V)

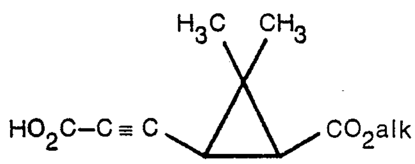

(V)

in which the cyclopropane copula is of 1R cis structure, which is submitted to the action of an esterification agent so as to obtain the compound with the formula (VI)

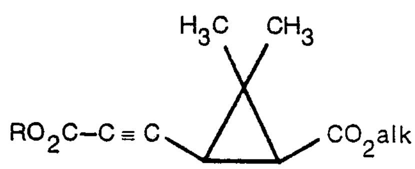

(VI)

in which the cyclopropane copula is of 1R cis structure and in which R retains the same significance as previously,

or with a derivative with the formula

$$Hal—CO_2R$$

in which Hal represents a halogen atom and R retains its previous significance, so as to obtain directly the compound with the formula (VI)

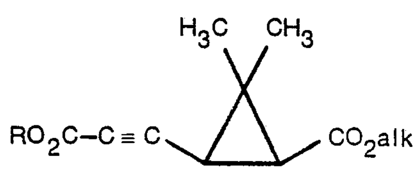

(VI)

in which the cyclopropane copula is of 1R cis structure, then the compound with the formula (VI) is submitted to the action of a mild hydrogenation agent, so as to obtain the compound with the formula (VII)

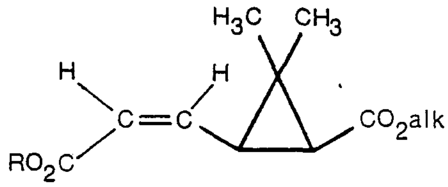

(VII)

in which the cyclopropane copula is of 1R cis structure and in which the double liaison has Z geometry, which is submitted to the action of an acid hydrolysis agent able to cleave selectively the ester function on the carbon at position 1 of the cyclopropane, so as to obtain the corresponding compound with the formula (II).

4. Process according to Claim 1, characterized in that first the compound with the formula (V) is submitted to the action of a mild hydrogenation agent in order to obtain the compound with the formula (VIII)

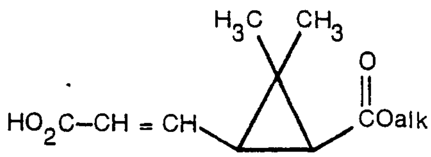

(VIII)

in which the cyclopropane copula is of 1R cis structure and the double bond has Z geometry, which is submitted to the action of an esterification agent, so as to obtain the corresponding compound with the formula (VII)

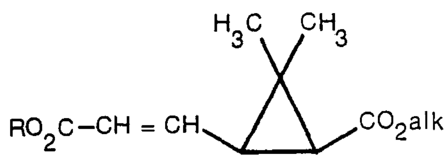

(VII)

in which the cyclopropane copula is of 1R cis structure and the double bond Z geometry, in which R retains its previous significance, then the synthesis as described in Claim 3 is continued.

5. Process according to Claims 1 and 3, characterized in that a compound with the formula (VI)

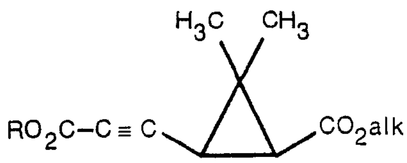

(VI)

in which the cyclopropane copula is of 1R cis structure and in which R and alk are defined as in Claims 1 and 3, is submitted to the action of an acid hydrolysis agent able to cleave selectively the ester function at position 1 of the cyclopropane, so as to obtain the compound with the formula (IX)

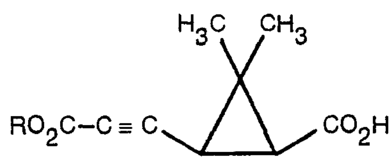

(IX)

in which the cyclopropane copula has 1R cis structure and in which R is defined as previously, which either is submitted, if appropriate, in the form of a functional derivative, to the action of an alcohol with the formula (III)

$$A'—OH \qquad (III)$$

in which A' retains the same significance as in Claim 1, so as to obtain the compound with the formula (X)

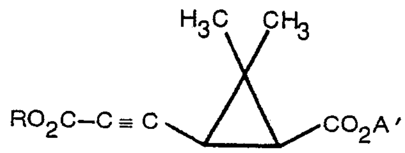

(X)

in which the cyclopropane copula is of 1R cis structure and in which R and A' retain the same significance as previously, which is submitted to the action of a mild hydrogenation agent, so as to obtain the compound with the formula (I'),

or is submitted first to the action of a mild hydrogenation agent, so as to obtain the compound with the formula (II)

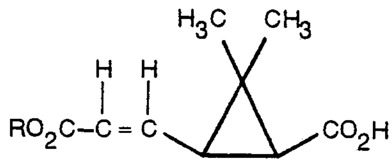

(II)

in which R is defined as previously, the cyclopropane copula is of 1R cis structure and the double bond Z geometry, then, if appropriate, in the form of a functional derivative, to the action of an alcohol (III), so as to obtain the compound with the formula (I').

6. Process for the preparation of the compounds with the formula (I'), as defined in Claim 1, characterized in that a compound with the formula (XI)

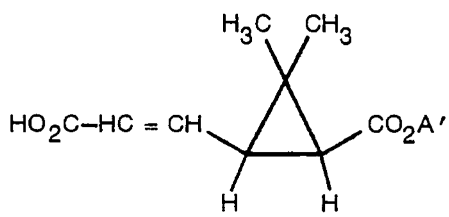

(XI)

in which the cyclopropane copula has 1R cis structure and the double bond has Z geometry, is submitted to the action of an esterification agent, so as to obtain the corresponding compound with the formula (I').

7. Process according to Claim 6, characterized in that the compound with the formula (XI) is prepared by submitting an acid with the formula (V)

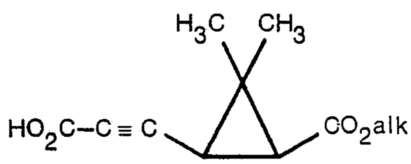

(V)

in which the cyclopropane copula has 1R cis structure and in which alk represents an alkyl radical containing from 1 to 8 carbon atoms, to the action of 2,2,2-trichloroethanol, so as to obtain the compound with the formula (XII)

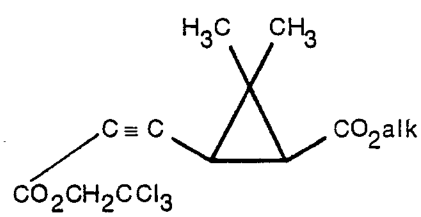

(XII)

in which the cyclopropane copula is of 1R cis structure, which is submitted to the action of an acid hydrolysis agent in order to obtain the compound with the formula (XIII)

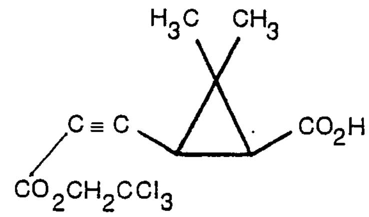

(XIII)

in which the cyclopropane copula is of 1R cis structure which is submitted to the action of an alcohol with the formula (III)

A'—OH (III)

in which A' retains the same significance as in Claim 1, in order to obtain the compound with the formula (XIV)

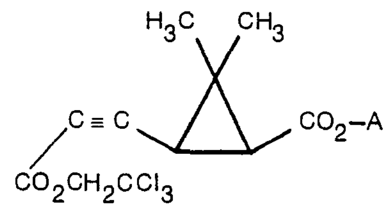

(XIV)

in which the cyclopropane copula is of 1R cis structure, which is submitted to the action of a cleavage agent of the ester function carried by the acetylene carbon, in order to obtain the compound with the formula (XV)

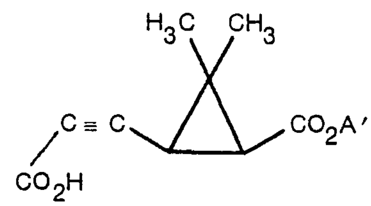

(XV)

in which the cyclopropane copula is of 1R cis structure, which is submitted to the action of a mild hydrogenation agent so as to obtain the compound with the formula (XI).

8. Process according to Claims 6 and 7, characterized in that a compound with the formula (XV)

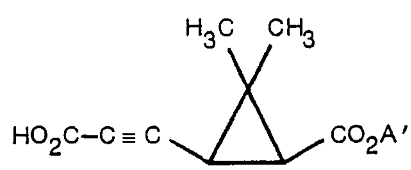

(XV)

in which the cyclopropane copula is of 1R cis structure and A' is defined as in Claim 1, is submitted to the action of an esterification agent, so as to obtain the compound with the formula (X)

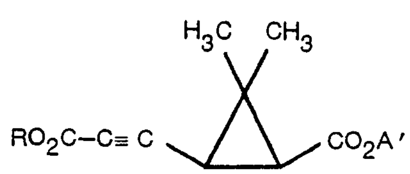

(X)

in which the cyclopropane copula is of 1R cis structure and R and A' are defined as in Claim 1, which is submitted to the action of a mild hydrogenation agent so as to obtain the compound with the formula (I') in which the cyclopropane copula is of 1R cis structure and the geometry of the double bond is Z.

9. Process for the preparation of the compounds with the formula (I'), in which R represents an alkyl radical substituted by one or more hydroxy radicals, according to which there are first prepared, by a process according to any one of the Claims 1 to 8, compounds with the formula (I') in which R represents an alkyl radical substituted by one or more protected hydroxy radicals, and characterized in that these compounds are submitted to the action of an acid hydrolysis agent.

10. Process according to any one of the Claims 1 to 9, for preparing in all the possible stereo-isomeric forms or in the form of mixtures, the compounds with the formula (I'), as defined in Claim 1, answering to the formula (I)

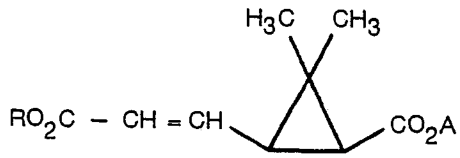

(I)

in which the cyclopropane copula is of 1R cis structure and the double bond has Z geometry, in which A represents

either an alkyl radical containing from 1 to 18 carbon atoms,

or a benzyl radical, possibly substituted as defined in Claim 1,

or a group

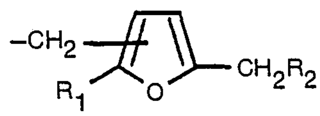

in which the substituents $R_1$ and $R_2$ are as defined in Claim 1

or a group

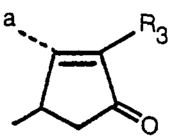

in which *a* and $R_3$ are as defined in Claim 1,

or a group

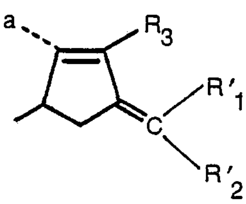

in which *a*, $R'_1$, $R'_2$ and $R_3$ are as defined in Claim 1,

or a group

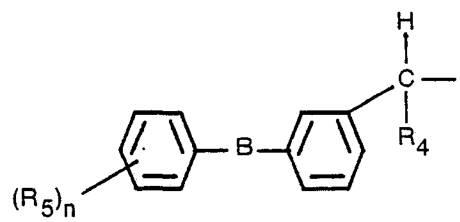

in which B represents an oxygen atom,

a group

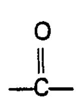 ,

or $—CH_2—$, $R_4$ is as defined in Claim 1, $R_5$ represents a chlorine atom or a methyl radical and n represents a numeral 0, 1 or 2,

or a group

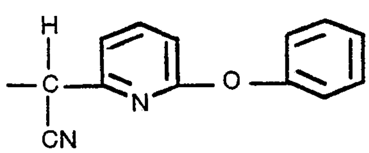

or a group

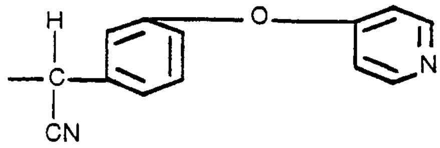

or a group

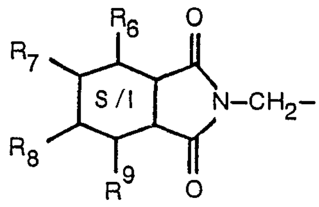

in which the substituents $R_6$, $R_7$, $R_8$, $R_9$ and S/I are as defined in Claim 1, or a group

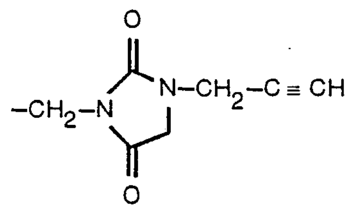

and R is as defined in Claim 1, characterized in that at the start, compounds with the formulae III, XI or XV are used, in which A' has the values of A indicated previously.

11. Process according to any one of the Claims 1 to 10, characterized in that at the start compounds are used with the formulae III, XI or XV, in which A' represents an α-cyano-3-phenoxybenzyl group in the S, R or SR form or a 2-methyl-4-oxo-3-(2-propenyl)-2-cyclopenten-1-yl group, in the S, R or RS form.

12. Process according to any one of the Claims 1 to 11, characterized in that at the start compounds are used with the formulae II, $B_2$, VI or esterification agents including a group r which is an alkyl radical containing from 1 to 18 carbon atoms substituted by one or more functional groups mentioned in Claim 1.

13. Process according to Claim 12, characterized in that R represents an alkyl radical substituted by one or more halogen atoms.

14. Process according to Claim 13, characterized in that R is substituted by one or more fluorine atoms.

15. Process according to Claim 14, characterized in that R represents a group $—CH_2—CF_3$.

16. Process according to any one of the Claims 1 to 8, 10 to 12 and 14 to 15, characterized in that (1R cis)-2,2-dimethyl-3[(Z)-3-oxo-3-(2-(1,1,1,3,3,3-hexafluoro)propoxy)-1-propenyl]cyclopropane carboxylate of (S) α-cyano-3-3-phenoxybenzyl is prepared.

17. Use of the compounds with the formula (I'), as defined in Claim 1, for combatting parasites of vegetation, parasites of premises and parasites of warm-blooded animals.

18. Compositions intended for combatting parasites of vegetation, parasites of premises and parasites of warm-blooded animals, characterized in that they contain as active principle at least one of the products defined in Claim 1.

19. Insecticide compositions containing as active principle at least one of the products defined in Claim 1.

20. Insecticide compositions containing as active principle the product of Claim 16.

21. Compositions intended for animal feeding containing as active principle at least one of the products defined in Claim 1.

22. Combinations endowed with insecticide, acaricide or nematocide activity, characterized in that they contain as active material, on the one hand, one at least of the compounds with the general formula (I'), and, on the other hand, one at least of the pyrethrinoid esters chosen from the group constituted by the esters of allethrolones, of 3, 4, 5, 6-tetrahydrophthalimidomethyl alcohol, of 5-benzyl-3-furylmethyl alcohol, of 3-phenoxybenzyl alcohol and α-cyano-3-phenoxybenzyl alcohols with chrysanthemic acids, by the esters of 5-benzyl-3-furyl methyl alcohol with 2,2-dimethyl-3-(2-oxo-3-tetrahydrothiophenylidenemethyl)-cyclopropane-1-carboxylic acids, by the esters of 3-phenoxybenzyl alcohol and α-cyano-3-phenoxybenzyl alcohols with 2,2-dimethyl-3-(2,2-dichlorovinyl)cyclopropane-1-carboxylic acids, by the esters of α-cyano-3-phenoxybenzyl alcohols with 2,2-dimethyl-3-(2,2-dibromovinyl)cyclopropane-1-carboxylic acids, by the esters of 3-phenoxybenzyl alcohol with 2-parachlorophenyl-2-isopropyl acetic acids, by the esters of allethrolones, of 3,4,5,6-tetrahydrophthalimidomethyl alcohol, of 5-benzyl-3-furylmethyl alcohol, of 3-phenoxybenzyl alcohol and of α-cyano-3-phenoxybenzyl alcohols with 2,2-dimethyl-3-(1,2,2,2-tetrahaloethyl)cyclopropane-1-carboxylic acids, in which "halo" represents a fluorine, chlorine or bromine atom, it being understood that the compounds (I') can exist in all their possible stereo-isomeric forms, as can the acid and alcohol copulas of the above pyrethrinoid esters.

23. Compositions according to any one of the Claims 18 to 21, characterized in that the compounds with the formula (I') answer to the formula (I) as defined in Claim (20).